# EUROPEAN PATENT SPECIFICATION

(11) **EP 4 126 943 B1**
(45) Date of publication and mention of the grant of the patent: **22.04.2026**
(21) Application number: 21716969.7
(22) Date of filing: 23.03.2021
(51) Int. Cl.: C07K 16/24, A61K 31/573, A61K 39/395, A61P 17/00, A61P 31/04

(54) **METHODS FOR TREATING ATOPIC DERMATITIS AND RELATED DISORDERS**
VERFAHREN ZUR BEHANDLUNG VON ATOPISCHER DERMATITIS UND VERWANDTEN ERKRANKUNGEN
MÉTHODES DE TRAITEMENT DE LA DERMATITE ATOPIQUE ET DES TROUBLES ASSOCIÉS

(30) Priority: 23.03.2020 US 202062993443 P; 11.06.2020 US 202063037783 P; 05.08.2020 US 202063061497 P; 21.08.2020 US 202063068593 P; 30.10.2020 AU 2020260519; 30.10.2020 CA 3097453; 30.10.2020 JP 2020182046; 30.10.2020 US 202017084957
(43) Date of publication of application: 08.02.2023
(73) Proprietor: MedImmune Limited, Cambridge Cambridgeshire CB21 6GH (GB)
(72) Inventor: COLICE, Gene, Gaithersburg, Maryland 20878 (US); VAN DER MERWE, Rene, Cambridgeshire SG8 7RE (GB); BAVEREL, Paul, Cambridge Cambridgeshire CB2 8RA (GB)
(74) Representative: Mewburn Ellis LLP
(86) International application number: PCT/EP2021/057463
(87) International publication number: WO 2021/191220

(56) References cited:
- WO-A1-2017/139290
- WO-A1-2018/158332
- US-A1- 2019 270 803
- WOLLENBERG ANDREAS ET AL: "Treatment of atopic dermatitis with tralokinumab, an anti-IL-13 mAb", JOURNAL OF ALLERGY AND CLINICAL IMMUNOLOGY, ELSEVIER, AMSTERDAM, NL, vol. 143, no. 1, 12 June 2018 (2018-06-12), pages 135 - 141, XP085572598, ISSN: 0091-6749, DOI: 10.1016/J.JACI.2018.05.029
- SIMPSON ET AL: "Efficacy and Safety of Tralokinumab Monotherapy in Adult Patients with Moderate-to-Severe Atopic Dermatitis: Results from Two 52-Week, Phase 3 Trials (ECZTRA 1 and ECZTRA 2)", SKIN - THE JOURNAL OF CUTANEOUS MEDICINE - 2020 - NOVEMBER ISSUE & POSTER PRESENTATIONS (FC20 DERMATOLOGY CONFERENCE�), vol. 4, no. 6, 27 October 2020 (2020-10-27), pages 1 - 10, XP093122756, Retrieved from the Internet <URL:https://jofskin.org/index.php/skin/article/view/1066>
- JOHN COLLETT ED - AULTON M E (ED) 2: "DOSAGE REGIMENS", 1 January 2001, PHARMACEUTICS. THE SCIENCE OF DOSAGE FORM DESIGN ED. 2, CHURCHILL LIVIGSTONE, PAGE(S) 275 - 288, ISBN: 978-0-443-05517-1, XP003030862
- FLEMING PATRICK ET AL: "Risk of infection in patients with atopic dermatitis treated with dupilumab: A meta-analysis of randomized controlled trials", JOURNAL OF THE AMERICAN ACADEMY OF DERMATOLOGY, MOSBY, INC, US, vol. 78, no. 1, 4 October 2017 (2017-10-04), pages 62, XP085304886, ISSN: 0190-9622, DOI: 10.1016/J.JAAD.2017.09.052
- WOLLENBERG ET AL.: "A phase 2b dose-ranging efficacy and safety study of tralokinumab in adult patients with moderate to severe atopic dermatitis (AD) ED - Lim Henry W", JOURNAL OF THE AMERICAN ACADEMY OF DERMATOLOGY, vol. 76, no. 6, 2018, XP085049048, ISSN: 0190-9622, DOI: 10.1016/J.JAAD.2017.04.099
- DATABASE EMBASE [online] 12 December 2018 (2018-12-12), GUTTMAN-YASSKY E ET AL: "-Tralokinumab significantly reduces Staphylococcus aureus colonization in adult patients with moderate-to-severe atopic dermatitis (AD): Results from a Phase 2b, randomized, doubleblind, placebo-controlled study (NCT02347176)", XP055811153, Database accession no. EMB-625817671
- DATABASE EMBASE [online] 1 May 2019 (2019-05-01), GUTTMAN-YASSKY E ET AL: "Tralokinumab significantly reduces Staphylococcus aureus colonization in adult patients with moderate-to-severe atopic dermatitis: Results from a Phase IIb, randomized, double-blind, placebo-controlled study", XP055811158, Database accession no. EMB-629676602
- DATABASE EMBASE [online] 11 June 2019 (2019-06-11), GUTTMAN-YASSKY E ET AL: "Tralokinumab phase 2b study: Effects of the anti-interleukin-13 monoclonal antibody on staph. aureus skin colonisation and systemic levels of inflammatory biomarkers in patients with atopic dermatitis", XP055811163, Database accession no. EMB-634426605
- WOLLENBERG ANDREAS ET AL: "POSTER - A Phase 2b Dose-Ranging Efficacy and Safety Study of Tralokinumab in Adult Patients with Moderate to Severe Atopic Dermatitis", SKIN THE JOURNAL OF CUTANEOUS MEDICINE, vol. 2, 23 February 2018 (2018-02-23), pages S29, XP055811194, Retrieved from the Internet <URL:https://jofskin.org/index.php/skin/article/view/281> DOI: 10.25251/skin.2.supp.28

## Description

### FIELD OF THE INVENTION

The present invention relates to methods for treating atopic dermatitis in a subject using the anti-IL-13 antibody tralokinumab.

### BACKGROUND TO THE INVENTION

Atopic dermatitis (AD) is a heterogeneous inflammatory skin disease arising from genetic and environmental factors that disrupt skin barrier function and immune response (Leung, D.Y. and Guttman-Yassky, E. Deciphering the complexities of atopic dermatitis: shifting paradigms in treatment approaches. J Allergy Clin Immunol. 2014; 134: 769-779). Current management generally involves treatment combinations to suppress inflammation, restore skin barrier function, and prevent superinfection (Wollenberg, A., Oranje, A., Deleuran, M., Simon, D., Szalai, Z., Kunz, B. et al. ETFAD/EADV Eczema task force 2015 position paper on diagnosis and treatment of atopic dermatitis in adult and paediatric patients. J Eur Acad Dermatol Venereol. 2016; 30: 729-747).

Topical corticosteroids (TCSs) are overwhelmingly the most frequently prescribed class of drugs for AD patients, although long-term application of a TCS is not recommended. Topical calcineurin inhibitors (TCI) are generally effective and safe as short-term treatments. Skin malignancies and increased risk of lymphomas have prompted regulatory authorities to require a warning regarding the long-term safety of topical tacrolimus and pimecrolimus in their prescribing information, for example. First generation antihistamines are widely prescribed for acute symptomatic treatment of pruritus (itching), although their effectiveness is limited and largely attributed to their sedating effect. Oral immunosuppressants and glucocorticoids are effective, but are sometimes associated with severe toxicity and side effects, thus limiting their use to short term and/or intermittent therapy.

Cyclosporine A (CSA), a therapy for severe AD in some territories, is an immunosuppressant affecting both humoral and cellular immune responses, which increases susceptibility to infections and decreases cancer immunosurveillance. Other commonly recognized toxicities include hypertension and impaired renal and hepatic function. In addition, CSA interacts with other commonly used drugs, potentially affecting their metabolism and effect. Systemic immunosuppressants are typically reserved for treatment of moderate-to-severe AD because of their associated with adverse events and unsuitability for long-term use (Wollenberg, A., Oranje, A., Deleuran, M., Simon, D., Szalai, Z., Kunz, B. et al. ETFAD/EADV Eczema task force 2015 position paper on diagnosis and treatment of atopic dermatitis in adult and paediatric patients. J Eur Acad Dermatol Venereal. 2016; 30: 729-747). Therefore more effective and well-tolerated therapies are required that target the mechanisms of AD pathophysiology rather than simply providing symptom relief.

A key feature of AD is upregulation of IL-13 and interleukin-4 (IL-4) in lesional and nonlesional skin, suggesting both cytokines can contribute to AD pathogenesis (see Nomura, I., Goleva, E., Howell, M.D., Hamid, Q.A., Ong, P.Y., Hall, C.F. et al. Cytokine milieu of atopic dermatitis, as compared to psoriasis, skin prevents induction of innate immune response genes. J Immunol. 2003; 171: 3262-3269; Tazawa, T., Sugiura, H., Sugiura, Y., and Uehara, M. Relative importance of IL-4 and IL-13 in lesional skin of atopic dermatitis. Arch Dermatol Res. 2004; 295: 459-464). Moreover, AD severity is associated with increased IL-13 and associated chemokine mRNA and serum levels, whereas reductions in IL-13 concentrations have correlated with treatment response and improved clinical outcomes. Although treatment with dupilumab, a human mAb that inhibits both IL-4 and IL-13 signaling, has demonstrated improvements in AD symptoms, the relative contribution of each of these cytokines to AD pathogenesis was unclear.

In real-world clinical practice, the prevalence of conjunctivitis in patients receiving dupilumab has been estimated to be 26% based on a systematic review of the literature (Halling AS et al. Real-world evidence of dupilumab efficacy and risk of adverse events: A systematic review and meta-analysis. J Am Acad Dermatol 2020)) and can be persistent despite adequate ophthalmological treatment (Achten R et al. Long-term follow-up and treatment outcomes of conjunctivitis during dupilumab treatment in patients with moderate-to-severe atopic dermatitis. J Allergy Clin Immunol Pract 2020). Additionally, the incidence of ocular complications with dupilumab, such as conjunctivitis, has been shown to increase with AD severity ( Simpson EL et al. Two Phase 3 Trials of Dupilumab versus Placebo in Atopic Dermatitis. N Engl J Med 2017;376:1090-1091; Thyssen JP et al. Incidence, prevalence, and risk of selected ocular disease in adults with atopic dermatitis. J Am Acad Dermatol 2017;77:280-286 e281). Ophthalmological side effects during dupilumab treatment have only been observed in patients with AD and not in studies of chronic sinusitis with nasal polyps, asthma or eosinophilic esophagitis (Bachert C et al. Effect of subcutaneous dupilumab on nasal polyp burden in patients with chronic sinusitis and nasal polyposis: a randomized clinical trial. JAMA 2016;315:469-479; Castro M et al. Dupilumab Efficacy and Safety in Moderate-to-Severe Uncontrolled Asthma. N Engl J Med 2018;378:2486-2496; Hirano I et al. Efficacy of dupilumab in a phase 2 randomized trial of adults with active eosinophilic esophagitis. Gastroenterology 2020;158:111-122.e110), suggesting that there are AD-specific predisposing factors. Since the introduction of dupilumab as a treatment for AD, conjunctivitis has been identified as an important adverse event, which has resulted in an increased collaboration with ophthalmologists (Agnihotri G et al. A Clinician's Guide to the Recognition and Management of Dupilumab-Associated Conjunctivitis. Drugs in R&D 2019;19:311-318; Wollenberg et al. 2018; Wollenberg A et al. Conjunctivitis occurring in atopic dermatitis patients treated with dupilumab-clinical characteristics and treatment. J Allergy Clin Immunol Pract 2018).

IL-13 is a 114 amino acid cytokine with an unmodified molecular mass of approximately 12 kDa. IL-13 is most closely related to IL-4 with which it shares 30% sequence homology at the amino acid level. The human IL-13 gene is located on chromosome 5q31 adjacent to the IL-4 gene. Although initially identified as a Th2 CD4 + lymphocyte derived cytokine, IL-13 is also produced by Th1 CD4+ T-cells, CD8+ T lymphocytes NK cells, and non-T-cell populations such as mast cells, basophils, eosinophils, macrophages, monocytes and airway smooth muscle cells. IL-13 has been linked with a number of diseases, in particular, diseases which are caused by an inflammatory response. For example, administration of recombinant IL-13 to the airways of naive non-sensitised rodents was shown to cause many aspects of the asthma phenotype including airway inflammation, mucus production and airways hyper-responsiveness. A similar phenotype was observed in a transgenic mouse in which IL-13 was specifically overexpressed in the lung. In this model, more chronic exposure to IL-13 also resulted in fibrosis.

A number of genetic polymorphisms in the IL-13 gene have also been linked to allergic diseases. In particular, a variant of the IL-13 gene in which the arginine residue at amino acid 130 is substituted with glutamine (Q130R) has been associated with bronchial asthma, atopic dermatitis and raised serum IgE levels. This particular IL-13 variant is also referred to as the Q110R variant (arginine residue at amino acid 110 is substituted with glutamine) by some groups who exclude the 20 amino acid signal sequence from the amino acid count.

Tralokinumab (also known as CAT-354 and BAK502G9) is a fully human therapeutic antibody that binds to and neutralizes IL-13, including the Q130R variant (see Popovic et al. J. Mol. Biol. (2017) 429(2): 208-219; May, R.D., Monk, P.D., Cohen, E.S., Manuel, D., Dempsey, F., Davis, N.H. et al. Preclinical development of CAT-354, an IL-13 neutralizing antibody, for the treatment of severe uncontrolled asthma. Br J Pharmacol. 2012; 166: 177-193).

Tralokinumab has previously been tested in phase 2b study of 204 adults for the treatment of AD - where patients received 45 mg, 150 mg, or 300 mg of subcutaneous tralokinumab, or placebo, every 2 weeks for 12 weeks with concomitant topical glucocorticoids - and was found to improve change from baseline in Eczema Area Severity Index (EASI) score, together with improvements in Scoring atopic dermatitis (SCORAD), Dermatology Life Quality Index (DLQI), and pruritus numeric rating scale scores, as compared to placebo (Wollenberg J. Allergy Clin. Immunol. (2019) 143(1):135-141).

There remains a desire in the art for further and improved treatments for AD that address, for example, at least some of the concerns referred to above.

### SUMMARY OF THE INVENTION

The inventors have found that a patient's response to the IL-13 binding protein tralokinumab is maintained when the dosing frequency of the antibody is decreased. Numerous advantages are associated with reducing dosing frequency, for example, improved patient compliance (e.g. less injections), reduction in the total amount of drug required per patient, and reduced clinician involvement (if, for example, the treatment cannot be self-administered). Patients can sometimes develop antibodies to a therapeutic protein, which neutralise the therapeutic effect. This tends not to be an issue for short term use (e.g. cancer therapy), but the likelihood increases with duration of use and drug exposure. Reducing drug exposure through dosing frequency may help prevent this effect. Increased drug exposure also increases the likelihood of side effects. Reduced dosing frequency may reduce side effects. It follows that tolerability (the balance between the efficacy of a therapeutic and its side effects) can also be improved.

Thus, in one aspect, the invention provides an interleukin-13 (IL-13) binding protein for use in a method of treating atopic dermatitis in a subject, wherein the method comprises the steps of: (i) administering prior doses of the IL-13 binding protein to the subject, wherein each prior dose is administered 2 weeks after the immediately preceding prior dose, wherein the first prior dose is around 600 mg and is administered to the subject at day 0, and each prior dose after the first prior dose is around 300 mg is administered to the subject at weeks 2, 4, 6, 8, 10, 12, 14 and 16; and wherein if the subject achieves an IGA score of 0 or 1 and/or ≥75% improvement of EASI-75 over baseline in the subject; (ii) administering a first dose of the IL-13 binding protein to the subject, wherein the first dose is around 300 mg and is administered to the subject at week 20; and (iii) administering secondary doses of the IL-13 binding protein to the subject, wherein each secondary dose of the IL-13 binding protein is administered to the subject 4 weeks after the immediately preceding dose, wherein each secondary dose is around 300 mg of IL-13 binding protein and is administered to the subject at weeks 24, 28, 32, 36, 40, 44, 48 and 52; and wherein the IL-13 binding protein is tralokinumab.

### DESCRIPTION OF FIGURES

**Figure 1****.** Proportion of patients (%) achieving IGA-0/1 and EASI-75 following 16 weeks treatment with tralokinumab/TCS or placebo/TCS (control). * = significant difference compared to control (p<0.05).
**Figure 2****.** Scores for patient reported outcomes at baseline and following 2 weeks of treatment with tralokinumab/TCS or placebo/TCS (control). ERSI = Eczema-Related Sleep Interference, POEM = Patient-Oriented Eczema Measure, DLQI = Dermatology Life Quality Index. * = significant difference compared to control (p<0.05).
**Figure 3****.** Proportion of patients (%) achieving IGA-0/1 following 16 weeks of treatment with tralokinumab monotherapy or placebo (control) in two trials, ECZTRA 1 and ECZTRA 2. * = significant difference compared to control (p<0.05).
**Figure 4****.** Proportion of patients (%) achieving EASI-75 following 16 weeks of treatment with tralokinumab monotherapy or placebo (control) in two trials, ECZTRA 1 and ECZTRA 2. * = significant difference compared to control (p<0.05).

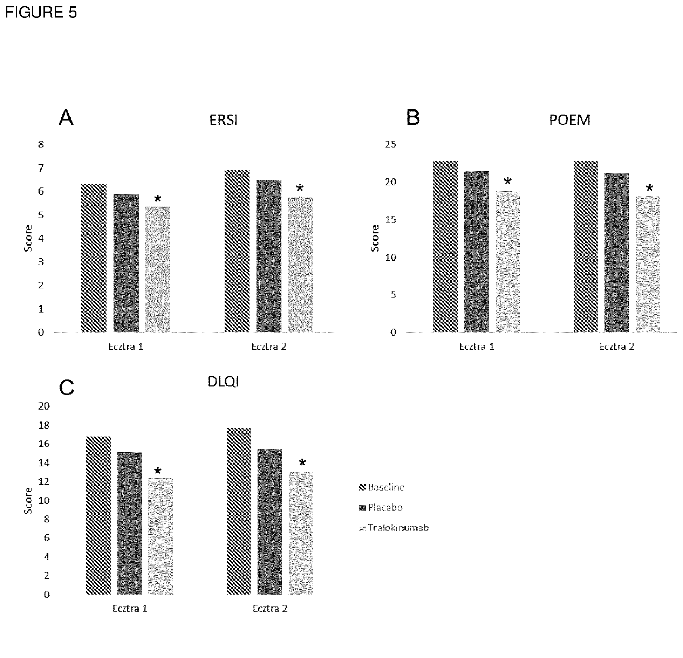

**Figure 5****.** Scores for patient reported outcomes at baseline and following 2 weeks of treatment tralokinumab monotherapy or placebo (control) in two trials, ECZTRA 1 and ECZTRA 2. * = significant difference compared to control (p<0.05). 5A ERSI = Eczema-Related Sleep Interference scores. 5B POEM = Patient-Oriented Eczema Measure scores. 5C DLQI = Dermatology Life Quality Index scores.
**Figure 6****.** Proportion of patients (%) achieving IGA-0/1 and EASI-75 at week 32. Patients were responders to tralokinumab/TCS treatment at week 16. At week 16 responders were split into two groups and treated with tralokinumab/TCS every 2 weeks (Q2W) or 4 weeks (Q4W) for the remainder of the study.

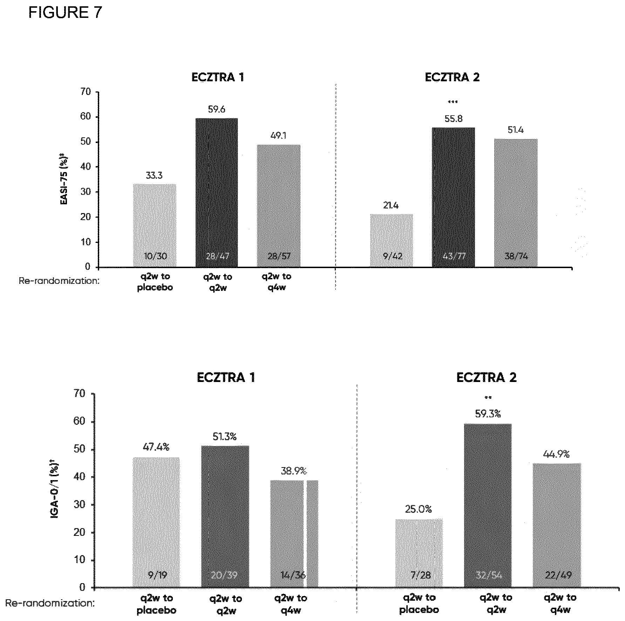

**Figure 7****.** Proportion of patients (%) achieving IGA-0/1 and EASI-75 at week 52 of ECZTRA 1 and ECZTRA 2 trials. Patients were responders to tralokinumab treatment at week 16. At week 16 responders were split into groups and treated with tralokinumab every 2 weeks (Q2W) or 4 weeks (Q4W) for the remainder of the study. ^{†}Assessed in patients achieving IGA-0/1 at week 16 without use of rescue medication after initial randomization to tralokinumab;^{‡}Assessed in patients achieving EASI-75 at week 16 without use of rescue medication after initial randomization to tralokinumab. Patients who, after week 16, received rescue medication or were transferred to open-label treatment were considered nonresponders at week 52; Missing values imputed as nonresponse.
**Figure 8****.** Scores for secondary endpoints (SCORAD, pruritus, DLQI and EASI) at baseline and following 16 weeks treatment with tralokinumab or placebo (control) in ECZTRA 1 and ECZTRA 2 trials. SCORAD = Scoring atopic dermatitis (SCORAD); DLQI = Dermatology Life Quality Index; EASI = Eczema Area and Severity Index.
**Figure 9****.** Mean percent change from baseline in worst Daily Pruritus NRS (weekly average from week 0-16).
**Figure 10****.** Achievement of EASI-50 and EASI-90 following 16 weeks treatment with tralokinumab/TCS or placebo/TCS (control) (ECZTRA 3 trial). Patients with missing data imputed as nonresponders. *p<0.05 versus placebo + TCS; **p<0.01 versus placebo + TCS; ***p<0.001 versus placebo + TCS. EASI-50, at least 50% reduction in EASI score; EASI-90, at least 90% reduction in EASI score.
**Figure 11****.** Eczema-Related Sleep Interference (ERSI) scores following 16 weeks of treatment in the ECZTRA 1-3 trials. *P<0.05; ** *P<0.01; **P<0.001.*
**Figure 12****.** Patient-Oriented Eczema Measure (POEM) scores following 16 weeks of treatment in the ECZTRA 1-3 trials. *P<0.05; ** *P<0.01; **P<0.001.*
**Figure 13****.** Adjusted mean change from baseline in HADS total score by Week for the pivotal phase 3 trials and ECZTRA 1+2, initial treatment period: FAS.
**Figure 14****.** Proportion of patients in the ECZTRA1+2 trials reporting sleep interference no days, 1-2 days, 3-4 days, 5-6 days or every day.
**Figure 15****.** Comparison of S*.aureus* colonisation in EASI-75 responders from tralokinumab and placebo groups.

### DETAILED DESCRIPTION

The invention relates to methods for treating atopic dermatitis in a subject using the anti-IL-13 antibody tralokinumab.

### Atopic Dermatitis

"Atopic dermatitis" (AD), as used herein, means an inflammatory skin disease characterized by intense pruritus (e.g. severe itch) and by scaly and dry eczematous lesions.

The term "atopic dermatitis" includes AD caused by or associated with epidermal barrier dysfunction, allergy (e.g. allergy to certain foods, pollen, maid, dust mite, animals, etc.), radiation exposure, and/or asthma. In some embodiments, the present invention relates to moderate-to-severe or severe AD.

As used herein, "moderate-to-severe AD" is characterized by intensely pruritic, widespread skin lesions that are often complicated by persistent bacterial, viral or fungal infections. Moderate-to-severe AD also includes chronic AD. In many cases, the chronic lesions include thickened plaques of skin, lichenification and fibrous papules. In general, patients affected by moderate-to-severe AD also have more than 20% of the body's skin affected, or 10% of skin area in addition to involvement of the eyes, hands and body folds. Moderate-to-severe AD is also considered to be present in patients who frequently require treatment with a topical corticosteroid. In the clinical studies reported herein a subject with "moderate to severe AD" was a subject having an IGA score of 3-4.

As used herein, "severe AD" refers to chronic relapsing AD that is refractory to treatment with medium-potency and high-potency TCS and/or immunosuppressant therapy. Severe AD is also characterized by chronic intensely pruritic lesions affecting more than 20% of the body surface area. Severe AD can be considered to be present in subjects with chronic AD according to the Eichenfield criteria (Eichenfield et al 2014, J. Am. Acad. Dermatol. 70: 338-351), for which treatment with a potent topical corticosteroid (TCS) is indicated, and/or where the subject is resistant to treatment with a systemic corticosteroid and/or non-steroidal immunosuppressant. In the clinical studies reported herein a subject with "severe AD" was a subject having an IGA score of 4. Thus, in certain embodiments, the method treats severe AD in a subject, where the subject has an IGA score of 4 at baseline. A subject with "severe AD" may have AD on at least 10% of their body surface area at screening and baseline, an EASI score of ≥20 at screening and baseline, and an IGA score of ≥3 at screening and baseline.

### Subject

As used herein, the term "subject" includes human and non-human animals, particularly mammals. Typically, the subject is a human, as shown in the examples below.

A subject with AD (especially moderate-to-severe AD or severe AD) may be resistant, non-responsive or inadequately responsive to treatment with a non-steroid systemic immunosuppressant. The term "non-steroid systemic immunosuppressant" includes cyclosporine A (CSA), methotrexate, mycophenolate mofetil, azathioprine, and interferon-gamma. In certain embodiments, the term also includes immunobiologics such as tumor necrosis factor alpha (TNFa) inhibitors (e.g. an anti-TNFa antibody such as infliximab), CD11a inhibitors (e.g. an anti-CD11a antibody such as efalizumab), IgE inhibitors (e.g. omalizumab), CD20 inhibitors (e.g. rituximab). Thus, in some cases, the methods described herein may treat AD in subjects that are resistant, nonresponsive (refractory) or inadequately responsive to treatment with a systemic immunosuppressant. The term "resistant, non-responsive or inadequately responsive to a systemic immunosuppressant" refers to a subject with AD that has been treated with a systemic immunosuppressant and the immunosuppressant did not have a therapeutic effect, e.g. a subject with moderate-to-severe AD or severe AD (such as those with chronic relapsing AD) that has been treated with a non-steroid systemic immunosuppressant for between 1-3 months and did not show a decrease in one or more AD-associated parameter score(s). The time for the assessment of a therapeutic effect will vary depending on the typical timeframe for onset of action of the non-steroid systemic immunosuppressant. Such timeframes are well known. For example, for cyclosporine the onset of action is typically 2-6 weeks, but for other non-steroid systemic immunosuppressants it is typically around 8-12 weeks.

In some embodiments, immunosuppressant treatment has been deemed not medically advisable by a physician for the subject. Such a subject may be identified by the following criteria: (1) no prior immunosuppressant exposure; (2) not currently a candidate for immunosuppressant treatment due to: medical contraindication(s); or hypersensitivity to the immunosuppressant or excipient(s); use of concomitant medications prohibited with immunosuppressant; or increased susceptibility to immunosuppressant induced renal damage or increased risk of serious infections; (3) previous intolerance and/or unacceptable toxicity on previous exposure to an immunosuppressant; and/or (4) requirement for immunosuppressant at doses or duration beyond that specified in the prescribing information.

In any of the methods described herein for the treatment of atopic dermatitis, e.g. severe atopic dermatitis, the subject may be one in which the atopic dermatitis is not adequately controlled by cyclosporine A (CSA), e.g. oral cyclosporine A, or the subject has contraindications to cyclosporine A (CSA), e.g. oral cyclosporine A.

An inadequate response to CSA is defined as flare of AD on CSA tapering after a maximum of 6 weeks of high dose (5 mg/kg/day) to maintenance dose (2 to 3 mg/kg/day) or a flare after a minimum of 3 months on maintenance dose. Flare is defined as increase in signs or symptoms leading to escalation of therapy, which could be an increase in dose, a switch to a higher potency class of TCS, or the start of another systemic non-steroidal immunosuppressive drug.

Contraindications to CSA include:
i. medical contraindications (e.g. uncontrolled hypertension on medication) or hypersensitivity to CSA active substance or excipients;
ii. use of prohibited concomitant medications (e.g. statins, digoxin, macrolide, antibiotics, barbiturates, anti-seizure, non-steroidal anti-inflammatory drugs, diuretics, angiotensin-converting-enzyme inhibitors, St John's Wort);
iii. increased susceptibility to CSA-induced renal damage (elevated creatinine) and liver damage (elevated function tests), or increased risk of serious infections;
iv. intolerance or unacceptable toxicity (e.g. elevated creatinine, elevated liver function tests, uncontrolled hypertension, paraesthesia, headache, nausea, hypertrichosis), or
v. requirement for CSA at doses >5 mg/kg/day, or duration beyond those specified in the prescribing information (>1 year).

### Treatment of AD

The methods described herein treat AD. Generally, the terms "treat", "treating", "treatment", or the like, mean to alleviate (reduce, minimise, or eliminate) symptoms, or to reduce, minimise or eliminate the causation of symptoms either on a temporary or permanent basis.

### AD-associated parameters

Various AD-associated parameters are available to measure the severity of AD and the impact of a drug on AD. These include Investigators Global Assessment (IGA); Eczema Area and Severity Index (EASI); SCORing Atopic Dermatitis (SCORAD); and/or pruritus Numeric Rating Scale (NRS). The methods described herein may improve in an AD-associated parameter in the subject. Alternately, the methods may maintain improvement in an AD-associated parameter in the subject. The AD-associated parameter may be selected from: Investigators Global Assessment (IGA); Eczema Area and Severity Index (EASI); Scoring atopic dermatitis (SCORAD); and/or pruritus Numeric Rating Scale (NRS).

The IGA is an instrument used in clinical trials to rate the severity of the subject's global AD and is based on a 5-point scale ranging from 0 (clear) to 4 (severe) based on the condition of the disease at the time of evaluation.

| **Score** | **Disease severity** | **Standard IGA scale** | **IGA morphological descriptors** |
|---|---|---|---|
| 0 | Clear | No inflammatory signs of atopic dermatitis | No erythema and no elevation (papulation/infiltration). |
| 1 | Almost clear | Just perceptible erythema, and just perceptible papulation/infiltration | Barely perceptible erythema and/or minimal lesion elevation (papulation/infiltration) that is not widespread. |
| 2 | Mild disease | Mild erythema and mild papulation/infiltration | Visibly detectable, light pink erythema and very slight elevation (papulation/infiltration). |
| 3 | Moderate disease | Moderate erythema and moderate papulation/infiltration | Dull red, clearly distinguishable erythema and clearly perceptible but not extensive elevation (papulation/infiltration). |
| 4 | Severe disease | Severe erythema and severe papulation/infiltration | Deep/dark red erythema, marked and extensive elevation (papulation/infiltration). |

The EASI is a validated measure used in clinical practice and clinical trials to assess the severity and extent of AD (Hanifin et al. "The eczema area and severity index (EASI): assessment of reliability in atopic dermatitis. EASI Evaluator Group. Experimental dermatology" (2001) 10(1): 11-18). SCORAD is one of the most commonly used disease severity scores in clinical trials with AD and in clinical practice (see *"*European Task Force on Atopic Dermatitis. Severity scoring of atopic dermatitis: the SCORAD index. Consensus report of the European task force on atopic dermatitis" Dermatology (1993) 186(1): 23-31).

Worst Daily Pruritus NRS is established according to FDA and EMA recommendations (see, e.g. FDA "The Food and Drug Administration. Guidance for Industry. Patient-Reported Outcome Measures: Use in Medical Product Development to Support Labeling Claims. 2009" and EMA "Reflection paper on the regulatory guidance for the use of health-related quality of life (HRQoL) measures in the evaluation of medicinal products. EMEA/CHMP/EWP/139391/2004. 2005). For pruritus NRS, a subject assesses their worst itch severity over the past 24 hours using an 11 point NRS ("Worst Daily Pruritus NRS") from 0 (no itch) to 10 (worst itch imaginable).

For each AD-associated parameter the improvement or maintained improvement is measured relative to baseline. An improvement in this context can be a reduction in IGA score, a reduction in EASI score, a reduction in SCORAD score (where >50 severe, 25-50 is moderate, <25 is mild), reduction in pruritus NRS score, where each score is compared to baseline.

The baseline is an initial measurement of an AD-associated parameter or patient-related outcome (or any other parameter) that is taken before initiation of treatment by the method described herein, i.e. a measurement taken before the "baseline dose" (defined elsewhere).

An Investigator's Global Assessment 0 or 1 (IGA 0/1; clear or almost clear skin) and/or ≥ 75% improvement of Eczema Area and Severity Index (EASI-75) are the regulatory primary efficacy endpoints in Phase 3 clinical trials in AD. Thus, the methods described herein may preferably achieve or maintain an Investigator's Global Assessment (IGA) score of 0 or 1 and/or ≥ 75% improvement of Eczema Area and Severity Index (EASI-75) over baseline (e.g. as shown in Examples 1 and 4 herein).

Additionally, or alternatively, the methods described herein may improve at least one patient-related outcome (PRO) selected from the group consisting of: worst daily pruritus Numerical Rating Scale (NRS) (see pruritus NRS discussed above), eczema-related sleep interference, Patient Oriented Eczema Measure (POEM), Dermatology Life Quality Index (DLQI), Patient Global Impression of Bother (PGI-B), Hospital Anxiety and Depression Scale (HADS), Short Form (36) Health Survey (SF-36) and EuroQoL 5-Dimension Health Questionnaire 5 Level (EQ-5D-5L) .

For eczema-related sleep interference NRS, a subject rates how much their eczema interfered with their sleep the previous night using an 11 point NRS from 0 (no interference) to 10 (complete interference). The POEM is a validated questionnaire used to assess disease symptoms in AD patients in both clinical practice and clinical trials (see Charman et al. "The patient-oriented eczema measure: development and initial validation of a new tool for measuring atopic eczema severity from the patients perspective" Arch Dermatol. (2004) 140(12): 1513-1519). DLQI is a patient-reported validated questionnaire with content specific to subjects with dermatology conditions (see Finlay et al. "Dermatology Life Quality Index (DLQI) - a simple practical measure for routine clinical use" Clin Exp Dermatol. (1994) 19(3): 210-216). The Patient Global Impression of Bother (PGI-B) is designed to capture the subject's perception of how bothered they have been by their AD over the past 24 hours at the time of completion. A 5-point categorical response scale will be used ('not at all', 'slightly', 'somewhat', 'a lot', 'very much'). The Hospital Anxiety and Depression Scale (HADS) is a Likert-scale tool widely used to detect states of anxiety and depression in a general hospital setting (see Zigmond AS, Snaith RP. "The hospital anxiety and depression scale". Acta Psychiatr Scand. 1983;67(6):361-70). The tool consists of 14 items that assess the subject's anxiety (7 items) and depression (7 items) during the last week. Each item is scored from 0 to 3, with high scores indicating more severe anxiety or depression. Short Form (36) Health Survey (SF-36) is a patient-reported survey designed to evaluate health status by generating scores for 8 health domains (physical functioning, role physical, bodily pain, general health, vitality, social functioning, role emotional, and mental health) and 2 psychometrically derived summary scores (a physical component summary and a mental component summary). (see Ware JEJ, Sherbourne CD. "The MOS 36-item short-form health survey (SF-36). I. Conceptual framework and item selection" Med Care. 1992;30(6):473-83). EuroQoL 5-Dimension Health Questionnaire 5 Level (EQ-5D-5L) is a standardised measure of health status developed by the EuroQoL group to provide a simple, generic measure of health for clinical and economic appraisal (see Greiner W et al. "A single European currency for EQ-5D health states. Results from a six-country study" The European journal of health economics: HEPAC : health economics in prevention and care. 2003;4(3):222-31). The EQ-5D-5L is a self-administered questionnaire used to assess health status 'today' and is divided into 2 sections: The first section includes 5 dimensions (mobility, self-care, usual activity, pain/discomfort, and anxiety/depression); each dimension is assessed by the subject using a 5-point scale ('no problems', 'slight problems', 'moderate problems', 'severe problems', and 'extreme problems'). The second section consists of a vertical visual analogue scale anchored at 0 ('the worst health you can imagine') and 100 ('the best health you can imagine').

The methods may maintain improvement in at least one patient-related outcome (PRO) selected from the group consisting of: worst daily pruritus Numerical Rating Scale (NRS), eczema-related sleep interference, Patient Oriented Eczema Measure (POEM), Dermatology Life Quality Index (DLQI), Patient Global Impression of Bother (PGI-B), Hospital Anxiety and Depression Scale (HADS), Short Form (36) Health Survey (SF-36) and EuroQoL 5-Dimension Health Questionnaire 5 Level (EQ-5D-5L). For each PRO, the improvement or maintained improvement is relative to baseline. An improvement in this context can be a reduction (e.g. a ≥ 3 point reduction) in the PRO score (or, for example, a ≥ 4 point reduction for DLQI), where the score is compared to baseline.

The methods described herein may achieve: (a) ≥ 50% improvement of Eczema Area and Severity Index (EASI-50); (b) ≥ 2 point reduction of IGA score; (c) ≥ 3 point reduction in pruritus NRS; (d) ≥ 4 point reduction in POEM score; (e) ≥ 4 point reduction in DLQI score; (f) ≥ 0.4 point reduction in eczema-related sleep interference; (g) ≥ 1-point reduction in PGI-B score; (h) ≥ 2 point or ≥ 3 point reduction in HADS score (i) ≥ 4 point increase in SF-36 Physical Component Summary Score and/or ≥ 2 point increase in SF-36 Mental Component Summary Score; and/or (j) ≥0.2 point increase in EQ-5D-5L index score. The methods described herein may maintain: (a) ≥ 50% improvement of Eczema Area and Severity Index (EASI-50); (b) ≥ 2 point reduction of IGA score; (c) ≥ 3 point reduction in pruritus NRS; (d) ≥ 4 point reduction in POEM score; (e) ≥ 4 point reduction in DLQI score; (f) ≥ 0.4 point reduction in eczema-related sleep interference; (g) ≥ 1-point reduction in PGI-B score; (h) ≥ 2 point or ≥ 3 point point reduction in HADS score; (i) ≥ 4 point increase in SF-36 Physical Component Summary Score and/or ≥ 2 point increase in SF-36 Mental Component Summary Score; and/or (j) ≥0.2 point increase in EQ-5D-5L index score.

For example, the methods described herein may achieve one or more (in particular all) of the following: (a) ≥ 50% improvement of Eczema Area and Severity Index (EASI-50); (b) ≥ 2 point reduction of IGA score; (c) ≥ 4 point reduction in POEM score; and (d) ≥ 4 point reduction in DLQI score. The methods may maintain one or more (or all) of the following: (a) ≥ 50% improvement of Eczema Area and Severity Index (EASI-50); (b) ≥ 2 point reduction of IGA score; (c) ≥ 4 point reduction in POEM score; and (d) ≥ 4 point reduction in DLQI score.

### TCS dependence

Long-term application of a TCS is not recommended because of the risk of skin atrophy, dyspigmentation, acneiform eruptions, and risks associated with systemic absorption (e.g. hypothalamic pituitary axis effects, Cushing's disease, etc.). Repeated application of any topical therapy over a long period of time or to large surface areas can also lead to reduced patient compliance.

The methods described herein may reduce the topical corticosteroid (TCS) dependence of the subject with AD (especially moderate-to-severe or severe AD).

Reduced dependence may be assessed by comparing the cumulative amount (in grams) of a formulation containing TCS applied by a subject after initiation of a method described herein over a particular time interval (e.g. 16 weeks), as compared to a placebo-treated subject. For example, a subject may use at least 0.2 g less, at least 0.3 g less, at least 0.4 g less or at least 0.5 g less TCS per day, as compared to a placebo-treated subject. Typically, a subject may use at least 0.5 g less TCS per day, as compared to a placebo-treated subject (e.g. as in Example 1).

Reduced dependence may also be assessed by the number of TCS-free days (which may still include lower potency TCS and TCI) after initiation of the method, as compared to the same measurement performed at baseline.

A TCS can be classified as group I, group II, group III and group IV topical corticosteroid. According to the Anatomical Therapeutic Classification System of World Health Organization, corticosteroids are classified as weak/lower potency (group I), moderately potent (group II) and potent (group III) and very potent (group IV), based on their activity as compared to hydrocortisone. Group IV TCS (very potent) are up to 600 times as potent as hydrocortisone and include clobetasol propionate and halcinonide. Group III TCS (potent) are 50 to 100 times as potent as hydrocortisone and include betamethasone valerate, betamethasone dipropionate, diflucortolone valerate, hydrocortisone-17-butyrate, mometasone furoate, and methylprednisolone aceponate. Group II TCS (moderately potent) are 2 to 25 times as potent as hydrocortisone and include clobetasone butyrate, and triamcinolone acetonide. Group I TCS (mild) includes hydrocortisone.

The term "TCS-free day" means a day in which the subject does not use a TCS of Group II, Group III or Group IV.

For example, the subject the number of TCS-free days may increase by about 0.5 day, about 0.75 day, about 1 day, about 1.5 days, about 2 days, about 3 days or more averaged over a week, as compared to a placebo-treated subject. Typically, the number of TCS-free days may increase by about 0.5 day, as compared to a placebo-treated subject (e.g. as illustrated in Example 1).

As shown in the examples below, tralokinumab can be administered as a monotherapy, i.e. without TCS, as so can be used to wean a subject off TCS use. Accordingly, in the methods described herein a medium-potency or high-potency TCS may be administered alongside tralokinumab. The amount of TCS can then be reduced by at least 20%, at least 30%, at least 40%, at least 50%, at least 60%, at least 70%, at least 80%, at least 90%, at least 95%, or around 100% after initiation of the method (e.g. over a 3-4 month period), as compared to the amount of TCS at baseline.

### Treatment of a skin infection

Some of the methods described herein may treat a skin infection. Generally, the terms "treat", "treating", "treatment", or the like, mean to alleviate (reduce, minimise, or eliminate) symptoms, or to reduce, minimise or eliminate the causation of symptoms either on a temporary or permanent basis.

The patient to be treated has a microbial skin infection, such as a bacterial infection, a fungal infection or a viral infection. For example, the skin infection may be *Staphylococcus aureus* infection, *Streptococcus* infection, impetigo, cellulitis, infected dermatitis, eczema herpeticum, folliculitis, infected blister, mycosis and tinea versicolor.

In some examples, the IL-13 binding protein is not administered in combination with a topical corticosteroid. "Not administered in combination with" means "not administered in the same course of treatment". In some examples, the IL-13 binding protein is administered as a monotherapy for treating skin infection in a subject having moderate to severe AD.

In some examples, the IL-13 binding agent is administered with a second therapeutic agent, such as an anti-bacterial agent, an anti-viral agent, an anti-fungal agent, another IL-13 antagonist, an IgE inhibitor, a non-steroid anti-inflammatory drug (NSAID) or interferon γ (IFNy). The second therapeutic agent may be administered to the subject before, after or concurrently with the IL-13 binding protein.

### Treatment of pruritus

Some of the methods described herein may treat pruritus (i.e. itching).

Treatment of pruritus means a reduction of Worst Daily Pruritus Numerical Rating Score (NRS) compared to baseline, e.g. before treatment. In some examples, treatment of pruritus is characterised by a ≥ 1-point reduction, a ≥ 2-point reduction, a ≥ 3-point reduction, or a ≥ 4-point reduction in Worst Daily Pruritus NRS from baseline e.g. before treatment.

Worst Daily Pruritus NRS is established according to FDA and EMA recommendations (see, e.g. FDA "The Food and Drug Administration. Guidance for Industry. Patient-Reported Outcome Measures: Use in Medical Product Development to Support Labeling Claims. 2009" and EMA "Reflection paper on the regulatory guidance for the use of health-related quality of life (HRQoL) measures in the evaluation of medicinal products. EMEA/CHMP/EWP/139391/2004. 2005). For pruritus NRS, a subject assesses their worst itch severity over the past 24 hours using an 11 point NRS ("Worst Daily Pruritus NRS") from 0 (no itch) to 10 (worst itch imaginable).

In some examples, the IL-13 binding protein is not administered in combination with a topical corticosteroid. "Not administered in combination with" means "not administered in the same course of treatment". In some examples, the IL-13 binding protein is administered as a monotherapy for treating pruritus in a subject having moderate to severe AD.

In some examples, the subject may be one in which the atopic dermatitis is not adequately controlled by cyclosporine A (CSA), e.g. oral cyclosporine A, or the subject has contraindications to cyclosporine A (CSA), e.g. oral cyclosporine A.

### Treatment of eczema-related sleep interference

Some of the methods described herein may treat eczema-related sleep interference.

For eczema-related sleep interference numerical rating score (NRS), a subject rates how much their eczema interfered with their sleep the previous night using an 11 point NRS from 0 (no interference) to 10 (complete interference).

Treatment of eczema-related sleep interference means a reduction of eczema-related sleep interference NRS compared to baseline e.g. before treatment. In some examples, treatment of eczema-related sleep interference NRS is characterised by a ≥ 0.4-point reduction, a ≥ 1-point reduction, a ≥ 2-point reduction, or a ≥ 4-point reduction in eczema-related sleep interference NRS from baseline, e.g. before treatment.

### Treatment of anxiety and depression

Some of the methods described herein may treat anxiety and/or depression.

To assess anxiety and/or depression using HADS, a patient scores fourteen items on a questionnaire from 0-3, resulting in a total score of 0 to 21 for either anxiety or depression.

Treatment of anxiety and/or depression means a reduction in HADS score compared to baseline e.g. before treatment, for example a reduction in HADS anxiety score and/or HADS depression score. In some examples, treatment of anxiety and/or depression is characterised by a ≥ 1-point reduction, a ≥ 2-point reduction, a ≥ 3-point reduction, or a ≥ 4-point reduction in HADS score from baseline, e.g. before treatment.

In some examples, the methods described herein treat anxiety and/or depression in a subject having AD, for example moderate to severe AD. In some examples, the subject may have a baseline (e.g. before treatment) HADS score of ≥8, for example a baseline HADS anxiety score and/or HADS depression score of ≥8.

### Treatment of patients experiencing conjunctivitis

The methods described herein may be used to treat atopic dermatitis in a subject who has experienced conjunctivitis when treated with tralokinumab in combination with a topical corticosteroid.

The methods described herein may be used to treat atopic dermatitis in a subject who has experienced conjunctivitis when treated with an anti-IL4Rα antibody or an antibody that inhibits IL4/IL13 signalling, e.g. dupilumab. In some examples, the subject has discontinued dupilumab therapy as a result of experiencing conjunctivitis.

### IL-13 binding protein

An IL-13 binding protein is a protein that specifically binds to and neutralizes human IL-13.

Herein, the term "specifically binds" means that a protein (such as an antibody or antigen-binding fragment thereof) forms a complex with an antigen that is relatively stable under physiological conditions. Methods for determining whether a protein specifically binds to an antigen are well known in the art and include, for example, equilibrium dialysis, surface plasmon resonance (e.g. using a BIAcore 200 Biosensor (BIAcore AB), and the like. For example, an IL-13 binding protein (e.g. an anti-IL-13 antibody or IL-13 binding fragment thereof) that "specifically binds" IL-13 may bind IL-13 with a K_{D} of less than about 1000 nM, less than about 500 nM, less than about 100 nM, less than about 50 nM, less than about 20 nM, less than about 10 nM, less than about 5 nM, less than about 1 nM, less than about 0.5 nM, less than about 0.25 nM, less than about 0.1 nM or less than about 0.05 nM, as measured by surface plasmon resonance at 25°C. The exemplified antibody, tralokinumab binds human bound human IL-13 with a K_{D} of 178 pM, as measured by surface plasmon resonance (see WO 2005/007699 for detailed methods). Although an IL-13 binding protein specifically binds human IL-13, it may have cross-reactivity to other antigens, such as IL-13 from other (non-human) species.

Methods for measuring neutralisation activity are well known in the art. Neutralisation activity can be measured in an IL-13 dependent TF-1 cell proliferation assay relative to a control antibody that is not directed to IL-13, as described in WO 2005/007699. In this assay, inhibition of IL-13 dependent proliferation is determined by measuring the reduction in incorporation of tritiated thymidine into the newly synthesized DNA of dividing cells. Briefly, commercial TF-1 cells are maintained according to supplied protocols. Assay media comprises RPMI-1640 with GLUTAMAX I (Invitrogen) containing 5% FBS and 1% sodium pyruvate. Prior to each assay, TF-1 cells are pelleted by centrifugation at 300 x g for 5 minutes, the media removed by aspiration and the cells resuspended in assay media.

This process is repeated twice with cells resuspended at a final concentration of 10⁵ cells/mL in assay media. Test solutions of antibody (in triplicate) are diluted to the desired concentration in assay media. An antibody that is not directed at IL-13 is used as a negative control. Recombinant bacterially derived human or murine IL-13 is added to a final concentration of 50 ng/mL when mixed with the appropriate test antibody in a total volume of 100 µL/well in a 96 well assay plate. The concentration of IL-13 used in the assay is selected as the dose that at final assay concentration gives approximately 80% of the maximal proliferative response. All samples are incubated for 30 minutes at room temperature. 100 µL of resuspended cells are then added to each assay point to give a total assay volume of 200 µL/well. Assay plates are incubated for 72 hours at 37°C under 5% CO₂. 25 µL of tritiated thymidine (10 µCi/mL) is then added to each assay point and assay plates are returned to the incubator for a further 4 hours. Cells are harvested on glass fibre filter plates (Perkin Elmer) using a cell harvester. Thymidine incorporation is determined using a Packard TopCount microplate liquid scintillation counter.

### Anti-IL-13 antibodies and IL-13-binding thereof

The term "antibody", as used herein, includes immunoglobulin molecules comprising four polypeptide chains, two heavy (H) chains and two light (L) chains inter-connected by disulfide bonds, as well as multimers thereof (e.g. IgM). In a typical antibody, each heavy chain comprises a heavy chain variable region (abbreviated herein as HCVR or V_{H}) and a heavy chain constant region. The heavy chain constant region comprises three domains, C_{H}1, C_{H}2 and C_{H}3. Each light chain comprises a light chain variable region (abbreviated herein as LCVR or V_{L}) and a light chain constant region. The light chain constant region comprises one domain (C_{L}1). The V_{H} and V_{L} regions can be further subdivided into regions of hypervariability, termed complementarity determining regions (CDRs), interspersed with regions that are more conserved, termed framework regions (FR). Each VH and VL is composed of three CDRs and four FRs, arranged from amino-terminus to carboxy-terminus in the following order: FR1, CDR1, FR2, CDR2, FR3, CDR3, FR4. In some cases, the FRs of the anti-IL-13 antibody (or IL-13-binding fragment or derivative thereof) may be identical to the human germline sequences, or may be naturally or artificially modified.

The heavy chain constant region of the antibodies may be from any types of constant region, such as IgG, IgM, IgD, IgA, and IgE. Generally, the antibody is an IgG (e.g. isotype IgG1, IgG2, IgG3 or IgG4). Preferably, the antibody is an IgG4, as exemplified herein.

An antibody may be a mouse, human, primate, humanized or chimeric antibody. An antibody may be polyclonal or monoclonal. For therapeutic applications, monoclonal and human (or humanized) antibodies are preferred.

An antibody can be a multispecific (e.g. bispecific) antibody. A multispecific antibody or antigen-binding fragment of an antibody will typically comprise at least two different variable domains, wherein each variable domain is capable of specifically binding to a separate antigen or to a different epitope on the same antigen. Any multispecific antibody format may be adapted for use in the context of an antibody or antigen binding fragment of an antibody as described herein using routine techniques available in the art. For example, the methods that use of bispecific antibodies, wherein one arm of an immunoglobulin is specific for IL-13, and the other arm of the immunoglobulin is specific for a second therapeutic target or is conjugated to a therapeutic moiety.

An IL-13-binding fragment of an anti-IL-13 antibody may be any naturally occurring, enzymatically obtainable, synthetic, or genetically engineered polypeptide. Such fragments may be derived, e.g. from full antibody molecules using any suitable standard techniques such as proteolytic digestion or recombinant genetic engineering techniques involving the manipulation and expression of DNA encoding antibody variable and optionally constant domains. Such DNA is known and/or is readily available from, e.g. commercial sources, DNA libraries (including, e.g. phage-antibody libraries), or can be synthesized. The DNA may be sequenced and manipulated chemically or by using molecular biology techniques, for example, to arrange one or more variable and/or constant domains into a suitable configuration, or to introduce codons, create cysteine residues, modify, add or delete amino acids, etc.

Non-limiting examples of IL-13-binding fragments include: Fab, Fab', F(ab')2, Fd, Fv, single-chain Fv (scFv), disulphide-linked Fvs, dAb fragments, and other engineered molecules, such as domain-specific antibodies, single domain antibodies, domain-deleted antibodies, chimeric antibodies, CDR-grafted antibodies, diabodies, triabodies, tetrabodies, minibodies, nanobodies (e.g. monovalent nanobodies, bivalent nanobodies, etc.), small modular immunopharmaceuticals (SMIPs), and shark variable IgNAR domains.

An IL-13-binding fragment of an anti-IL-13-binding antibody will typically comprise at least one variable domain. The variable domain may be of any size or amino acid composition and will generally comprise at least one CDR which is adjacent to or in frame with one or more framework sequences. In antigen-binding fragments having a V_{H} domain associated with a V_{L} domain, the V_{H} and V_{L} domains may be situated relative to one another in any suitable arrangement. For example, the variable region may be dimeric and contain V_{H}-V_{H}, V_{H}-V_{L} or V_{L}-V_{L} dimers. Alternatively, the antigen-binding fragment of an antibody may contain a monomeric V_{H} or V_{L} domain.

The antibody used in the methods described herein is the anti-IL-13 antibody, tralokinumab (as described in the *"*International Nonproprietary Names for Pharmaceutical Substances (INN)" list 102 (WHO Drug Information (2009) 23(4): pp 348)). Tralokinumab is a fully human IgG4-lambda antibody, which specifically binds and neutralises human IL-13.

**Table 1**

| **SEQ ID number** | **Name** | **Sequence** |
|---|---|---|
| Tralokinumab | | |
| SEQ ID NO: 1 | HCDR1 | NYGLS |
| SEQ ID NO: 2 | HCDR2 | WISANNGD TNYGQEF QG |
| SEQ ID NO: 3 | HCDR3 | DSSSSWARWFFDL |
| SEQ ID NO: 4 | LCDR1 | GGNIIGSKLVH |
| SEQ ID NO: 5 | LCDR2 | DDGDRPS |
| SEQ ID NO: 6 | LCDR3 | QVWDTGSDPVV |
| SEQ ID NO: 7 | cDNA heavy chain variable domain | caggtccagctggtgcagtctggggctgaggtgaagaagcctgggg cctcagtgaaggtctcctgcaaggcttctggttacacctttacaaattat ggtctcagctgggtgcgacaggcccctggacaagggcttgagtggat gggatggatcagcgctaataatggcgacacaaattatggacaggaatt |
| | | ccagggcagagtcaccatgaccacagatacatccacgagcacagcc tacatggagttgaggagcctgagatctgacgacacggccgtttattact gtgcgagagactccagcagcagctgggcccgctggtttttcgatctct ggggccgggggacactggtcaccgtctcctca |
| SEQ ID NO: 8 | polypeptide sequence heavy chain variable region | QVQLVQSGAEVKKPGASVKVSCKASGYTFT NYGLSWVRQAPGQGLEWMGWISANNGDTN YGQEFQGRVTMTTDTSTSTAYMELRSLRSD DTAVYYCARDSSSSWARWFFDLWGRGTLV TVSS |
| SEQ ID NO: 9 | cDNA light chain variable domain | tcctatgtgctgactcagccaccctcggtgtcagtggccccaggaaag acggccaggattacctgtgggggaaacatcattggaagtaaacttgta cactggtaccagcagaagccaggccaggcccctgtgctggtcatcta tgatgatggcgaccggccctcagggatccctgagcgattctctggctc caactctgggaacacggccaccctgaccatcagcagggtcgaggcc ggggatgaggccgactattattgtcaggtgtgggatactggtagtgat cccgtggtattcggcggagggaccaagctgaccgtcctaggt |
| SEQ ID NO: 10 | polypeptide sequence light chain variable region | SYVLTQPPSVSVAPGKTARITCGGNIIGSKLV HWYQQKPGQAPVLVIYDDGDRPSGIPERFSG SNSGNTATLTISRVEAGDEADYYCQVWDTG SDPVVFGGGTKLTVL |
| SEQ ID NO: 11 | Heavy chain | QVQLVQSGAEVKKPGASVKVSCKASGYTFT NYGLSWVRQAPGQGLEWMGWISANNGDTN YGQEFQGRVTMTTDTSTSTAYMELRSLRSD DTAVYYCARDSSSSWARWFFDLWGRGTLV TVSSASTKGPSVFPLAPCSRSTSESTAALGCL VKDYFPEPVTVSWNSGALTSGVHTFPAVLQS SGLYSLSSVVTVPSSSLGTKTYTCNVDHKPS NTKVDKRVESKYGPPCPSCPAPEFLGGPSVF LFPPKPKDTLMISRTPEVTCVVVDVSQEDPE |
| | | VQFNWYVDGVEVHNAKTKPREEQFNSTYR VVSVLTVLHQDWLNGKEYKCKVSNKGLPSS IEKTISKAKGQPREPQVYTLPPSQEEMTKNQ VSLTCLVKGFYPSDIAVEWESNGQPENNYKT TPPVLDSDGSFFLYSRLTVDKSRWQEGNVFS CSVMHEALHNHYTQKSLSLSLGK |
| SEQ ID NO: 12 | Light chain | SYVLTQPPSVSVAPGKTARITCGGNIIGSKLV HWYQQKPGQAPVLVIYDDGDRPSGIPERFSG SNSGNTATLTISRVEAGDEADYYCQVWDTG SDPVVFGGGTKLTVLGQPKAAPSVTLFPPSS EELQANKATLVCLISDFYPGAVTVAWKADS SPVKAGVETTTPSKQSNNKYAASSYLSLTPE QWKSHRSYSCQVTHEGSTVEKTVAPTECS |

Methods for identifying, isolating and testing (e.g. binding and neutralisation) of antibodies and fragment thereof are well-known in the art. See WO 2005/007699, which teaches the identification and characterisation of various anti-IL13 antibodies and fragments and provides suitable methods for doing so.

### Dose and dosing regimen

The invention provides an interleukin-13 (IL-13) binding protein for use in a method of treating atopic dermatitis (AD) in a subject, wherein the method comprises the steps of: (i) administering prior doses of the IL-13 binding protein to the subject, wherein each prior dose is administered 2 weeks after the immediately preceding prior dose, wherein the first prior dose is around 600 mg and is administered to the subject at day 0, and each prior dose after the first prior dose is around 300 mg is administered to the subject at weeks 2, 4, 6, 8, 10, 12, 14 and 16; and wherein if the subject achieves an IGA score of 0 or 1 and/or ≥75% improvement of EASI-75 over baseline in the subject; (ii) administering a first dose of IL-13 binding protein to the subject, wherein the first dose is around 300 mg and is administered to the subject at week 20; and (iii) administering secondary doses of the IL-13 binding protein to the subject, wherein each secondary dose of the IL-13 binding protein is administered to the subject 4 weeks after the immediately preceding dose, wherein each secondary dose is around 300 mg of IL-13 binding protein and is administered to the subject at weeks 24, 28, 32, 36, 40, 44, 48 and 52; and wherein the IL-13 binding protein is tralokinumab.

The term "dose" refers to the amount (mass) of IL-13 binding protein administered to the subject on the particular treatment day. For example, a dose of 300 mg of IL-13 binding protein means that on a treatment day a total of 300 mg of IL-13 binding protein is given to the subject. Typically, a dose is administered in a single administration step (e.g. one injection). However, in some embodiments, one, two, three or more administration steps (e.g. one, two, three or more injections) may be used to provide the subject with the desired dose.

The terms "prior dose", "first dose", and "secondary dose" refer to the temporal sequence of administration of the IL-13 binding protein. The term "first dose" is a single dose of IL-13 binding protein that is followed by secondary doses. The first dose is preceded by prior doses. Subsequent to the first dose are secondary doses.

The phrase "immediately preceding dose" means, in a sequence of multiple doses, the dose of IL-13 binding protein which is administered to a patient prior to the administration of the very next dose in the sequence, with no intervening doses of the IL-13 binding protein.

"Dosing frequency" is the frequency of administering a dose of the IL-13 binding protein. Thus, a decrease in dosing frequency means an increase in the time interval between doses. Common terminology used in relation to dosing frequency is QW (once weekly), Q2W (once every 2 weeks), Q3W (every 3 weeks), or Q4W (every 4 weeks).

The first dose is about 300 mg of tralokinumab (e.g. as illustrated in the examples).

Each secondary dose is administered to the subject 4 weeks after the immediately preceding dose (as exemplified herein).

Herein, the phrase "low disease state" is an Investigator's Global Assessment (IGA) score of 0 or 1 and/or ≥ 75% improvement of Eczema Area and Severity Index (EASI-75) over baseline.

Each secondary dose is about 300 mg of tralokinumab. Preferably, each administration is by subcutaneous injection.

Each prior dose is about 300 mg of tralokinumab (e.g. as illustrated in the examples).

Administering prior doses achieves an Investigator's Global Assessment (IGA) score of 0 or 1 and/or ≥ 75% improvement of Eczema Area and Severity Index (EASI-75) over baseline in a subject, which is then maintained by method steps (i) and (ii) (e.g. as illustrated in Example 4).

The very first dose of said prior doses is a bolus dose which is double the amount of the doses following the bolus dose, such that the very first dose of said prior doses is 600 mg dose and each dose following the 600 mg dose is 300 mg doses.

The bolus dose is given as a first dose of the above mentioned "prior doses". The bolus is twice the amount of the dose administered with the next administration, i.e. a dose of 600 mg is used as a bolus dose when the next dose administered is 300 mg.

### Administration

In the methods described herein, the IL-13 binding protein (e.g. an anti-IL-13 antibody or an IL-13-binding fragment thereof) may be administered by any appropriate method. Typically, administration is parenteral, e.g. intradermal, intramuscular, intravenous and subcutaneous. Subcutaneous administration is particularly preferred (e.g. as illustrated in the examples). Each dose of the IL-13 binding protein may therefore be administered subcutaneously.

Administration is preferably in a "therapeutically effective amount", this being sufficient to show improvement or maintained improvement in one or more AD-associated parameter or patient-related outcome as described herein, or achievement of a low disease state.

Administration may be by any convenient route, for example by infusion or bolus injection, by absorption through epithelial or mucocutaneous linings (e.g. oral mucosa, rectal and intestinal mucosa, etc.).

Subcutaneous or intravenous delivery may be with a standard needle and syringe (e.g. including with a prefilled syringe). It is envisaged that the methods described herein will not be restricted to use in the clinic. Therefore, subcutaneous injection using a needle free device is also preferred. Such delivery devices can be reusable or disposable. Numerous reusable pen and autoinjector delivery devices are known in the art and may find use in the present invention. Examples include AUTOPEN^{™} (Owen Mumford, Inc., Woodstock, UK), DISETRONIC^{™} pen (Disetronic Medical Systems, Bergdorf, Switzerland), HUMALOG MIX 75/25^{™} pen, HUMALOG^{™} pen, HUMALIN 70/30^{™} pen (Eli Lilly and Co., Indianapolis, IN), NOVOPFN^{™} 1, 11 and 111 (Nova Nordisk, Copenhagen, Denmark), NOVOPEN JUNIOR^{™} (Nova Nordisk, Copenhagen, Denmark), BD^{™} pen (Becton Dickinson, Franklin Lakes, NJ), OPTIPEN^{™}, OPTIPEN PRO^{™}, OPTIPEN STARLET^{™}, and OPTICLIK^{™} (Sanofi-Aventis, Frankfurt, Germany). Exemplary disposable pen delivery devices for subcutaneous delivery that may find use in the present invention applications include the SOLOSTAR^{™} pen (Sanofi-Aventis), the FLEXPEN^{™} (Nova Nordisk), and the KWIKPEN^{™} (Eli Lilly), the SURECLICK^{™} Autoinjector (Amgen, Thousand Oaks, CA), the PENLET^{™} (Haselmeier, Stuttgart, Germany), the EPIPEN (Dey, L.P.), and the HUMIRA^{™} Pen (Abbott Labs, Abbott Park IL).

Each dose of IL-13 binding protein of is not necessarily administered in a single administration step (e.g. one injection or one tablet etc.). Indeed, depending on the concentration of the IL-13 binding protein (e.g. in the pharmaceutical composition), one, two, three or more administration steps (e.g. one, two, three or more injections) may be required to provide the subject with the required amount IL-13 binding protein (e.g. a 300 mg dose, for example). Thus, in some embodiments, each dose of the IL-13 binding protein is administered in one or two injections (e.g. subcutaneously). Typically subcutaneous injections have a volume of around 1.5 mL or less, such as a volume of from 0.2 to 1.5 mL, e.g. around 1 mL.

### Monotherapy and combination therapy

The methods described herein may be a monotherapy (e.g. as in Examples 4 and 5). As used herein, the term "monotherapy" is a therapy which uses a single drug to treat a disease or condition. Therefore, a subject that is treated with a monotherapy will receive only a single drug to treat the relevant disorder, e.g. AD, a skin infection, pruritus or eczema-related sleep interference. For example, an anti-IL-13 antibody monotherapy refers to a monotherapy which comprises the administration of anti-IL-13 antibody to the subject as the sole drug for the treatment of AD or a skin infection.

The methods described herein may be a combination therapy (e.g. as in Examples 1-3). As used herein, the term "combination therapy" is a therapy which uses more than one drug to treat a disease or condition. For example, a subject that is treated with a combination therapy will receive more than one drug (e.g. two, three or more) to treat AD.

In some embodiments, the IL-13 binding protein is administered in combination with a topical therapy (such as a topical corticosteroid or a topical calcineurin inhibitor). In some instances, the additional treatment (e.g. TCS or TCI) is administered as needed by the subject.

In some cases, the IL-13 binding protein is administered in combination with a second therapeutic agent selected from the group consisting of a topical corticosteroid, a topical calcineurin inhibitor, an anti-histamine, an emollient, or an anti-bacterial therapeutic. In some cases, the IL-13 binding protein is administered in combination with a Group I, Group II, Group III or Group IV corticosteroid. Preferably, the IL-13 binding protein can be administered in combination with mometasone furoate (e.g. 0.1% cream), as illustrated in Example 1.

### Pharmaceutical compositions and formulations

The present invention envisages methods where each dose of tralokinumab is administered as a pharmaceutical composition.

The pharmaceutical compositions may be formulated with suitable carriers, excipients, and other agents that provide suitable transfer, delivery, tolerance, and the like. A multitude of formulations can be found in the formulary known to all pharmaceutical chemists: Remington's Pharmaceutical Sciences, Mack Publishing Company, Easton, PA.

The dose administered to a patient according to the methods described herein may be varied depending upon the age and the size of the patient, symptoms, conditions, route of administration, and the like. The dose can be calculated according to body weight or body surface area.

Thus, the pharmaceutical compositions may comprise, in addition to the active ingredient (i.e. tralokinumab), a pharmaceutically acceptable excipient, carrier, buffer, stabiliser or other materials well known to those skilled in the art. Such materials should be non-toxic and should not interfere with the efficacy of the active ingredient. The precise nature of the carrier or other material will depend on the route of administration, which may be oral, or by injection, e.g. intravenous or subcutaneous. Pharmaceutical compositions for oral administration may be in tablet, capsule, powder or liquid form. A tablet may comprise a solid carrier such as gelatin or an adjuvant. Liquid pharmaceutical compositions generally comprise a liquid carrier such as water, petroleum, animal or vegetable oils, mineral oil or synthetic oil. Physiological saline solution, dextrose or other saccharide solution or glycols such as ethylene glycol, propylene glycol or polyethylene glycol may be included.

For intravenous injection or subcutaneous injection, the pharmaceutical composition may be a parenterally acceptable aqueous solution which is pyrogen-free and has suitable pH, isotonicity and stability. Those of relevant skill in the art are well able to prepare suitable solutions using, for example, isotonic vehicles such as Sodium Chloride Injection, Ringer's Injection, Lactated Ringer's Injection. Preservatives, stabilisers, buffers, antioxidants and/or other additives may be included, as required.

The pharmaceutical composition may be a liquid formulation or a lyophilized formulation which is reconstituted before use. As excipients for a lyophilized formulation, for example, sugar alcohols, or saccharides (e.g. mannitol or glucose) may be used. In the case of a liquid formulation, the pharmaceutical composition is usually provided in the form of containers with defined volume, including sealed and sterilized plastic or glass vials, ampoules and syringes, as well as in the form of large volume containers like bottles. Preferably, in the methods described herein, the pharmaceutical composition is a liquid formulation.

Exemplary pharmaceutical compositions that can be used in the context of the present invention are disclosed in, for example, WO 2007/036745 and WO 2018/158332.

Preferably, tralokinumab may be present within the pharmaceutical composition at a concentration of from 1 mg/mL to 200 mg/mL, more preferably 150 mg/mL.

Preferably, the pharmaceutical composition may be buffered to a pH of 5.2 to 5.7, most preferably 5.5 (e.g. ± 0.1). The selection of such a pH confers significant stability to the pharmaceutical composition. Examples of alternative buffers that control the pH in this range include succinate, gluconate, histidine, citrate, phosphate, glutarate, cacodylate, sodium hydrogen maleate, tris(hydroxymethyl)aminomethane (Tris), 2-(N-morpholino) ethanesulphonic acid (MES), imidazole. Preferably, the buffer is acetate buffer, more preferably sodium acetate buffer.

Preferably, the acetate buffer is present within the pharmaceutical composition in an amount of from 1 mM to 100 mM, more preferably from 30 mM to 70 mM, especially 50 mM.

It will be appreciated that references to "pharmaceutically acceptable excipient" includes references to any excipient conventionally used in pharmaceutical compositions. Such excipients may typically include one or more surfactant, inorganic or organic salt, stabilizer, diluent, solubilizer, reducing agent, antioxidant, chelating agent, preservative and the like.

Examples of a typical surfactant include: nonionic surfactants (HLB 6 to 18) such as sorbitan fatty acid esters (e.g. sorbitan monocaprylate, sorbitan monolaurate, sorbitan monopalmitate), glycerine fatty acid esters (e.g. glycerine monocaprylate, glycerine monomyristate, glycerine monostearate), polyglycerine fatty acid esters (e.g. decaglyceryl monostearate, decaglyceryl distearate, decaglyceryl monolinoleate), polyoxyethylene sorbitan fatty acid esters (e.g. polyoxyethylene sorbitan monolaurate, polyoxyethylene sorbitan monooleate, polyoxyethylene sorbitan monostearate, polyoxyethylene sorbitan monopalmitate, polyoxyethylene sorbitan trioleate, polyoxyethylene sorbitan tristearate), polyoxyethylene sorbitol fatty acid esters (e.g. polyoxyethylene sorbitol tetrastearate, polyoxyethylene sorbitol tetraoleate), polyoxyethylene glycerine fatty acid esters (e.g. polyoxyethylene glyceryl monostearate), polyethylene glycol fatty acid esters (e.g. polyethylene glycol distearate), polyoxyethylene alkyl ethers (e.g. polyoxyethylene lauryl ether), polyoxyethylene polyoxypropylene alkyl ethers (e.g. polyoxyethylene polyoxypropylene glycol ether, polyoxyethylene polyoxypropylene propyl ether, polyoxyethylene polyoxypropylene cetyl ether), polyoxyethylene alkylphenyl ethers (e.g. polyoxyethylene nonylphenyl ether), polyoxyethylene hydrogenated castor oils (e.g. polyoxyethylene castor oil, polyoxyethylene hydrogenated castor oil), polyoxyethylene beeswax derivatives (e.g. polyoxyethylene sorbitol beeswax), polyoxyethylene lanolin derivatives (e.g. polyoxyethylene lanolin), and polyoxyethylene fatty acid amides (e.g. polyoxyethylene stearyl amide); anionic surfactants such as C10-C18 alkyl sulfates salts (e.g. sodium cetyl sulfate, sodium lauryl sulfate, sodium oleyl sulfate), polyoxyethylene C10-C18 alkyl ether sulfates salts with an average of 2 to 4 moles of ethylene oxide (e.g. sodium polyoxyethylene lauryl sulfate), and C8-C18 alkyl sulfosuccinate ester salts (e.g. sodium lauryl sulfosuccinate ester); and natural surfactants such as lecithin, glycerophospholipid, sphingophospholipids (e.g. sphingomyelin), and sucrose esters of C12-C18 fatty acids. The surfactant may be selected from polyoxyethylene sorbitan fatty acid esters. Preferred surfactants are polysorbate 20, 21, 40, 60, 65, 80, 81 and 85, most preferably polysorbate 20 and 80, especially polysorbate 80.

Preferably, the surfactant is present within the pharmaceutical composition in an amount of from 0.001% to 0.1% (w/w), more preferably 0.005% and 0.05% (w/w), especially 0.01% (w/w).

Examples of a typical inorganic salt include: sodium chloride, potassium chloride, calcium chloride, sodium phosphate, sodium sulphate, ammonium sulphate, potassium phosphate and sodium bicarbonate or any other sodium, potassium or calcium salt. Preferably, the inorganic salt is sodium chloride.

Preferably, the inorganic salt is present within the pharmaceutical composition in an amount of from 10 mM to 200 mM, more preferably from 60 mM to 130 mM, especially 85 mM.

Examples of a reducing agent include N-acetylcysteine, Nacetylhomocysteine, thioctic acid, thiodiglycol, thioethanolamine,thioglycerol, thiosorbitol, thioglycolic acid and a salt thereof, sodium thiosulfate, glutathione, and a Cl-C7 thioalkanoic acid.

Examples of an antioxidant include erythorbic acid, dibutylhydroxytoluene, butylhydroxyanisole, alpha-tocopherol, tocopherol acetate, L-ascorbic acid and a salt thereof, L-ascorbic acid palmitate, L-ascorbic acid stearate, sodium bisulfite, sodium sulfite, triamyl gallate and propyl gallate.

Examples of a chelating agent include disodium ethylenediaminetetraacetate (EDTA), sodium pyrophosphate and sodium metaphosphate.

Examples of a stabiliser include creatinine, an amino acid selected from histidine, alanine, glutamic acid, glycine, leucine, phenylalanine, methionine, isoleucine, proline, aspartic acid, arginine, lysine and threonine, a carbohydrate selected from sucrose, trehalose, sorbitol, xylitol and mannose, surfactants selected from polyethylene glycol (PEG; e.g. PEG3350 or PEG 4000) or polyoxyethylene sorbitan fatty acid esters (e.g. polysorbate 20 or polysorbate 80), or any combination thereof.

In one preferred embodiment the stabiliser comprises a single carbohydrate (e.g. trehalose).

In an alternatively preferred embodiment the stabilizer comprises an amino acid in combination with a carbohydrate (e.g. trehalose and alanine or trehalose, alanine and glycine).

In a further alternatively preferred embodiment the stabiliser comprises an amino acid in combination with a carbohydrate and a surfactant (e.g. trehalose, alanine and PEG3350; trehalose, proline and PEG3350; trehalose, alanine and polysorbate 80; trehalose, proline and polysorbate 80; trehalose, alanine, glycine and PEG3350; trehalose, alanine, glycine and polysorbate 80).

In a yet further alternatively preferred embodiment the stabiliser comprises an amino acid in combination with a surfactant (e.g. alanine and PEG3350 or alanine, glycine and PEG3350).

In a yet further alternatively preferred embodiment the stabiliser comprises a carbohydrate in combination with a surfactant (e.g. trehalose and PEG3350 or trehalose and polysorbate 80).

Examples of a preservative include octadecyldimethylbenzyl ammonium chloride, hexamethonium chloride, benzalkonium chloride (a mixture of alkylbenzyldimethylammonium chlorides in which the alkyl groups are long chain compounds), benzethonium chloride, aromatic alcohols such as phenol, butyl and benzyl alcohol, alkyl parabens such as methyl or propyl paraben, catechol, resorcinol, cyclohexanol, 3-pentanol, and m-cresol. In a preferred embodiment, the pharmaceutical composition comprises tralokinumab, a surfactant and an inorganic salt buffered to a pH of 5.5 ± 0 .1 with acetate buffer.

In a further preferred embodiment, the pharmaceutical composition comprises tralokinumab, sodium chloride and polysorbate 80, buffered to a pH of 5.5 ± 0 .1 with sodium acetate buffer.

In a yet further preferred embodiment, the pharmaceutical composition comprises tralokinumab, 50 mM sodium acetate buffer, 85 mM sodium chloride, 0.01% (w/v) polysorbate 80, wherein the pharmaceutical composition has a pH of 5.5.

In a yet further preferred embodiment, the pharmaceutical composition comprises 150 mg/mL of tralokinumab, 50 mM sodium acetate buffer, 85 mM sodium chloride, 0.01% (w/v) polysorbate 80, wherein the pharmaceutical composition has a pH of 5.5.

### Other definitions

Unless defined otherwise, all technical and scientific terms used herein have the same meaning as commonly understood to one of ordinary skill in the art to which this invention belongs.

The articles "a" and "an"" refer to one or to more than one (i.e., to at least one) of the grammatical object of the article. By way of example, "an element" means one element or more than one element.

The terms "comprise" and "comprising" are used in the inclusive, open sense, meaning that additional elements may be included.

In general, methods "comprising" a number of steps do not require the steps to be performed in a particular order. Where a method comprises a number of sequentially numbered or alphabetical steps (e.g. (1), (2), (3); (i), (ii), (iii); or (a), (b), (c) etc.), this implies that the steps must be performed in the prescribed order unless stated otherwise.

The term "including" is used herein to mean "including but not limited to".

The term "about" in relation to a numerical value x is optional and means, for example, x ±10%.

Generally, the terms "treat", "treating", "treatment", or the like, mean to alleviate (reduce, minimise, or eliminate) symptoms, or to reduce, minimise or eliminate the causation of symptoms either on a temporary or permanent basis.

### EXAMPLES

The invention is further illustrated by the following examples. It will be appreciated that the examples are for illustrative purposes only and are not intended to limit the invention as described above. Modification of detail may be made without departing from the scope of the invention.

### Example 1: Tralokinumab/TCS combination therapy is effective in treating moderate to severe atopic dermatitis

A Phase 3 trial (EZCTRA 3) was conducted to assess the efficacy and safety of a combination therapy of tralokinumab and (as needed) topical corticosteroid (TCS) in moderate-to-severe AD.

### Methods

This was a double-blind, randomized 32 week study (NCT03363854). Patients with moderate-to-severe AD (IGA of 3 or 4) were randomized 2:1 and administered subcutaneous tralokinumab 300 mg (Q2W+TCS) or control (placebo Q2W+TCS) every 2 weeks. A loading dose of 600 mg Tralokinumab or placebo was given on day 0. Throughout the entire treatment period, all patients applied a thin layer of supplied TCS as needed (mometasone furoate 0.1% cream; Europe: Class 3 [potent]; US: Class 4 [mid-strength]); provided in kit sizes of 180-200 g at each visit) once daily to areas with active lesions. An additional lower potency TCS or topical calcineurin inhibitor [TCI] was prescribed if needed for use on body areas where the supplied TCS was inadvisable, areas of thin skin (e.g., face, skin fold areas, genital areas), or on areas where continued treatment with TCS was considered unsafe. TCS use was continually monitored for safety and appropriateness and was discontinued gradually when control was achieved. Patients were instructed to return used and unused tubes at each trial visit to allow measurement of the amount of TCS used. An emollient was applied twice daily (or more, as needed) for at least 14 days before randomization and throughout the trial (including safety follow-up). For lesional skin, emollient was only applied when TCS was not applied. Rescue treatment in the form of topical and systemic medications was permitted to control intolerable AD symptoms. Patients receiving higher potency TCS (Europe: Class >3; US: Class <4) continued treatment, while those receiving systemic corticosteroids or nonsteroidal systemic immunosuppressive drugs temporarily discontinued treatment until cessation of rescue (and a 5 half-life washout for systemic treatment).

Primary endpoints were an Investigator's Global Assessment (IGA) score of 0 or 1 (IGA-0/1; clear or almost clear skin) and ≥ 75% improvement of Eczema Area and Severity Index (EASI-75) at week 16. At baseline, 46.3% of 380 randomized patients had severe AD (IGA-4). The mean baseline EASI score was 29.4. Additional secondary endpoints were the amount of TCS used and number of days without TCS use.

At week 16, tralokinumab responders (IGA-0/1 and/or EASI-75) were re-randomized 1:1 to tralokinumab Q2W or every 4 weeks (Q4W)+TCS for an additional 16 weeks. Placebo responders continued placebo Q2W+TCS; all non-responders received tralokinumab Q2W+TCS.

**Table 2: Demographic and clinical characteristics of randomized patients at baseline**

| **Characteristic** | | | **All randomized** (N=380) | **Placebo every other week + TCS** (N=127) | **Tralokinumab every other week + TCS** (N=253) |
|---|---|---|---|---|---|
| Median age, years (IQR) | | | 36.0 (27.0-51.0) | 34.0 (24.0-50.0) | 37.0 (28.0-52.0) |

| Sex, n (%) | | | | | |
|---|---|---|---|---|---|
| | Male | | 209 (55.0) | 84 (66.1) | 125 (49.4) |
| | Female | | 171 (45.0) | 43 (33.9) | 128 (50.6) |

| Race, n (%) | | | | | |
|---|---|---|---|---|---|
| | White | | 288 (75.8) | 85 (66.9) | 203 (80.2) |
| | Black or African American | | 35 (9.2) | 12 (9.4) | 23 (9.1) |
| | Asian | | 41 (10.8) | 24 (18.9) | 17 (6.7) |
| | Native Hawaiian or other Pacific Islander | | 2 (0.5) | 1 (0.8) | 1 (0.4) |
| | Other | | 14 (3.7) | 5 (3.9) | 9 (3.6) |
| Median duration of AD, years (IQR) | | | N=379 26.0 (17.0-39.0) | N=126 26.0 (18.0-39.0) | N=253 27.0 (17.0-39.0) |
| Median BSA, % (IQR) | | | 41.0 (28.0-69.5) | 40.0 (26.0-74.0) | 41.0 (30.0-63.0) |
| IGA, n (%) | | | | | |
| | Moderate | | 202 (53.2) | 66 (52.0) | 136 (53.8) |
| | Severe | | 176 (46.3) | 60 (47.2) | 116 (45.8) |
| | Missing* | | 2 (0.5) | 1 (0.8) | 1 (0.4) |
| Median EASI score (IQR) | | | N=378 25.5 (19.2-37.1) | N=126 26.5 (19.9-39.3) | N=252 24.7 (18.4-35.9) |
| Median SCORAD total score (IQR) | | | N=378 66.5 (57.9-77.6) | N=126 67.9 (59.4-79.0) | N=252 66.2 (57.6-76.3) |
| Median DLQI score (IQR) | | | N=375 18.0 (12.0-23.0) | N=125 18.0-(12.0-23.0) | N=250 18.0 (12.0-23.0) |
| Median weekly average of worst daily pruritus NRS score (IQR) | | | N=377 8.0 (6.6-8.9) | N=126 8.0 (7.0-9.0) | N=251 8.0-(6.6-8.7) |

| History of allergic conjunctivitis (atopy form), n (%) | | | | | |
|---|---|---|---|---|---|
| | | Current | 84 (22.1) | 26 (20.5) | 58 (22.9) |
| | | Past | 45 (11.8) | 11 (8.7) | 34 (13.4) |
| History of asthma (atopy form), n (%) | | | | | |
| | | Current | 177 (46.6) | 58 (45.7) | 119 (47.0) |
| | | Past | 47 (12.4) | 19 (15.0) | 28 (11.1) |

| History of atopic keratoconjunctivitis (atopy form), n(%) | | | | | |
|---|---|---|---|---|---|
| | | Current | 13 (3.4) | 5 (3.9) | 8 (3.2) |
| | | Past | 6 (1.6) | 4 (3.1) | 2 (0.8) |

| History of food allergy (atopy form), n (%) | | | | | |
|---|---|---|---|---|---|
| | | Current | 138 (36.3) | 48 (37.8) | 90 (35.6) |
| | | Past | 12 (3.2) | 3 (2.4) | 9 (3.6) |

| History of hay fever (atopy form), n (%) | | | | | |
|---|---|---|---|---|---|
| | | Current | 210 (55.3) | 69 (54.3) | 141 (55.7) |
| | | Past | 20 (5.3) | 3 (2.4) | 17 (6.7) |

| | | | | | |
|---|---|---|---|---|---|
| *Patients did not receive a treatment dose and were not included in the FAS. | | | | | |

### Results

### Tralokinumab treatment significantly improved IGA and EASI scores after 16 weeks

380 patients were randomized in the initial treatment period; 253 to tralokinumab plus TCS every other week and 127 to placebo plus TCS every other week. One patient from each treatment group withdrew from the trial before being dosed; therefore, 252 patients treated with tralokinumab plus TCS every other week and 126 patients treated with placebo plus TCS every other week were included in the full analysis and safety analysis sets. Continuation treatment (based on response at week 16 and initial treatment) was assigned to 353 patients.

Baseline demographics and disease characteristics were balanced across treatment groups, with approximately half of patients having severe disease (IGA-4) at baseline; median duration of AD was 26.0 years and median body surface area involvement was 41%. A number of patients had comorbid atopic diseases. All patients received prior therapy, with almost all receiving TCS (98.2%) and 61.6% used systemic steroids. Cyclosporine was the most common prior oral immunosuppressant used (31.1%).

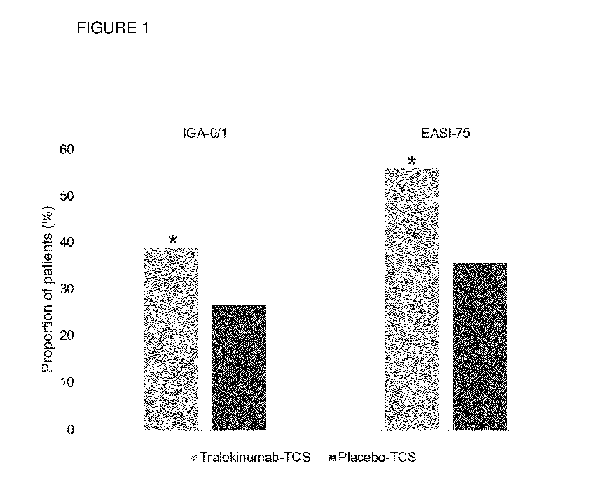

There were significant differences between tralokinumab plus TCS and placebo plus TCS for both primary endpoints. More patients achieved IGA-0/1 and EASI-75 with tralokinumab plus TCS compared with placebo plus TCS at week 16. IGA-0/1 was achieved by 38.9% of patients who received tralokinumab plus TCS versus 26.2% of patients who received placebo plus TCS (p=0.015). EASI-75 was achieved by 56% of patients who received tralokinumab plus TCS versus 35.7% of patients who received placebo plus TCS (p<0.001). The sensitivity, secondary, and tertiary analyses supported the results of the primary analysis, Fig 1.

Irrespective of IGA severity at baseline and history of atopic disease (asthma, food allergy and hay fever), the proportion of IGA-0/1 and EASI-75 responders was higher with tralokinumab plus TCS than placebo plus TCS. IGA-0/1 and EASI-75 response rates were higher in female patients and in patients aged ≥65 (tralokinumab plus TCS group only). Rescue medication use was higher with placebo plus TCS (10.2%) than with tralokinumab plus TCS (2.8%).

### Tralokinumab treatment reduced the need for TCS use

Rescue treatment was reported by 2.8% of tralokinumab-treated patients, compared to 10.2% of control patients. Significantly less TCS was used cumulatively by tralokinumab-treated than control patients over 16 weeks (134.9 g versus 193.5 g, Δ58.6 g; p=0.004). The number of TCS-free days (weekly average from eDiary, including lower potency TCS and TCI) was significantly higher for tralokinumab-treated compared to control patients at week 7 (Δ0.6 days; p=0.040) and from week 9 to 15 (range: Δ1.0 days; p=0.001 to Δ0.6 days; p=0.045). Tralokinumab-treated patients also had 0.5 more TCS-free days on average at week 16 (p=0.17).

At week 16, subjects in the tralokinumab group used 50% less of the supplied TCS compared to subjects who received placebo (p<0.001) [data not shown]. A greater proportion of patients used less than 5 g of TCS at week 15-16 with tralokinumab plus TCS (55.3%) versus placebo plus TCS (36.7%). The reduction in TCS use in the tralokinumab plus TCS treatment group was not compensated by use of lower potency TCS or TCI.

Results at week 16 may be seen in Table 3 below.

**Table 3.Efficacy outcomes for initial treatment period: full analysis set *Mean across multiple imputations, where applicable; ^{†}Patients who received rescue medication considered nonresponders. Patients with missing data at week 16 imputed as nonresponders; ^{‡}Mantel-Haenszel risk difference, stratified by region and baseline IGA; ^{§}Single imputation analyses: Cochran-Mantel-Haenszel test, stratified by region and baseline IGA. Multiple imputation analyses: Combined inference from multiple Mantel-Haenszel risk differences and associated SE; ^{∥}Data collected after permanent discontinuation of Tralokinumab or initiation of rescue medication not included. Repeated measurements model on post-baseline data: Change = Treatment*Week + Baseline*Week + Region + Baseline IGA. In case of no postbaseline assessments before initiation of rescue medication, the week 2 change is imputed as 0; ^{¶}Based on patients in FAS with a baseline pruritus NRS weekly average of at least 4; **Based on patients in FAS with a baseline pruritus NRS weekly average of at least 3; ^{††}Cochran-Mantel-Haenszel test, stratified by region and baseline IGA; ^{‡‡}Data collected after permanent discontinuation of Tralokinumab or initiation of rescue medication not included. Repeated measurements model: Change = Treatment*Week + Baseline*Week + Region + Baseline IGA. In case of no postbaseline assessments before initiation of rescue medication, the week 1 change is imputed as 0; ^{§§}Analysis only includes patients with baseline DLQI ≥4. ^{∥ ∥} Data collected after permanent discontinuation of Tralokinumab or initiation of rescue medication not included. The response variable was the logarithm of the [cumulative amount of TCS + 1]. Estimated parameters are back transformed using exponential function. A value of one was subtracted from the back-transformed adjusted mean and CI limits to account for adding the same factor before data transformation. Repeated measurements model: log[cumulative TCS amount + 1] (g) = Treatment*Week + Region + Baseline IGA.**

| **Outcome** | | | **Placebo every other week + TCS** (N=126) | **Tralokinumab every other week + TCS** (N=252) |
|---|---|---|---|---|
| **Primary endpoints** | | | | |
| IGA-0/1 at week 16, n (%) *^{,†} | | | 33/126 (26.2) | 98/252 (38.9) |
| | | Difference versus placebo every other week + TCS (95% CI)^{‡} | | 12.4 (2.9, 21.9) |
| | | | | p=0.015^{§} |
| EASI-75 at week 16, n (%)*^{,†} | | | 45/126 (35.7) | 141/252 (56.0) |
| | | Difference versus placebo every other week + TCS (95% CI)^{‡} | | 20.2 (9.8, 30.6) |
| | | | | p<0.001^{§} |
| EASI, LS mean % change from baseline (± SE) | | | -55.3 (± 3.2) | -71.3^{§} (± 2.2) |

| **Key secondary endpoints** | | | | |
|---|---|---|---|---|
| Adjusted mean change from baseline in SCORAD at week 16 (SE)^{∥} | | | -26.8 (1.80) | -37.7 (1.25) |
| | | Difference versus placebo every other week + TCS (95%CI) | | -10.9 (-15.2 to -6.6) |
| | | | | p<0.001 |
| | SCORAD 50, % responders^{†} | | 38.1 | 61.1^{§} |
| | SCORAD, LS mean %-change from baseline (± SE^{∥} | | -40.0 (± 2.6) | -55.9^{§} (± 1.8) |
| Worst daily pruritus NRS (weekly average) reduction ≥4 at week 16, n/N (%)*^{,†} | | | 43/126 (34.1) | 113/249^{¶} (45.4) |
| | | Difference versus placebo every other week + TCS (95%CI)^{‡} | | 11.3 (0.9, 21.6) |
| | | | | p=0.037^{§} |
| Adjusted mean change from baseline in DLQI at week 16 (SE)^{∥} | | | -8.8 (0.56) | -11.7 (0.39) |
| | | Difference versus placebo every other week + TCS (95%CI) | | -2.9 (-4.3 to -1.6) |
| | | | | p<0.001 |

| **Additional secondary endpoints** | | | | |
|---|---|---|---|---|
| Adjusted mean change from baseline in worst daily pruritus NRS (weekly average) at week 16 (SE)^{‡‡} | | | N=100 | N=221 |
| | | | -2.9 (0.21) | -4.1 (0.15) |
| | | Difference versus placebo every other week + TCS (95%CI) | | -1.2 (-1.7 to -0.7) |
| | | | | p<0.001 |
| DLQI reduction ≥4 at week 16, n/N (%)^{†} | | | 81/123^{§§} (65.9) | 207/248^{§§} (83.5) |
| | | Difference versus placebo every other week + TCS (95%CI)^{‡} | | 17.6 (8.0, 27.1) |
| | | | | p<0.001^{††} |
| Adjusted mean change from baseline in EASI at week 16 (SE) ^{∥} | | | N=108 | N=229 |
| | | | -15.6 (0.96) | -21.0 (0.67) |
| | | Difference versus placebo every other week + TCS (95%CI) | | -5.4 (-7.7 to -3.1) |
| | | | | p<0.001 |
| EASI-50 at week 16, n (%)^{†} | | | 73/126 (57.9) | 200/252 (79.4) |
| | | Difference versus placebo every other week + TCS (95%CI)^{‡} | | 21.3 (11.3, 31.3) |
| | | | | p<0.001^{††} |
| EASI-90 at week 16, n (%)^{†} | | | 27/126 (21.4) | 83/252 (32.9) |
| | | Difference versus placebo every other week + TCS (95%CI)^{‡} | | 11.4 (2.1, 20.7) |
| | | | | p=0.022^{††} |
| Cumulative amount of TCS used at week 16, adjusted geometric mean (SE) II II | | | N=108 | N=229 |
| | | | 98.6 (1.14) | 58.6 (1.10) |
| | | Ratio of means (95% CI) | | 0.6 (0.4, 0.8) |
| | | | | p=0.002 |

| **Other endpoints** | | | | |
|---|---|---|---|---|
| Adjusted mean change from baseline in SCORAD at week 2 (SE) | | | -16.4 (1.33) | -20.6 (0.93) |
| | | Difference versus placebo every other week + TCS (95% CI) | | -4.2 (-7.4 to -1.0) |
| | | | | p=0.010 |
| Adjusted mean change from baseline in DLQI at week 2 (SE) | | | -7.3 (0.53) | -8.9 (0.37) |
| | | Difference versus placebo every other week + TCS (95%CI) | | -1.7 (-2.9 to -0.4) |
| | | | | p=0.011 |
| Adjusted mean change from baseline in worth daily pruritus NRS (weekly average) at week 1 (SE) | | | N=125 | N=248 |
| | | | -1.3 (0.13) | -1.5 (0.09) |
| | | Difference versus placebo | | -0.2 (-0.6 to -0.1) |
| | | | | p=0.14 |

Other endpoint results may be seen in Table 4 below:

**Table 4.Least squares, SE: Standard error If needed to control intolerable symptoms of atopic dermatitis, patients were permitted to receive rescue treatment at the discretion of the investigator. a) Data after initiation of rescue medication or permanent discontinuation of treatment was excluded from the analyses. b) Subjects who received rescue treatment or had missing data were treated as non-responders. The percentage is calculated relative to the number of subjects with POEM ≥4 at baseline ^{§}p<0.001.**

| **Combination therapy** | | |
|---|---|---|
| | **ECZTRA 3 week 16** | |
| | **Placebo + TCS** | **TRADENAME 300 mg Q2W + TCS** |
| ***Patients randomised*** | 126 | 252 |
| Eczema-related sleep NRS, LS mean change from baseline (SE)^{a)} | -3.1 (0.22) | -4.3^{§} (0.15) |
| POEM, LS mean change from baseline (SE)^{a)} | -7.8 (0.66) | -11.8^{§} (0.46) |
| POEM (≥4-point improvement), responders^{b)} | 59.3% (73/123) | 78.4%^{§} (190/250) |

Other endpoint results for patients achieving clinical response at week 16 may be seen in Table 5:

**Table 5. LS: Least squares, SE: Standard error. If needed to control intolerable symptoms of atopic dermatitis, patients were permitted to receive rescue treatment at the discretion of the investigator. a) All patients were initially treated with TRADENAME 300 mg Q2W + TCS from Week 0 to Week 16. They were subsequently treated with TRADENAME 300 mg Q2W + TCS or Q4W + TCS. b) Responders at Week16 at are identified as patients achieving either IGA 0/1 and/or EASI75. c) Data after initiation of rescue medication or permanent discontinuation of treatment was excluded from the analyses. d) Number of responders divided by number of subjects having baseline value ≥ 4 of the given parameter. Subjects who received rescue treatment or had missing data were treated as non-responders.**

| | **Treatment regimen Week 16-32^{a})** | | | |
|---|---|---|---|---|
| | **Responders at Week 16^{b)}** | | | |
| | **Q2W + TCS** | | **Q4W + TCS** | |
| ***Patients randomised*** | **N=69** | | **N=69** | |
| ***Week number*** | **W16** | **W32** | **W16** | **W32** |
| DLQI, LS mean change from baseline (SE)^{c)} | -14.0 (0.6) | -14.6 (0.6) | -13.9 (0.6) | -13.7 (0.6) |
| POEM, LS mean change from baseline (SE)^{c)} | -15.2 (0.7) | -15.6 (0.7) | -14.1 (0.7) | -13.9 (0.8) |
| Eczema-related sleep NRS, LS mean change from baseline (SE)^{c)} | -5.2 (0.3) | -5.5 (0.3) | -4.8 (0.3) | -5.2 (0.3) |
| DLQI (≥4-point improvement), % responders^{d)} | 98.5% (65/66) | 89.4% (59/66) | 100.0% (68/68) | 83.8% (57/68) |
| POEM (≥4-point improvement), % responders^{d)} | 89.7% (61/68) | 88.2% (60/68) | 94.1% (64/68) | 83.8% (57/68) |

### Tralokinumab response was maintained over 32 weeks

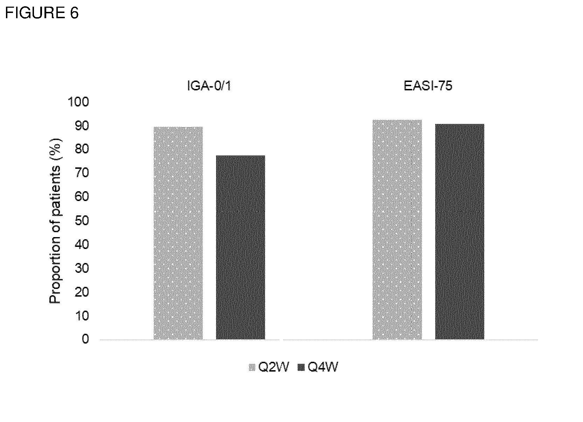

The majority of week 16 tralokinumab responders maintained their response at week 32 with tralokinumab, regardless of dosing frequency (2 week or 4 week). Among tralokinumab responders, 89.6% maintained an IGA-0/1 score and 92.5% maintained EASI-75 at week 32 when receiving tralokinumab every two weeks (Q2W+TCS). For tralokinumab Q4W+TCS, 77.6% achieved IGA-0/1 and 90.8% achieved EASI-75 at week 32. The results are shown in Figure 6.

Among all the subjects who achieved either IGA 0 or 1 or EASI-75 at week 16, the mean percentage improvement in EASI score from baseline was 93.5% at week 32 when maintained on Tralokinumab 300 mg Q2W + TCS and 91.5% at week 32 for subjects on Tralokinumab 300 mg Q4W + TCS.

Of the patients who did not achieve IGA-0/1 and/or EASI-75 with tralokinumab (Q2W+TCS) at week 16, 30.5% and 55.8% achieved IGA-0/1 and EASI-75 respectively at week 32, following continued tralokinumab Q2W+TCS treatment
Of the patients who achieved an EASI-75 or IGA-0/1 response at week 16 on tralokinumab plus TCS every other week, the majority were also EASI-90 responders and continued to maintain that response up to week 32, irrespective of continued every other week, or every 4 weeks dosing. Patients who did not achieve an EASI-75 or IGA-0/1 response at week 16 continued to improve with tralokinumab plus TCS every other week.

Other endpoints at week 16 and 32 are shown in Tables 6 and 7:

**Table 6. LS: Least squares, SE: Standard error. If needed to control intolerable symptoms of atopic dermatitis, patients were permitted to receive rescue treatment at the discretion of the investigator. a) Patients who received rescue treatment or had missing data were considered non-responders in the analyses. b) Data after initiation of rescue medication or permanent discontinuation of treatment was excluded from the analyses. c) The percentage is calculated relative to the number of subjects with a baseline value ≥ 4. d) All patients were initially treated with TRADENAME 300 mg Q2W + TCS from Week 0 to Week 16. They were subsequently treated with TRADENAME 300 mg Q2W + TCS or Q4W + TCS e) Responders at Week16 at are identified as patients achieving either IGA 0/1 and/or EASI75.**

| | **Treatment regimen Week 16-32^{d})** | | | | | |
|---|---|---|---|---|---|---|
| | **Responders at Week 16^{e)}** | | | | **Non-responders at Week 16** | |
| | **Q2W + TCS** | | **Q4W + TCS** | | **Q2W + TCS** | |
| ***Patients randomised*** | **N=69** | | **N=69** | | **N=95** | |
| ***Week number*** | **W16** | **W32** | **W16** | **W32** | **W16** | **W32** |
| EASI-50, % responders^{a)} | 100.0 | 98.6 | 97.1 | 91.3 | 63.2 | 76.8 |
| EASI-90, % responders^{a)} | 58.0 | 72.5 | 60.9 | 63.8 | 1.1 | 34.7 |
| EASI, LS % mean change from baseline (SE)^{b)} | -90.5 (2.7) | -93.2 (2.3) | -89.3 (2.7) | -91.5 (2.3) | -46.9 (2.4) | -73.5 (2.0) |
| SCORAD, LS % mean change from baseline (SE)^{b)} | -73.2 (2.1) | -79.2 (2.5) | -72.3 (2.1) | -73.3 (2.5) | -32.7 (1.8) | -54.5 (2.2) |
| Pruritus NRS (≥ 4-point improvement, % responders)^{a,c)} | 63.2 | 70.6 | 64.2 | 61.2 | 27.4 | 38.9 |
| Pruritus NRS, mean change from baseline (SE)^{b)} | -5.0 (0.2) | -5.4 (0.2) | -4.6 (0.2) | -4.9 (0.2) | -3.0 (0.2) | -3.7 (0.2) |

**Table 7. LS: Least squares, SE: Standard error. If needed to control intolerable symptoms of atopic dermatitis, patients were permitted to receive rescue treatment at the discretion of the investigator. a) Data after initiation of rescue medication or permanent discontinuation of treatment was excluded from the analyses. b) Subjects who received rescue treatment or had missing data were treated as non-responders. The percentage is calculated relative to the number of subjects with POEM ≥4 at baseline*p<0.05, ^{#}p< 0.01, ^{§}p<0.001.**

| **Monotherapy** | | | | |
|---|---|---|---|---|
| | **ECZTRA 1 week 16** | | **ECZTRA 2 week 16** | |
| | **Placebo** | **TRADENAME 300 mg Q2W** | **Placebo** | **TRADENAME 300 mg Q2W** |
| ***Patients randomised*** | 199 | 603 | 201 | 593 |
| Eczema-related sleep NRS, LS mean change from baseline (SE)^{a)} | -1.9 (0.2) | -2.6^{#} (0.1) | -1.5 (0.2) | -2.9^{§} (0.1) |
| POEM, LS mean change from baseline (SE)^{a)} | -3.0 (0.66) | -7.6^{§} (0.35) | -3.7 (0.66) | -8.8^{§} (0.33) |
| POEM (≥4-point improvement), responders^{b)} | 18.0% (35/194) | 43.0%^{§} (253/588) | 22.1% (44/199) | 54.4%^{§} (319/586) |
| SF-36, physical component, LS mean change from baseline (SE)^{a)} | 2.9 (0.56) | 4.5* (0.30) | 3.2 (0.57) | 5.8^{§} (0.29) |
| SF-36, mental component, LS mean change from baseline (SE)^{a)} | 0.3 (0.78) | 2.5* (0.42) | 0.5 (0.76) | 3.5^{§} (0.38) |

### Tralokinumab has a favorable safety profile

Table 8 shows the overall frequency and severity of adverse effects over 16 weeks.

### Summary of AEs and AESIs in the 16 week initial treatment period

**Table 8. *AEs collected during the exposure time in the initial treatment period are shown; ^{†}Classification according to MedDRA 20.0; ^{‡}PTs according to MedDRA 20.0 include conjunctivitis, conjunctivitis allergic and conjunctivitis viral**

| **Event** | | | **Week 16*** | |
|---|---|---|---|---|
| | | | **Placebo every other week + TCS** (N=126, PYE=37.94) | **Tralokinumab every other week + TCS** (N=252, PYE=75.03) |
| Adverse or serious adverse event, n (%) R | | | | |
| At least one adverse event | | | 84 (66.7) | 180 (71.4) |
| | | | 485.0 | 671.7 |
| At least one serious adverse event | | | 4 (3.2) | 2 (0.8) |
| | | | 10.54 | 2.67 |
| | Severity | | | |
| | | Mild | 69 (54.8) | 157 (62.3) |
| | | | 347.9 | 511.8 |
| | | Moderate | 30 (23.8) | 66 (26.2) |
| | | | 110.7 | 150.6 |
| | | Severe | 7 (5.6) | 7 (2.8) |
| | | | 26.36 | 9.33 |
| | Leading to discontinuation of IMP | | 1 (0.8) | 6 (2.4) |
| | | | 2.64 | 10.66 |
| | Not recovered/not | | 13 (10.3) | 48 (19.0) |
| | | resolved | 47.4 | 80.0 |
| | | Recovering/resolving | 7 (5.6) | 13 (5.2) |
| | | | 23.7 | 20.0 |
| | | Recovered/resolved | 78 (61.9) | 167 (66.3) |
| | | | 413.8 | 563.7 |
| | | Recovered/resolved with sequelae | 0 | 3 (1..2) |
| | | | | 4.0 |
| Frequent AEs (≥5% in any treatment group)^{†} | | | | |
| | | Viral upper respiratory tract infection | 14(11.1) | 49 (19.4) |
| | | | 47.44 | 85.29 |
| | | Upper respiratory tract infection | 6 (4.8) | 19 (7.5) |
| | | | 18.45 | 27.99 |
| | | Conjunctivitis^{‡} | 4 (3.2) | 28 (11.1) |
| | | | 10.54 | 42.65 |
| | | Injection site reaction | 0 | 17 (6.7) |
| | | | | 39.98 |
| | | Dermatitis atopic | 10 (7.9) | 6 (2.4) |
| | | | 31.63 | 10.66 |
| | | Headache | 6 (4.8) | 22 (8.7) |
| | | | 23.72 | 34.65 |
| AESIs - eye disorders | | | 7 (5.6) | 34 (13.5) |
| | | | 18.45 | 51.980 |
| | | Conjunctivitis^{‡} | 7 (5.6) | 33 (13.1) |
| | | | 18.45 | 50.64 |
| | | Keratoconjunctivitis | 0 | 1 (0.4) |
| | | | | 1.44 |
| | | Keratitis | 0 | 0 |
| AESIs - skin infections requiring systemic treatment | | | 7 (5.6) | 4 (1.6) |
| | | | 23.72 | 5.33 |
| AESIs - eczema herpeticum | | | 1 (0.8) | 1 (0.4) |
| | | | 2.64 | 1.33 |
| AESIs - malignancies diagnosed after randomization | | | 0 | 0 |

Overall, the safety profile at week 32 was comparable with the initial treatment period, as shown in Table 9 below.

**Table 9 *AEs collected during the exposure time in the continuation treatment period are shown. Responders and nonresponders presented as treated. A responder was defined as having IGA-0/1 or EASI-75 at week 16; ^{†}Classification according to MedDRA 20.0.**

| **Event, n (%) R** | | | **Week 32^{‡}** | | | | |
|---|---|---|---|---|---|---|---|
| | | | **Week 16 tralokinumab responders** | | **Week 16 tralokinu mab nonresponders** | **Week 16 placebo nonresponders** | **Week 16 placebo responders** |
| | | | **Tralokinu mab Q2W + TCS** (N=69, PYE=21.4 6) | **Tralokinu mab Q4W + TCS** (N=69, PYE=20.7 ) | **Tralokinu mab Q2W + TCS** (N=95, PYE=28.2 8) | **Tralokinu mab Q2W + TCS** (N=79, PYE=22.9 9) | **Placebo Q2W + TCS (N=41,** PYE=12. 25) |
| AEs | | | 48 (69.6) | 41 (59.4) | 62 (65.3) | 55 (69.6) | 26 (63.4) |
| | | | 540.5 | 439.6 | 654.2 | 552.5 | 359.3 |
| | Severity | | | | | | |
| | | Mild | 41 (59.4) | 35 (50.7) | 51 (53.7) | 41 (51.9) | 17 (41.5) |
| | | | 419.3 | 347.8 | 477.4 | 348.0 | 236.8 |
| | | Moderate | 16 (23.2) | 12 (17.4) | 30 (31.6) | 25 (31.6) | 12 (29.3) |
| | | | 111.8 | 91.78 | 173.3 | 195.8 | 122.5 |
| | | Severe | 2 (2.9) | 0 | 1 (1.1) | 2 (2.5) | 0 |
| | | | 9.32 | | 3.54 | 8.70 | |
| | Leading to discontinuation of IMP | | 0 | 1 (1.4) | 1 (1.1) | 2 (2.5) | 1 (2.4) |
| | | | | 4.83 | 3.54 | 8.70 | 8.17 |
| | Leading to withdrawal from trial | | 0 | 1 (1.4) | 0 | 2 (2.5) | 1 (2.4) |
| | | | | 4.83 | | 8.70 | 8.17 |
| | Recovered/resolved | | 43 (62.3) | 35 (50.7) | 56 (58.9) | 46 (58.2) | 22 (53.7) |
| | | | 451.9 | 328.5 | 509.2 | 400.2 | 302.1 |
| SAEs | | | 3 (4.3) | 0 | 2 (2.1) | 0 | 1 (2.4) |
| | | | 18.64 | | 7.07 | | 8.17 |
| Frequent AEs (≥5% in any treatment group) ^{∥} | | | | | | | |
| | Infections and infestations | | 30 (43.5) | 20 (29.0) | 40 (42.1) | 31 (39.2) | 17 (41.5) |
| | | | 205.0 | 125.6 | 233.4 | 195.8 | 187.8 |
| | Viral upper respiratory tract infection | | 12 (17.4) | 9 (13.0) | 20 (21.1) | 15 (19.0) | 7 (17.1) |
| | | | 60.57 | 48.30 | 99.01 | 65.25 | 65.32 |
| | | Conjunctivitis | 3 (4.3) | 0 | 3 (3.2) | 3 (3.8) | 1 (2.4) |
| | | | 13.98 | | 10.61 | 13.05 | 8.17 |
| | Upper respiratory tract infection | | 7 (10.1) | 3 (4.3) | 6 (6.3) | 3 (3.8) | 2 (4.9) |
| | | | 37.27 | 14.49 | 24.75 | 13.05 | 16.33 |
| | | Oral herpes | 3 (4.3) | 4 (5.8) | 4 (4.2) | 2 (2.5) | 1 (2.4) |
| | | | 13.98 | 19.32 | 17.68 | 8.70 | 8.17 |
| | General disorders and administration site conditions | | 10 (14.5) | 8 (11.6) | 12 (12.6) | 8 (10.1) | 0 |
| | | | 116.5 | 72.46 | 81.33 | 47.85 | |
| | | Injection site reaction | 5 (7.2) | 4 (5.8) | 5 (5.3) | 2 (2.5) | 0 |
| | | | 65.23 | 43.47 | 17.68 | 8.70 | |
| | Skin and subcutaneous tissue disorders | | 4 (5.8) | 2 (2.9) | 14 (14.7) | 10 (12.7) | 2 (4.9) |
| | | | 27.95 | 9.66 | 70.72 | 43.50 | 16.33 |
| | | AD | 1 (1.4) | 1 (1.4) | 8 (8.4) | 6 (7.6) | 2 (4.9) |
| | | | 4.66 | 4.83 | 28.29 | 26.10 | 16.33 |
| | Nervous system disorders | | 5 (7.2) | 8 (11.6) | 9 (9.5) | 4 (5.1) | 1 (2.4) |
| | | | 23.30 | 38.64 | 31.83 | 17.40 | 8.17 |
| | | Headache | 2 (2.9) | 5 (7.2) | 7 (7.4) | 2 (2.5) | 1 (2.4) |
| | | | 9.32 | 24.15 | 24.75 | 8.70 | 8.17 |
| | Gastrointestinal disorders | | 6 (8.7) | 7 (10.1) | 9 (9.5) | 12 (15.2) | 3 (7.3) |
| | | | 51.25 | 53.13 | 45.97 | 60.90 | 32.66 |
| | | Nausea | 3 (4.3) | 4 (5.8) | 3 (3.2) | 1 (1.3) | 0 |
| | | | 13.98 | 19.32 | 14.14 | 4.35 | |
| AESIs - eye disorders | | | 3 (4.3) | 1 (1.4) | 4 (4.2) | 6 (7.6) | 2 (4.9) |
| | | | 13.98 | 4.83 | 14.14 | 34.80 | 16.33 |
| | Conjunctivitis | | 3 (4.3) | 1 (1.4) | 4 (4.2) | 6 (7.6) | 1 (2.4) |
| | | | 13.98 | 4.83 | 14.14 | 30.45 | 8.17 |
| | Keratoconjunctivitis | | 0 | 0 | 0 | 1 (1.3) | 1 (2.4) |
| | | | | | | 4.35 | 8.17 |
| AESIs - skin infections requiring systemic treatment | | | 0 | 0 | 1 (1.1) | 2 (2.5) | 0 |
| | | | | | 3.54 | 8.70 | |
| AESIs - eczema herpeticum | | | 0 | 0 | 1 (1.1) | 1 (1.3) | 0 |
| | | | | | 3.54 | 8.70 | |
| AESIs - malignancies diagnosed after randomization | | | 0 | 1 (1.4) | 0 | 0 | 1 (2.4) |
| | | | | 4.83 | | | 8.17 |

Overall, tralokinumab used for a period up to 32 weeks in combination with TCS was well-tolerated and had an acceptable safety profile in adults with moderate-to-severe AD. The most frequent AEs (in ≥5% patients in any treatment group) occurring in a greater proportion of patients treated with tralokinumab Q2W plus TCS versus placebo Q2W plus TCS were viral upper respiratory tract infection, conjunctivitis, headache, upper respiratory tract infection, and injection site reaction. Conjunctivitis (as a preferred term) occurred in 10.9% of patients receiving tralokinumab plus TCS (total i.e. all tralokinumab groups over the entire treatment period), which is greater than the 2.6% of patients who experienced conjunctivitis with tralokinumab (total) in the Phase 2 trial (Wollenberg et al., The Journal of Allergy and Clinical Immunology 2019; 143: 135-141) and the 4.0% and 3.8% who experienced conjunctivitis with dupilumab as monotherapy (total) in SOLO1 and 2 (Simpson et al., The New England Journal of Medicine 2016; 375: 2335-2348), but lower than the 17.8% who experienced conjunctivitis with dupilumab plus TCS (total) in LIBERTY AD CHRONOS (Blauvelt et al., Lancet 2017; 389: 2287-2303). Unlike dupilumab, tralokinumab specifically binds to the IL-13 cytokine and does not affect IL-4 signalling. However, the mechanistic consequences resulting in ocular complications remain unclear. As an AESI, conjunctivitis was more frequently reported with tralokinumab Q2W plus TCS than placebo Q2W plus TCS at week 16 (13.1% versus 5.6%); however, these were all mild or moderate and most resolved. Notably, fewer skin infections requiring systemic treatment occurred with tralokinumab Q2W plus TCS compared to placebo Q2W plus TCS.

These findings corroborate dupilumab clinical trials and real-world data which show that patients with moderate-to severe AD have a greater risk of developing conjunctivitis compared to placebo patients (Akinlade B et al. Conjunctivitis in dupilumab clinical trials. Br J Dermatol 2019;181:459-473; Ferreira S, Torres T. Conjunctivitis in patients with atopic dermatitis treated with dupilumab. Drugs Context 2020;9:2020-2022-2023; Halling AS et al. Real-world evidence of dupilumab efficacy and risk of adverse events: A systematic review and meta-analysis. J Am Acad Dermatol 2020). Likewise, conjunctivitis cases with dupilumab were mostly mild to moderate and resolved during the treatment period with ophthalmologic treatment. The risk factors observed in this analysis are similar to risk factors identified in dupilumab clinical trials and real-world data ( Akinlade B et al. Conjunctivitis in dupilumab clinical trials. Br J Dermatol 2019;181:459-473; Ferreira S, Torres T. Conjunctivitis in patients with atopic dermatitis treated with dupilumab. Drugs Context 2020;9:2020-2022-2023; Thyssen JP et al. Incidence, prevalence, and risk of selected ocular disease in adults with atopic dermatitis. J Am Acad Dermatol 2017;77:280-286 e281; Treister AD et al. Risk Factors for Dupilumab-Associated Conjunctivitis in Patients With Atopic Dermatitis. JAMA Dermatol 2018;154:1208-1211; Wollenberg A et al. Laboratory safety of dupilumab in moderate-to-severe atopic dermatitis: results from three phase III trials (LIBERTY AD SOLO 1, LIBERTY AD SOLO 2, LIBERTY AD CHRONOS). Br J Dermatol 2020;182:1120-1135). However, the overall incidence rates of conjunctivitis compared to dupilumab clinical trials appear to be lower.

Tralokinumab + TCS was associated with lower rates of severe and serious infections, eczema herpeticum, and skin infections requiring systemic treatment versus placebo + TCS.

### Conclusions

Tralokinumab 300 mg Q2W+TCS is efficacious in treating moderate-to-severe AD, with a favorable safety profile. TCS use is significantly lower in tralokinumab-treated patients than placebo, demonstrating the potential steroid-sparing effects of tralokinumab. Tralokinumab maintained efficacy in responders when the dosing frequency was 2 weeks. Surprisingly, a response was also maintained at a dosing frequency of 4 weeks.

### Example 2: Tralokinumab/TCS combination therapy improves short term patient reported outcomes

Patient-reported outcomes (PRO) were used to assess the benefits of these treatment regimens as experienced by the patients.

### Methods

Patient reported outcomes (PROs) experienced by patients were investigated during the tralokinumab/TCS combination therapy trial described in Example 1. PROs included ≥ 4-point reduction in worst daily pruritus Numerical Rating Scale (NRS), reduction in eczema-related sleep interference, Patient-Oriented Eczema Measure (POEM), and change in Dermatology Life Quality Index (DLQI) scores.

At baseline the mean eczema-related sleep interference was 6.9, mean POEM was 22.3 and the mean DLQI score was 17.6. The baseline worst daily pruritus score was 7.7.

### Results

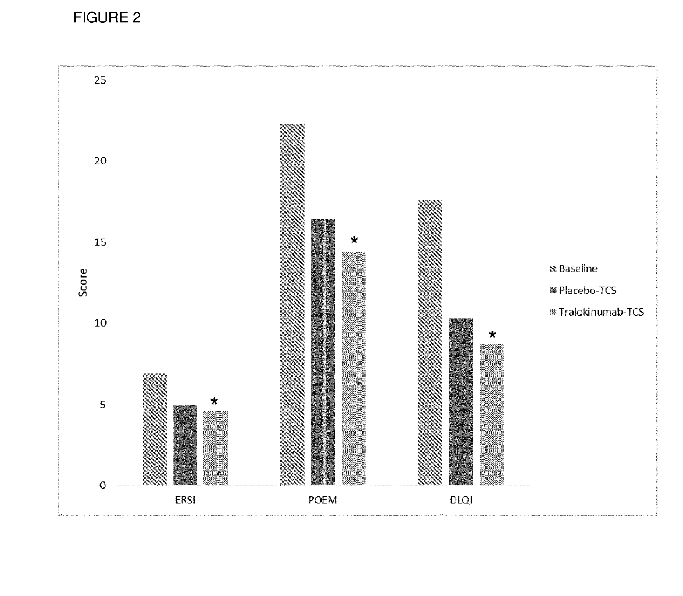

Following 2 weeks of treatment, eczema-related sleep interference, Patient-Oriented Eczema Measure (POEM), and change in Dermatology Life Quality Index (DLQI) scores were significantly reduced in tralokinumab/TCS patients compared to placebo/TCS patients (Figure 2). The mean eczema-related sleep interference decreased by 2.3 points compared to a decrease of 1.9 in the control group (p=0.037) (Figure 2). Mean POEM was significantly reduced in the tralokinumab/TCS treatment group (-7.9 compared to -5.9 for placebo/TCS; p=0.006). The mean reported DLQI score decreased 8.9 points following tralokinumab/TCS treatment (compared to -7.3 for placebo/TCS; p=0.011). Mean improvements from baseline for DLQI and POEM reached the minimal clinical important difference (MCID) in addition to statistical significance.

By week 3, the proportion of patients with a ≥ 4-point reduction in worst daily pruritus from baseline was significantly higher with tralokinumab treatment, compared to the control group (27.3% compared to 17.5%; p=0.029).

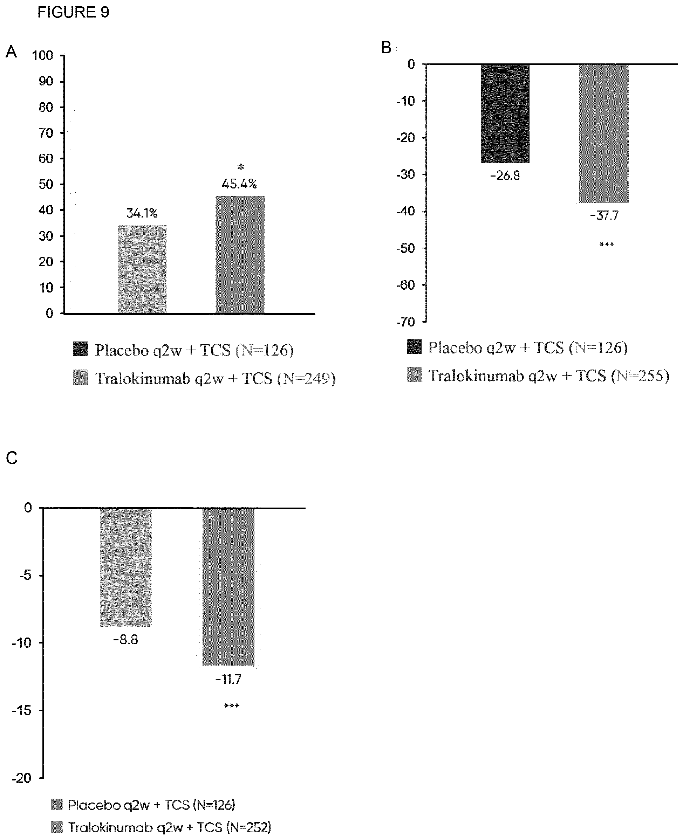

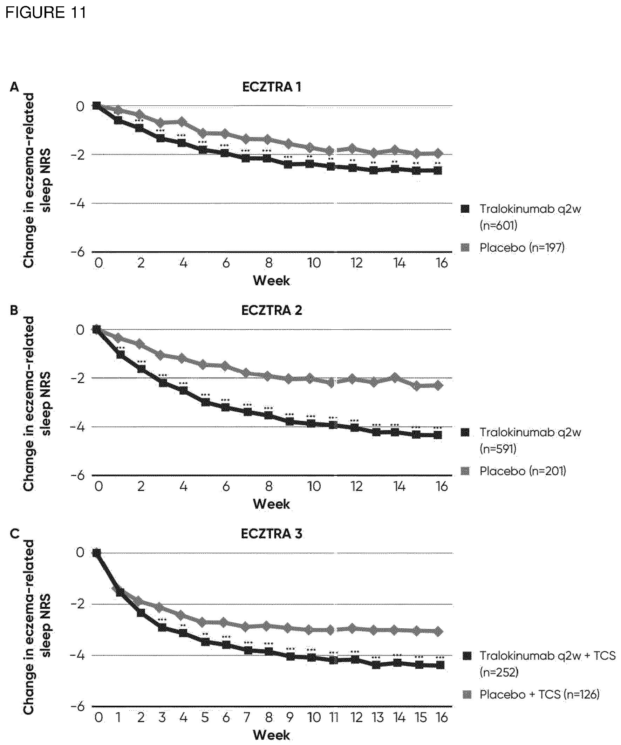

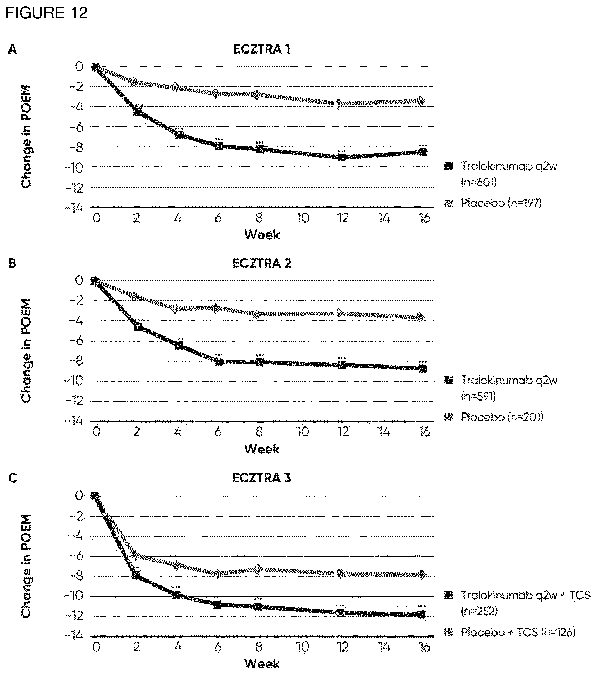

Figures 9, 11 and 12 shows that tralokinumab/TCS significantly improved all secondary endpoints at week 16 *versus* placebo/TCS.

### Conclusions

Early significant improvements in PROs were seen for tralokinumab/TCS combination therapy. Patients experience benefit from tralokinumab soon after treatment is initiated, with significant improvements following only 2 weeks of treatment.

### Example 3: Further analysis of Tralokinumab/TCS combination therapy efficacy

The Investigator's Global Assessment 0 or 1 (IGA 0/1; clear or almost clear skin) and/or ≥ 75% improvement of Eczema Area and Severity Index (EASI-75) assessed in Example 1 above are the regulatory primary efficacy endpoints in Phase 3 trials in AD. However, these endpoints do not comprehensively capture the full burden of AD.

Further analyses were used to assess the response to treatment with tralokinumab/TCS based on targets and timepoints typically used in clinical practice.

### Methods

Data from the experiment described in Example 1 were further analysed to assess the patient response at 3 and 6 months after treatment with tralokinumab/TCS. The response was based on clinician-assessed signs (EASI), patient-reported symptoms (pruritus and POEM), and patient-reported quality of life scores (DLQI and Patient Global Impression of Bother (PGI-B)). The chosen time points reflected typical follow-up for adult AD patients initiating a new treatment. All 252 patients who received tralokinumab/TCS in Example 1 were included in this analysis.

### Results

Table 10 shows the proportion of tralokinumab/TCS-treated patients achieving target outcomes at week 12.

**Table 10**

| **Outcome** | **Proportion of patients (%) treated with tralokinumab/TCS** | **Proportion of patients (%) in placebo/control group** |
|---|---|---|
| EASI-50 | 79 | 58.7 |
| ≥ 3 point reduction in worst daily pruritus | 59 | 42.1 |
| ≥ 4 point reduction in POEM | 78 | 56.1 |
| ≥ 4-point reduction in DLQI | 77 | 65.9 |
| ≥ 1-point reduction in PGI-B | 80.2 | 72.2 |

A high proportion of patients (79.4%; 196/247 assessed) achieved both a ≥ 1-point reduction in PGI-B and at least one of the other endpoints shown in Table 10.

At week 24, 81.0% (204/252) achieved EASI-50 (≥ 50% improvement of EASI) and 69.0% achieved EASI-75. For patients in the sub-group with both a ≥ 1-point reduction PGI-B and any of the other endpoints shown in Table 10 at 12 weeks (n=196), 90.8% achieved EASI-50 and 75.5% achieved EASI-75 at week 24.

The improvement in DLQI was maintained beyond week 16 in all treatment groups. The high level of maintained response with tralokinumab every other week or every 4 weeks was not associated with an increased use of TCS. The worst daily pruritus NRS scores decreased from 2.6 to 2.2 between weeks 16 and 32 in the tralokinumab plus TCS every other week group and from 3.0 to 2.7 in the tralokinumab plus TCS every 4 weeks group.

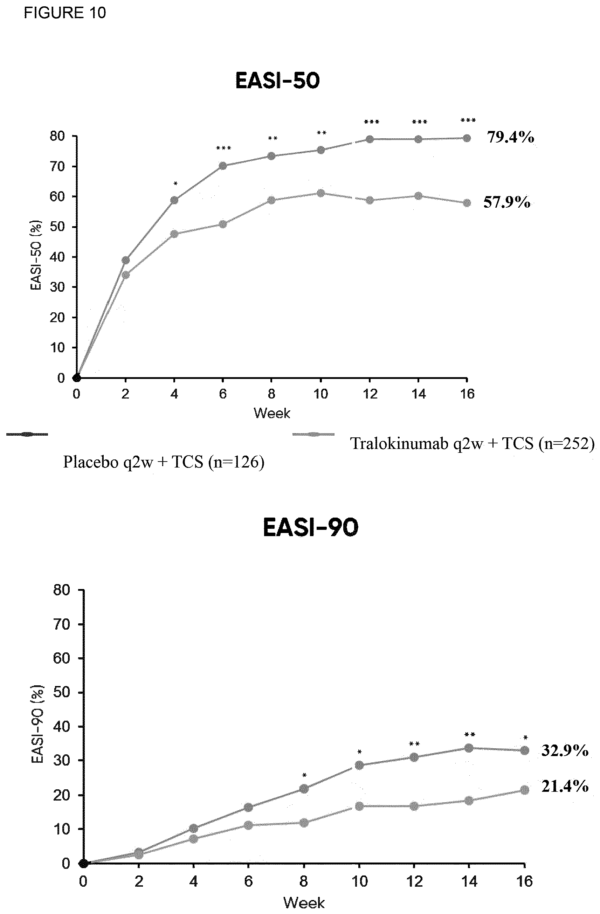

As shown in Figure 10, more patients achieved EASI-50 and EASI-90 with tralokinumab/TCS *versus* placebo/TCS at week 16.

### Conclusions

Tralokinumab/TCS combination therapy is associated with a high proportion of patients achieving and maintaining improvements in AD symptoms and AD-related quality of life after 3 and 6 months of treatment (typical follow up times in clinical practice). Tralokinumab combination therapy therefore provides a measurable benefit to patients at clinically relevant timepoints.

### Example 4: Tralokinumab monotherapy is effective in treating moderate to severe atopic dermatitis

Two 52-week trials of tralokinumab monotherapy in moderate-to-severe AD (ECZTRA 1 and ECZTRA 2) were conducted to assess the efficacy of tralokinumab alone.

### Methods

Patients were enrolled across Europe (ECZTRA 1: Germany, France and Spain; plus the UK, Italy, Poland and Russia in ECZTRA 2), North America (ECZTRA 1: USA; ECZTRA 2: USA and Canada), Asia (ECZTRA 1: Japan; ECZTRA 2: Korea), and Australia (ECZTRA 2) for two double-blind, randomized, placebo-controlled 52-week trials of tralokinumab monotherapy in moderate-to-severe AD. Inclusion criteria included: diagnosis of AD for >1 year, EASI score of ≥ 16 at baseline, IGA score of ≥ 3 at baseline, and an average pruritus NRS score of ≥ 4 prior at baseline.

Of 802 patients randomized in ECZTRA 1 and 794 patients randomized in ECZTRA 2, 50.7% and 48.7% had severe AD (IGA-4), respectively; mean EASIs were 32.4 and 32.2 at baseline. Although patients were enrolled into ECZTRA 1 and 2 had similar baseline disease characteristics, some baseline measures differed by region. For example, the proportion of patients with severe AD (IGA-4) was 50.7% (ECZTRA 1) and 48.7% (ECZTRA 2) for the overall study populations, while severe AD was higher in Japan (66.1%) and Australia (63.6%) as compared to Europe (52.6%/51.3%), North America (36.4%/43.2%) and Korea (43.6%). Of the patients with severe AD (IGA-4) at baseline, higher median baseline EASI scores were observed in Japan (46.6%) and Australia (47.2%), as compared to Europe (35.6%/41.2%), North America (35.3%/32.6%), and Korea (37.2%). These regional difference may explain the minor differences in therapeutic responses observed from ECZTRA 1 and ECZTRA 2.

Table 11 shows the demographic and clinical characteristics of randomized patients at baseline.

**Table 11**

| **Characteristic** | | **ECZTRA 1** | | **ECZTRA 2** | |
|---|---|---|---|---|---|
| | | Placebo (N=199) | Tralokinumab every other week (N=603) | Placebo (N=201) | Tralokinumab every other week (N=593) |
| Median age, years (IQR) | | 37.0 (26.0-49.0) | 37.0 (27.0-48.0) | 30.0 (23.0-46.0) | 34.0 (25.0-48.0) |
| Male, n (%) | | 123 (61.8) | 351 (58.2) | 114 (56.7) | 359 (60.5) |
| Race, n (%) | | | | | |
| | White | 138 (69.3) | 426 (70.6) | 123 (61.2) | 374 (63.1) |
| | Black | 18 (9.0) | 41 (6.8) | 17 (8.5) | 43 (7.3) |
| | Asian | 40 (20.1) | 120 (19.9) | 52 (25.9) | 154 (26.0) |
| | Other or missing data | 3 (1.5) | 16 (2.6) | 9 (4.5) | 22 (3.7) |
| Median disease duration, years (IQR) | | 28.0 (18.0-41.0) | 27.0 (19.0-38.0) | 25.0 (18.0-36.0) | 25.5 (17.0-39.0) |
| Median affected body surface area, % (IQR) | | 52.5 (31.0-77.0) | 50.0 (33.0-70.0) | 50.0 (31.0-74.0) | 50.0 (31.0-74.0) |
| Median EASI (IQR) | | 30.3 (22.0-41.5) | 28.2 (21.3-40.0) | 29.6 (20.6-41.4) | 28.2 (19.8-40.8) |
| IGA 4, n (%) | | 102 (51.3) | 305 (50.6) | 101 (50.2) | 286 (48.2) |
| Median total SCORAD (IQR) | | 70.8 (63.8-81.0) | 69.2 (61.5-79.1) | 69.9 (61.9-79.1) | 69.5 (60.5-79.1) |
| Median weekly average of worst daily pruritus NRS | | 7.9 (6.9-8.7) | 7.9 (6.7-8.9) | 8.1 (7.1-9.0) | 8.0 (7.0-9.0) |
| (IQR) | | | | | |
| Median DLQI (IQR) | | 16.0 (13.0-22.0) | 17.0 (12.0-22.0) | 18.0 (12.5-24.0) | 18.0 (13.0-23.0) |

The studies comprised an initial 16-week treatment period and a 36-week maintenance treatment period. After a two-week washout period for TCS and other topical treatments, tralokinumab or placebo was given subcutaneously every other week for 16 weeks. Patients were randomized 3:1 and administered subcutaneous tralokinumab 300 mg or placebo every 2 weeks (Q2W) for 16 weeks. Primary endpoints were IGA-0/1 and EASI-75, achieved without the use of rescue medication.

Depending on their randomization scheme, patients received a loading dose of tralokinumab (600 mg) or placebo on day 0.

Patients were instructed to use a stable dose of an emollient applied twice daily or more as needed for 2 weeks before the baseline visit and throughout the trials. Rescue treatment for AD could be provided if medically necessary at the discretion of the investigator, to control intolerable symptoms, and did not prevent transfer to maintenance or open-label treatment. However, patients who received rescue treatment were considered non-responders in the primary analyses (see Statistical analysis below). Patients were to temporarily discontinue treatment if a systemic corticosteroid or nonsteroidal systemic immunosuppressive drug was used as rescue (see the Methods section in the online Supplementary Appendix for more detail); however, patients continued study treatment if rescue was limited to topical medication.

After the 16-week initial treatment period, tralokinumab-treated patients who achieved the pre-specified criteria for clinical response - defined as achievement of IGA 0 (clear) or 1 (almost clear), or 75% improvement in Eczema Area and Severity Index (EASI-75) - were transferred to the maintenance treatment phase and re-randomised 2:2:1 to receive tralokinumab 300 mg every other week or every 4 weeks, or placebo for an additional 36 weeks. Patients who achieved clinical response criteria with placebo continued to receive placebo every other week to maintain blinding of the study. The placebo cohort from week 16 was not randomised in the 36-week maintenance treatment period, and was not included in these analyses. Patients who did not achieve the clinical response criteria at week 16 were transferred to open-label tralokinumab 300 mg every other week with optional use of TCS. In addition, patients were transferred from maintenance treatment to open-label tralokinumab after week 16 if they failed to meet specified clinical response criteria over a 4-week period. All patients had a final safety follow-up 16 weeks after the last dose of study medication, unless transferred to the long-term ECZTEND trial (NCT03587805).

Patients were assessed every other week for clinical efficacy and safety measures, and laboratory measurements were taken every fourth week throughout the trials. Serum samples for determination of presence or absence of anti-drug antibodies (ADA) were collected at Weeks 0, 4, 16, 28, 52, and 66. Samples confirmed positive for ADA in the confirmatory step underwent ADA endpoint titre determination and were analysed for the presence of neutralising antibodies (nAB).

### Results

### Tralokinumab monotherapy significantly improved IGA and EASI scores after 16 weeks

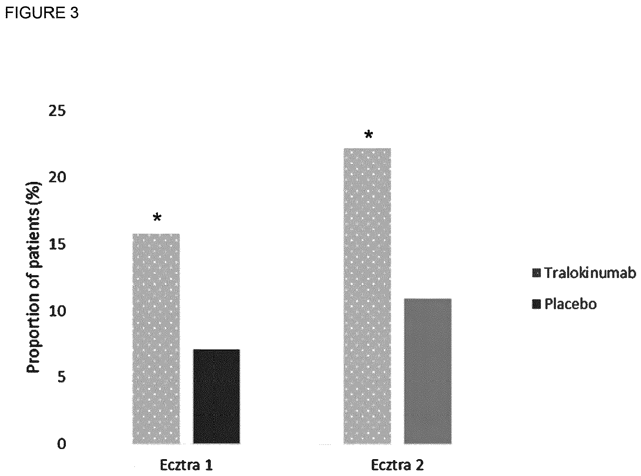

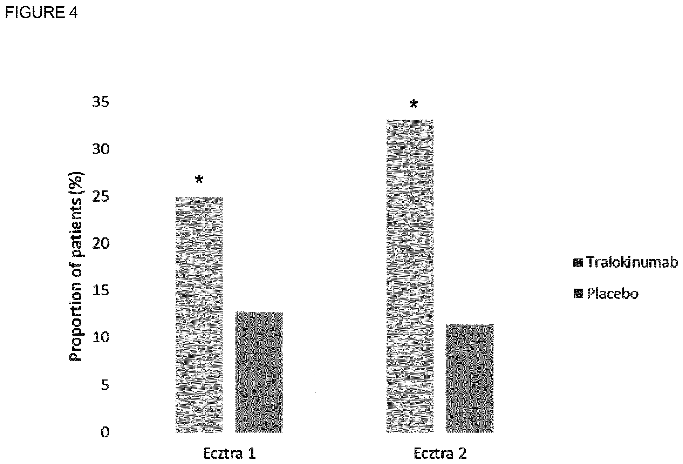

At week 16, IGA-0/1 responses were reported in significantly more patients treated with tralokinumab compared to the placebo control, in both ECZTRA 1 (15.8% tralokinumab compared to 7.1% placebo; p=0.002) and ECZTRA 2 (22.2% tralokinumab compared to 10.9% placebo; p<0.001) (see Figure 3). EASI-75 responses were 25.0% for tralokinumab compared to 12.7% for placebo (ECZTRA 1) and 33.2% for tralokinumab compared to 11.4% for placebo (ECZTRA 2) (both p<0.001) (see Figure 4).

The results of both primary and secondary outcomes at week 16 may be seen in Table 12 below:

**Table 12 *Treatment comparisons with Cochran-Mantel-Haenszel test, stratified by region and baseline IGA;**

| **Outcome*** | | | **ECZTRA 1** | | **ECZTRA 2** | |
|---|---|---|---|---|---|---|
| | | | Placebo (N=197) | Tralokinumab every other week (N=601 ) | Placebo (N=201) | Tralokinumab every other week (N=591 ) |
| **Primary endpoints^{§}** | | | | | | |
| | IGA | of 0 or 1 at week 16, n (%)^{†} | 14/197 (7.1) | 95/601 (15.8) | 22/201 (10.9) | 131/591 (22.2) |
| | | Difference versus placebo (95% CI) | | 8.6 (4.1, 13.1) *P=0.002* | | 11.1 (5.8, 16.4) *P*<0.001 |
| | EASI-75 at week 16, n (%)^{†} | | 25/197 (12.7) | 150/601 (25.0) | 23/201 (11.4) | 196/591 (33.2) |
| | | Difference versus placebo (95% CI) | | 12.1 (6.5, 17.7) *P*<0.001 | | 21.6 (15.8, 27.3) *P*<0.001 |
| EASI, LS mean % change from baseline (± SE) | | | **-28.5** (± 3.66) | -51.3^{§} (± 1.92) | -22.2 (±3.48) | -56.6^{§} (±1.79) |

| **Secondary endpoints** | | | | | | |
|---|---|---|---|---|---|---|
| | Adjusted mean change from baseline in SCORAD at week 16 | | -14.7 (1.80) | -25.2 (0.94) | -14.0 (1.79) | -28.1 (0.92) |
| | | Difference versus placebo (95% CI) | | -10.4 (-14.4, - 6.5) *P*<0.001 | | -14.0 (-18.0, - 10.1) *P*<0.001 |
| SCORAD, LS mean %-change from baseline (± SE^{c)} | | | -20,3 (2.72) | -36.7^{§} (± 1.42) | -20.6 (2.62) | -40.6^{§} (1.34) |
| | Improvement in worst daily pruritus NRS (weekly average) ≥4 points from baseline to week 16, n/N (%)^{††} | | 20/194 (10.3) | 119/594 (20.0) | 19/200 (9.5) | 144/575 (25.0) |
| | | Difference versus placebo (95% CI) | | 9.7 (4.4, 15.0) *P=0.002* | | 15.6 (10.3, 20.9) *P*<0.001 |
| | Adjusted mean change from baseline in DLQI at week 16 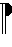 | | -5.0 (0.59) | -7.1 (0.31) | -4.9 (0.60) | -8.8 (0.30) |
| | | Difference versus placebo (95% CI) | | -2.1 (-3.4, - 0.8) *P=*0.002 | | -3.9 (-5.2, - 2.6) P<0.001 |

| Additional secondary endpoints | | | | | | |
|---|---|---|---|---|---|---|
| | Adjusted mean change from baseline in worst daily pruritus NRS (weekly average) at week 16 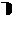 (SE) | | -1.7 (0.21) | -2.6 (0.11) | -1.6 (0.21) | -2.9 (0.11) |
| Difference versus placebo (95% CI) | | | | -0.9 (-1.4, - 0.4) *P*<0.001 | | -1.3 (-1.7, - 0.8) *P*<0.001 |
| | DLQI reduction ≥4 at week 16, n/N (%)^{†} | | 60/190 (31.6) | 258/578 (44.6) | 54/198 (27.3) | 325/577 (56.3) |
| | Difference versus placebo (95% CI) | | | 13.0 (5.4, 20.5) *P=*0.001 | | 28.9 (21.4, 36.3) *P*<0.001 |
| | Adjusted mean change from baseline in EASI at week 16 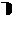 | | -9.0 (1.05) | -15.5 (0.55) | -7.0 (1.06) | -16.9 (0.55) |
| | Difference versus placebo (95% CI) | | | -6.4 (-8.8, - 4.1) *P*<0.001 | | -9.9 (-12.2, -7.5) *P*<0.001 |
| | EASI-50 at week 16, n (%)^{†} | | 42/197 (21.3) | 250/601 (41.6) | 41/201 (20.4) | 295/591 (49.9) |
| | | Difference | | 20.1 (13.3, | | 29.3 (22.5, |
| | | versus placebo (95% CI) | | 26.8) *P*<0.001 | | 36.1) *P*<0.001 |
| | | EASI-90 at week 16, n (%)^{†} | 8/197 (4.1) | 87/601 (14.5) | 11/201 (5.5) | 108/591 (18.3) |
| | | Difference versus placebo (95% CI) | | 10.3 (6.4, 14.1) *P*<0.001 | | 12.7 (8.3, 17.0) *P*<0.001 |

| **Other endpoints** | | | | | | |
|---|---|---|---|---|---|---|
| | | Worst daily pruritus NRS (weekly average) ≥3 at week 16, n/N (%)^{†,§} | 28/195 (14.4) | 177/597 (29.6) | 28/200 (14.0) | 199/583 (34.1) |
| | | Difference versus placebo (95% CI) | | 15.2 (9.2, 21.3) *P*<0.001 | | 20.1 (13.9, 26.2) *P*<0.001 |
| | | Adjusted mean change from baseline in SCORAD at week 2 | -5.0 (0.92) | -10.6 (0.53) | -3.9 (0.84) | -10.8 (0.49) |
| | | Difference versus placebo (95% CI) | | -5.6 (-7.7, - 3.5) *P*<0.001 | | -6.9 (-8.8, - 5.0) *P*<0.001 |
| | | SCORAD 75 at week 16, n (%)^{†} | 6/197 (3.0) | 53/601 (8.8) | 7/201 (3.5) | 68/591 (11.5) |
| | | Difference versus placebo (95% CI) | | 5.7 (2.5, 8.9) *P*=0.007 | | 8.0 (4.4, 11.6) *P*<0.001 |
| | | SCORAD 50 at week 16, n (%)^{†} | 23/197 (11.7) | 156/601 (26.0) | 29/201 (14.4) | 198/591 (33.5)1 |
| | | Difference versus placebo (95% CI) | | 14.1 (8.6, 19.6) *P*<0.001 | | 18.9 (12.8, 25.1) *P*<0.001 |
| | Adjusted mean change (SE) from baseline in worst daily pruritus NRS (weekly average) at week 1 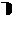 | | -0.2 (0.07) | -0.7 (0.04) | -0.3 (0.08) | -0.7 (0.05) |
| | Difference versus placebo (95% CI) | | | -0.4 (-0.6, - 0.3) *P*<0.001) | | -0.4 (-0.6, - 0.2) *P*<0.001 |
| | Adjusted mean change from baseline in DLQI at week 2 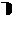 | | -2.5 (0.39) | -4.4 (0.22) | -2.2 (0.39) | -4.7 (0.23) |
| | | Difference versus placebo (95% CI) | | -2.0 (-2.8, - 1.1) *P*<0.001 | | -2.5 (-3.4, - 1.7) *P*<0.001 |

| | | | | | | |
|---|---|---|---|---|---|---|
| ^{†}Subjects who received rescue medication considered non-responders. Subjects with missing data at Week 16 imputed as non-responders; ^{‡}Based on patients in FAS with a baseline pruritus NRS weekly average of at least 4; ^{§}Based on patients in FAS with a baseline pruritus NRS weekly average of at least 3; 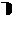 Data collected after permanent discontinuation of IMP or initiation of rescue medication not included. Repeated measurements model on post-baseline data: Change in measure = Treatment*Week + (Baseline measure)*Week + Region + Baseline IGA. | | | | | | |

### Tralokinumab monotherapy maintained IGA and EASI scores after 52 weeks

In ECZTRA 1 and ECZTRA 2, respectively, 185 and 227 patients were re-randomised after the initial 16-week treatment period 2:2:1 to continue treatment with tralokinumab every other week, reduce the dosing frequency of tralokinumab to every 4 weeks, or switch to placebo every 2 weeks.

The IGA and EASI 75 results may be in Table 13 below:

**Table 13 *Among patients with IGA of 0/1 at week 16 achieved without rescue medication after initial randomization to tralokinumab; ^{†}Among patients with EASI-75 at week 16 achieved without rescue medication after initial randomization to tralokinumab. EASI, Eczema Area and Severity Index; IGA, Investigator's Global Assessment.**

| | | **Outcome** | **ECZTRA 1** | | | **ECZTRA 2** | | |
|---|---|---|---|---|---|---|---|---|
| | | | **Placebo (N=35)** | **Tralokinu mab every other week (N=68)** | **Tralokinu mab every 4 weeks (N=76)** | **Placebo (N=46)** | **Tralokinum ab every other week (N=91)** | **Tralokinum ab every 4 weeks (N=89)** |
| | IGA of 0/1 at week 52, n/N (%)* | | 9/19 (47.4) | 20/39 (51.3) | 14/36 (38.9) | 7/28 (25.0) | 32/54 (59.3) | 22/49 (44.9) |
| | | Differe nce in percen tage versus placeb o (95% CI) | | 6.0 (-21.8, 33.7) *P*=0.68 | -9.5 (-37.1, 18.0) *P*=0.50 | | 34.1 (13.4, 54.9) *P*=0.004 | 19.9 (-1.2, 40.9) *P*=0.084 |
| | EASI-75 at week 52, n/N (%)^{†} | | 10/30 (33.3) | 28/47 (59.6) | 28/57 (49.1) | 9/42 (21.4) | 43/77 (55.8) | 38/74 (51.4) |
| | | Differe nce in percen tage versus placeb o (95% CI) | | 21.2 (-0.2, 42.6) *P=*0.056 | 11.7 (-8.7, 32.0) *P=*0.97 | | 33.7 (17.3, 50.0) *P*<0.001 | 30.0 (13.7, 46.4) *P*=0.001 |

At week 52, 51.3% (ECZTRA 1) and 59.3% (ECZTRA 2) of patients maintained IGA-0/1 with tralokinumab Q2W. Q4W responses were similar, with 38.9% (ECZTRA 1) and 44.9% (ECZTRA 2) of patients maintaining IGA-0/1. The results are shown in Figure 7. In patients who were re-randomised to placebo, 47.4% and 25.0% maintained response at week 52 in ECZTRA 1 and ECZTRA 2, respectively. There was no statistically significant difference in the proportion of patients maintaining an IGA 0/1 response at week 52 between patients continuing tralokinumab every other week and those who were initially treated with tralokinumab and switched to placebo at week 16 in ECZTRA 1. In ECZTRA 2, the difference in the proportion of patients maintaining IGA 0/1 at week 52 with tralokinumab every other week was significant compared to placebo; however, there was no statistically significant difference in IGA 0/1 response between tralokinumab every 4 weeks and placebo (*P*=0.084.

In ECZTRA 1 and ECZTRA 2, respectively, the proportion of patients who maintained EASI-75 at week 52, among patients achieving EASI-75 at week 16 on tralokinumab without rescue medication was 59.6% and 55.8% in patients who continued with tralokinumab every other week, 49.1% and 51.4% in patients who were re-randomised to tralokinumab every 4 weeks, and 33.3% and 21.4% in patients who were re-randomised to placebo.

Of the subjects randomised to tralokinumab, who did not achieve IGA 0 or 1 or EASI-75 at week 16 and were transferred to open-label Tralokinumab 300 mg Q2W + optional TCS, 20.8% in ECZTRA 1 and 19.3% in ECZTRA 2 achieved IGA 0 or 1 at week 52, and 46.1% in ECZTRA 1 and 39.3% in ECZTRA 2 achieved EASI-75 at week 52. The clinical response was mainly driven by continued tralokinumab treatment rather than optional TCS treatment. A higher proportion of subjects with IGA 2 or EASI-50 at week 16 achieved IGA 0 or 1 or EASI-75 at week 52 compared to subjects with IGA 3 or 4 or <EASI-50 at week 16.

In both studies, treatment with tralokinumab resulted in greater EASI-50 and EASI-90 responses than placebo at week 16, and a greater percent change in EASI score at week 16, with a separation between treatment arms (*P*<0.05) occurring from week 2 onward.

The proportion of patients achieving EASI-50 was greater with tralokinumab compared with placebo at each scheduled assessment in the initial treatment period, with a separation between treatment groups occurring from week 2. EASI-90 was achieved by more patients treated with tralokinumab than those who received placebo from week 4 to week 16, with a separation between treatment groups from week 6 onward. The percentage change from baseline in EASI score was greater with tralokinumab compared with placebo at each assessment including week 16 in both trials, with a separation between treatment groups (*P*<0.05) occurring from week 2 onward.

### Tralokinumab monotherapy has a favorable safety profile over 52 weeks

Adverse events (AEs) were similar between tralokinumab Q2W and placebo over 16 weeks; and the adverse-event profile over 52 weeks was comparable to the initial 16 weeks.

The incidence of AEs was comparable between tralokinumab and placebo in the initial treatment period of both studies. The majority of AEs were non-serious and mild or moderate in severity, with most resolved or resolving by the end of the treatment period, and few patients had AEs leading to permanent discontinuation of the investigational medicinal product (IMP). The most frequent AEs in the initial treatment period were worsening of AD and upper respiratory tract infections (mainly reported as common cold).

There was a low and comparable frequency of serious adverse events (SAEs) in both treatment groups in the initial treatment period of both studies. The majority of patients reporting SAEs recovered from the events. No marked differences in SAEs were observed between the treatment groups within each treatment period and between the treatment periods, and there was no clustering with respect to specific system organ class or event types.

Conjunctivitis - reported as part of standard AE reporting and as an AE of special interest - occurred with greater frequency in patients treated with tralokinumab than in those who received placebo. Most cases of conjunctivitis were mild and resolved by the end of the treatment period; one case led to treatment withdrawal. Tralokinumab was associated with lower rates of eczema herpeticum (0.5% tralokinumab vs 1.0% placebo in ECZTRA 1, and 0.3% tralokinumab vs 2.5% placebo in ECZTRA 2.

Skin infections requiring systemic treatment reported as an AE of special interest occurred more frequently in patients who received placebo than those who were treated with tralokinumab in ECZTRA 2; in ECZTRA 1, the frequencies were similar between the two groups. In ECZTRA 1, the reduction in *Staphylococcus aureus* colonisation on lesional skin, from baseline to week 16, as assessed by qPCR, was more than 10 times greater for tralokinumab-treated patients compared to those who received placebo: median 969 to 22 gene copies/cm² with tralokinumab compared with 649 to 238 gene copies/cm² with placebo.

Overall, in the maintenance treatment period, AEs were reported at a lower rate compared with tralokinumab every other week in the initial treatment period and the pattern of events was comparable to that in the initial treatment period. AEs were more frequently reported in the tralokinumab every other week group than in the tralokinumab every 4 weeks group. In total, 4 patients experienced SAEs in ECZTRA 1 (1 who received tralokinumab every other week and 3 who received tralokinumab every 4 weeks) and 3 patients had SAEs in ECZTRA 2, all in the tralokinumab every 4 weeks group. Two and three patients had AEs leading to permanent discontinuation of tralokinumab in ECZTRA 1 and ECZTRA 2, respectively.

A positive broad neutralising antibody (nAB) response was observed for 3 patients treated with tralokinumab in ECZTRA 1, and in 8 patients treated with tralokinumab in ECZTRA 2. Based on examination of tralokinumab concentrations, ADA responses, AEs, and IGA/EASI scores across the trials, it was considered that the presence of nAB did not have an impact on the efficacy and safety of tralokinumab for any of the subjects There were no noteworthy differences between treatment groups in laboratory values, vital signs, or electrocardiographic assessments. More subjects treated with tralokinumab experienced eosinophilia during the initial treatment period but the mean eosinophil levels returned to baseline values during the maintenance period, and the safety profile of subjects with eosinophilia (>1.5 x10⁹L) was comparable to that in the total trial population. Table 14 shows that the overall frequency and severity of adverse effects over 16 weeks.

### Summary of AEs and AESIs in the 16 week initial treatment period

**Table 14*Reporting adverse events at the level of PTs according to MedDRA 20.0 occurring in at least 5% of patients in any randomised group; ^{†}PTs according to MedDRA 20.0 include conjunctivitis, conjunctivitis allergic and conjunctivitis viral**

| | | | **ECZTRA 1** | | **ECZTRA 2** | |
|---|---|---|---|---|---|---|
| | | | **Placebo (N=196)** | **Tralokinumab every other week (N=602)** | **Placebo (N=200)** | **Tralokinumab every other week (N=592)** |
| Adverse events | | | | | | |
| | | Total number of adverse events | 491 | 1482 | 408 | 997 |
| | | Total number of serious adverse events | 11 | 24 | 6 | 10 |
| Patients with adverse events | | | | | | |
| | | ≥1 adverse event | 151 (77.0) | 460 (76.4) | 132 (66.0) | 364 (61.5) |
| | | ≥1 serious adverse event | 8 (4.1) | 23 (3.8) | 5 (2.5) | 10 (1.7) |
| | Severity | | | | | |
| | | Mild | 111 (56.6) | 385 (64.0) | 93 (46.5) | 288 (48.6) |
| | | Moderate | 98 (50.0) | 241 (40.0) | 84 (42.0) | 168 (28.4) |
| | | Severe | 16 (8.2) | 41 (6.8) | 16 (8.0) | 24 (4.1) |
| | Leading to permanent discontinuation of IMP | | 8 (4.1) | 20 (3.3) | 3 (1.5) | 9 (1.5) |
| | Not recovered/not resolved | | 35 (17.9) | 106 (17.6) | 25 (12.5) | 61 (10.3) |
| | Recovering/resolving | | 7 (3.6) | 36 (6.0) | 15 (7.5) | 20 (3.4) |
| | Recovered/resolved | | 139 (70.9) | 429 (71.3) | 125 (62.5) | 340 (57.4) |
| | Recovered/resolved with sequelae | | 0 | 6 (1.0) | 2 (1.0) | 9 (1.5) |
| Frequent AEs (≥5% in any treatment group), n (%)* | | | | | | |
| | Atopic dermatitis | | 75 (38.3) | 156 (25.9) | 67 (33.5) | 98 (16.6) |
| | Viral upper respiratory tract infection | | 41 (20.9) | 139 (23.1) | 17 (8.5) | 49 (8.3) |
| | Upper respiratory tract infection | | 2 (1.0) | 9 (1.5) | 17 (8.5) | 59 (10.0) |
| | Conjunctivitis^{†} | | 4 (2.0) | 43 (7.1) | 3 (1.5) | 18 (3.0) |
| | Skin infection | | 3 (1.5) | 6 (1.0) | 11 (5.5) | 12 (2.0) |
| | Pruritus | | 10 (5.1) | 32 (5.3) | 5 (2.5) | 12 (2.0) |
| | Headache | | 10 (5.1) | 28 (4.7) | 6 (3.0) | 16 (2.7) |
| AESIs - eye disorders | | | 7 (3.6) | 62 (10.3) | 6 (3.0) | 33 (5.6) |
| | Conjunctivitis^{†} | | 7 (3.6) | 60 (10.0) | 5 (2.5) | 31 (5.2) |
| | Keratoconjunctivitis | | 0 | 1 (0.2) | 0 | 2 (0.3) |
| | Keratitis | | 0 | 3 (0.5) | 1 (0.5) | 1 (0.2) |
| AESIs - skin infections requiring systemic treatment | | | 4 (2.0) | 13 (2.2) | 22 (11) | 21 (3.5) |
| AESIs - eczema herpeticum | | | 2 (1.0) | 3 (0.5) | 5 (2.5) | 2 (0.3) |
| AESIs - malignancies diagnosed after randomization | | | 0 | 0 | 0 | 1 (0.2) |

Table 15 shows that the overall frequency and severity of adverse effects during the 36-week maintenance period.

| **Event** | | **ECZTRA 1** | | | **ECZTRA 2** | | |
|---|---|---|---|---|---|---|---|
| | | **Place bo (N = 35)** | **Tralokinu mab Every Other Week (N = 68)** | **Tralokinu mab Every Four weeks (N = 76)** | **Place bo (N = 46)** | **Tralokinu mab Every Other Week (N = 91)** | **Tralokinu mab Every Four weeks (N = 89)** |
| **Adverse or serious adverse event- no. (%)** | | | | | | | |
| At least 1 adverse event | | 25 (71.4) | 54 (79.4) | 53 (69.7) | 32 (69.6) | 62 (68.1) | 56 (62.9) |
| At least 1 serious adverse event | | 0 | 1 (1.5) | 3 (3.9) | 0 | 0 | 3 (3.4) |
| Adverse event leading to withdrawal from trial | | 0 | 1 (1.5) | 1 (1.3) | 0 | 2 (2.2) | 1 (1.1) |

| **Adverse events (≥5% in any treatment group) - no. (%)*** | | | | | | | |
|---|---|---|---|---|---|---|---|
| Infections and infestations | | | | | | | |
| | Viral upper respiratory tract infection | 4 (11.4) | 14 (20.6) | 18 (23.7) | 7 (15.2) | 9 (9.9) | 6 (6.7) |
| | Upper respiratory tract infection | 1 (2.9) | 1 (1.5) | 2 (2.6) | 3 (6.5) | 14 (15.4) | 9 (10.1) |
| | Bronchitis | 2 (5.7) | 3 (4.4) | 7 (9.2) | 0 | 1 (1.1) | 3 (3.4) |
| | Influenza | 1 (2.9) | 4 (5.9) | 3 (3.9) | 1 (2.2) | 2 (2.2) | 1 (1.1) |
| | Nasopharyngi tis | 2 (5.7) | 0 | 3 (3.9) | 0 | 3 (3.3) | 2 (2.2) |
| | Conjunctivitis | 0 | 3 (4.4) | 4 (5.3) | 2 (4.3) | 5 (5.5) | 1 (1.1) |
| Other AEs | | | | | | | |
| | Atopic dermatitis | 13 (37.1) | 11 (16.2) | 14 (18.4) | 9 (19.6) | 13 (14.3) | 14 (15.7) |
| | Injection site reaction | 1 (2.9) | 5 (7.4) | 7 (9.2) | 0 | 4 (4.4) | 4 (4.5) |
| | Headache | 3 (8.6) | 6 (8.8) | 2 (2.6) | 0 | 2 (2.2) | 2 (2.2) |
| | Asthma | 0 | 4 (5.9) | 1 (1.3) | 3 (6.5) | 2 (2.2) | 3 (3.4) |
| | Dry eye | 0 | 0 | 0 | 3 (6.5) | 1 (1.1) | 0 |
| | Hypertension | 0 | 1 (1.5) | 2 (2.6) | 3 (6.5) | 1 (1.1) | 1 (1.1) |
| | Allergic conjunctivitis | 2 (5.7) | 3 (4.4) | 1 (1.3) | 1 (2.2) | 2 (2.2) | 3 (3.4) |
| | Liver function test increased/abn ormal | 2 (5.7) | 0 | 0 | 0 | 1(1.1) | 1 (1.1) |
| | Oropharyngea l pain | 2 (5.7) | 1 (1.5) | 0 | 0 | 1 (1.1) | 2 (2.2) |
| | Back pain | 0 | 3 (4.4) | 4 (5.3) | 0 | 3 (3.3) | 2 (2.2) |
| | Pruritus | 1 (2.9) | 2 (2.9) | 4 (5.3) | 2 (4.3) | 2 (2.2) | 2 (2.2) |

The incidence of eye disorders was collected as adverse events of special interest due to the increased incidence of eye disorders observed in clinical trials of dupilumab. Conjunctivitis occurred in less than 8% of patients receiving tralokinumab in either study in the intial treatment period and in less than 6% in any tralokinumab treatment arm in the maintenance periods. Almost all cases of conjunctivitis were mild/moderate and 1 case led to withdrawal.

### Conclusion

Both trials show that tralokinumab monotherapy significantly improved IGA and EASI scores in patients with moderate-to-severe AD after 16 weeks, with a favorable safety profile. Tralokinumab monotherapy maintained efficacy over 52 weeks in responders when the dosing frequency was 2 weeks and, surprisingly, a similar response was also maintained at a dosing frequency of 4 weeks.

### Example 5: Tralokinumab monotherapy improves patient reported outcomes

As discussed above, AD is a disease with a substantial patient burden, with symptoms including pain, itching, and sleep disturbance. The effect of tralokinumab monotherapy on patient-reported outcomes (PROs) were evaulated in phase 3 trials (ECZTRA 1, NCT03131648; ECZTRA 2, NCT03160885).

### Methods

PROs were analysed for patients in the ECZTRA 1 and 2 trials described in Example 4. Assessed PROs included ≥ 4-point reduction in worst daily pruritus Numerical Rating Scale (NRS); reduction in eczema-related sleep interference; Patient-Oriented Eczema Measure (POEM), SCORAD and change in Dermatology Life Quality Index (DLQI) scores.

The baseline eczema-related sleep interference scores were 6.3 (ECZTRA 1) and 6.9 (ECZTRA 2). The baseline mean POEM score was 22.8 for both ECZTRA 1 and ECZTRA 2. Baseline mean DLQI values were 16.8 (ECZTRA 1) and 17.7 (ECZTRA 2). The baseline worst daily pruritus scores were 7.7 (ECZTRA 1) and 7.9 (ECZTRA 2).

### Results

In both studies, there were significant differences between the tralokinumab and placebo groups with regard to all secondary endpoints in example 4.

Results for other endpoints may be seen in Table 16 below.

**Table 16.LS: Least squares, SE: Standard error. If needed to control intolerable symptoms of atopic dermatitis, patients were permitted to receive rescue treatment at the discretion of the investigator. a) Data after initiation of rescue medication or permanent discontinuation of treatment was excluded from the analyses. b) Subjects who received rescue treatment or had missing data were treated as non-responders. The percentage is calculated relative to the number of subjects with POEM ≥4 at baseline *p<0.05, ^{#}p< 0.01, ^{§}p<0.001.**

| **Monotherapy** | | | | |
|---|---|---|---|---|
| | **ECZTRA 1 week 16** | | **ECZTRA 2 week 16** | |
| | **Placebo** | **TRADENAME 300 mg Q2W** | **Placebo** | **TRADENAME 300 mg Q2W** |
| ***Patients randomised*** | 199 | 603 | 201 | 593 |
| Eczema-related sleep NRS, LS mean change from baseline (SE)^{a)} | -1.9 (0.2) | -2.6^{#} (0.1) | -1.5 (0.2) | -2.9^{§} (0.1) |
| POEM, LS mean change from baseline (SE)^{a)} | -3.0 (0.66) | -7.6^{§} (0.35) | -3.7 (0.66) | -8.8^{§} (0.33) |
| POEM (≥4-point improvement), responders^{b)} | 18.0% (35/194) | 43.0%^{§} (253/588) | 22.1% (44/199) | 54.4%^{§} (319/586) |

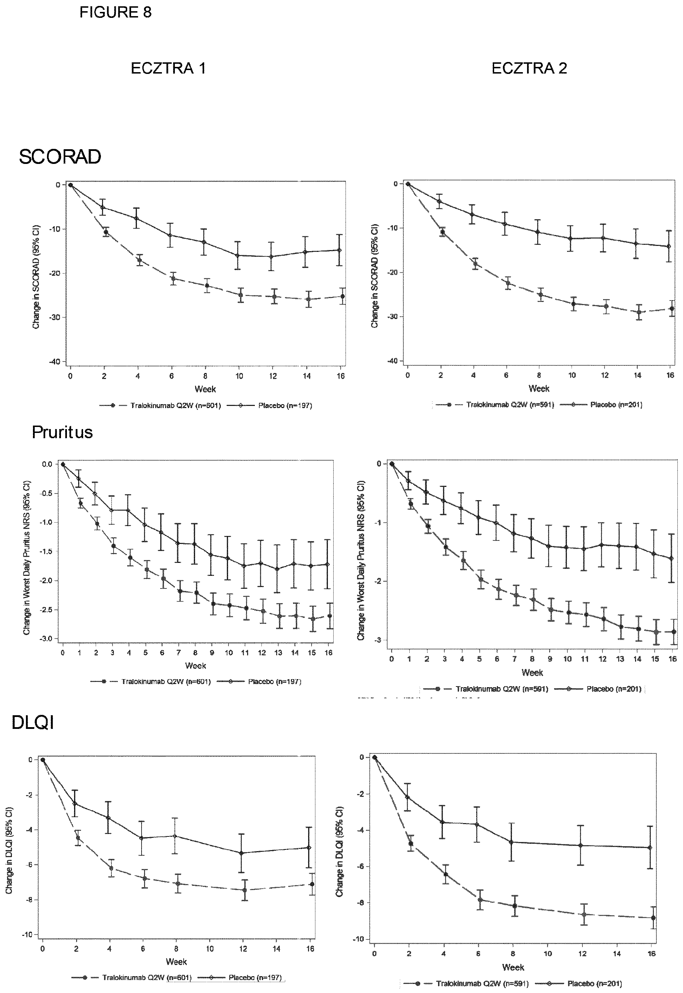

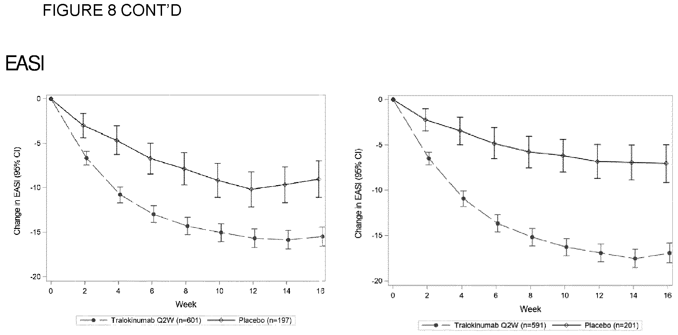

A significantly greater reduction in SCORAD was achieved in the tralokinumab group, compared with the placebo group at week 16 (*P*<0.001 vs placebo in both trials). The change from baseline in SCORAD was greater with tralokinumab compared with placebo throughout the initial treatment period, and a separation between the treatment groups (*P*<0.001) was observed from week 2 onward (Figure 8). The change from baseline in SCORAD sleep score was greater with tralokinumab vs PBO at each week, with a separation between treatment groups (p<0.01) from week 2.

A significantly greater proportion of patients achieved a reduction of worst daily pruritus NRS (weekly average) of ≥4 with tralokinumab (20% and 25%) compared with placebo (10.3% and 9.5%) at week 16 in ECZTRA 1 (*P*=0.002) and ECZTRA 2 (*P*<0.001), respectively. The reduction in worst daily pruritus NRS (weekly average) was greater with tralokinumab compared with placebo throughout the initial treatment period, with a separation between the treatment groups (*P*<0.05) observed from week 1 onward (Figure 8). By week 3, the proportion of patients with a ≥ 4-point reduction in worst daily pruritus from baseline (7.7/7.9) was significantly higher with tralokinumab compared to placebo in ECZTRA 1 (8.1% compared to 1.5%; p<0.001) and ECZTRA 2 (7.5% compared to 3.0%; p=0.021).

Eczema-related sleep NRS (weekly average) improved in both treatment groups in both studies, ECZTRA 1 and 2. The adjusted mean change (standard error [SE]) from baseline at week 16 was greater with tralokinumab vs Placebo: -2.6 (0.12) vs -1.9 (0.23); p=0.007 in ECZTRA 1 and -2.9 (0.12) vs -1.5 (0.22); p<0.001 in ECZTRA 2.

Change from baseline in eczema-related sleep NRS was larger with tralokinumab vs placebo at each week, with a separation between-treatment groups (p<0.001) from week 1.

By week 2 in both trials there was a decreased score in eczema-related sleep interference in tralokinumab-treated patients, which was significantly different from the control groups. In ECZTRA 1 tralokinumab-treatment decreased eczema-related sleep interference by 0.9 compared to a decrease of 0.4 in the control group (p <0.001). Eczema-related sleep interference changes in the ECZTRA 2 trial were -1.1 for tralokinumab compared to -0.4 for control (p<0.001).

SCORAD sleep score improved in both treatment groups in both studies. The adjusted mean change (SE) from baseline at week 16 was greater with tralokinumab vs placebo: -2.6 (0.14) vs -1.8 (0.26); p=0.004 in ECZTRA 1 and -3.0 (0.14) vs -1.8 (0.28); p<0.001 in ECZTRA 2, with separation between groups from week 2.

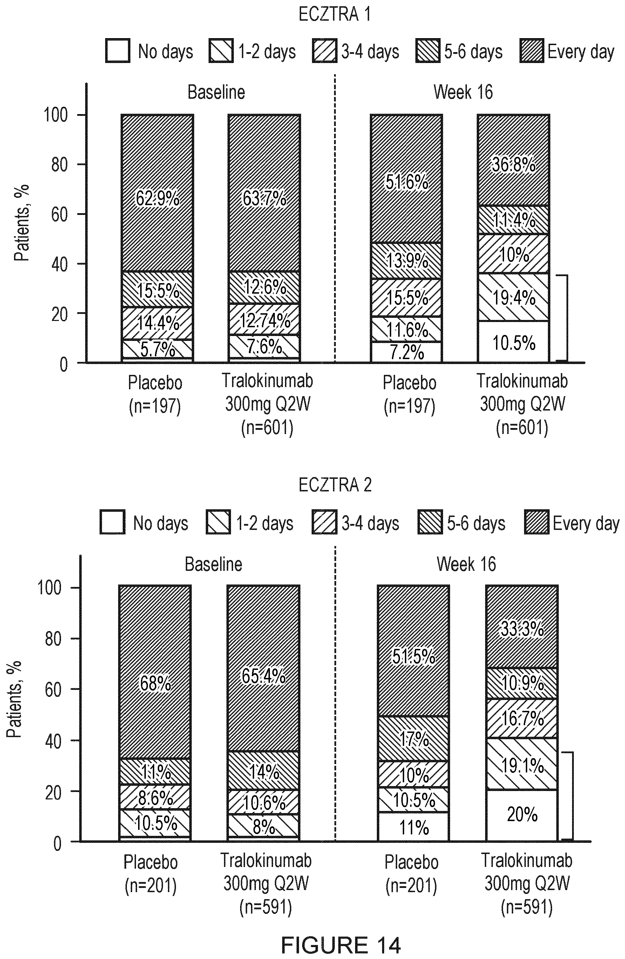

POEM sleep score improved to a greater extent with tralokinumab compared with PBO from week 2 onwards (p<0.001) and the adjusted mean change (SE) from baseline to week 16 was greater with tralokinumab vs PBO: -1.2 (0.07) vs -0.6 (0.13) in ECZTRA 1 and - 1.3 (0.07) vs -0.6 (0.13) in ECZTRA 2 (both p<0.001). A greater proportion of tralokinumab treated patients (35.9-39.1%) reported "No day" or "1-2 days" of sleep interference at week 16 vs placebo (see Figure 14).

Change from baseline in DLQI was greater with tralokinumab than with placebo in ECZTRA 1 (-7.1 vs -5.0; *P*=0.02) and ECZTRA 2 (-8.8 vs -4.9; *P*<0.001) at week 16, and at each scheduled assessment throughout the initial treatment period, with a separation between treatment arms (*P*<0.05) from week 2 onwards.

By week 2 in both trials there was a decreased score in POEM and DLQI in tralokinumab treated patients, which was significantly different from the placebo:
DLQI was decreased by 4.4 for tralokinumab compared to 2.5 for control (p<0.001) in ECZTRA 1, and decreased by 4.7 for tralokinumab compared to 2.2 for control (p<0.001) in ECZTRA 2. Mean improvements from baseline for DLQI reached the minimal clinical important difference (MCID) in addition to statistical significance.

Mean POEM was significantly reduced from baseline relative to placebo in ECZTRA 1 (-4.0 for tralokinumab compared to -1.3 for control; p<0.001) and ECZTRA 2 (-4.6 for tralokinumab compared to -1.6 for control; p<0.001). Mean improvements from baseline for POEM reached the minimal clinical important difference (MCID) in addition to statistical significance.

Figures 8, 11 and 12 shows data for secondary endpoints up to 16 weeks. Improvements in SCORAD, pruritus, DLQI, ERSI and POEM were observed early in the studies (see Figures 8, 11 and 12). Improvement in EASI started soon after the start of treatment and was greater with tralokinumab than with placebo in both studies (Figure 8).

### Conclusions

Tralokinumab monotherapy results in early significant improvements in PROs, as observed in the two trials. In particular, patients experience early benefit from tralokinumab monotherapy after initiation of treatment with significant improvement after only 2 weeks.

### Example 6: Conjunctivitis in tralokinumab-treated adult patients with moderate-to-severe atopic dermatitis: pooled results from five clinical trials

### Materials and methods

Patients treated with tralokinumab 300 mg or placebo (PBO) every 2 weeks were pooled from five trials: Ph 3 ECZTRA 1/2 (tralokinumab monotherapy) and ECZTRA 3 (tralokinumab in combination with topical corticosteroids), Ph 2 ECZTRA 5 (vaccine response in tralokinumab-treated patients with AD) and Ph 2b (efficacy and safety evaluation of tralokinumab) trials. Adverse events (AEs) were summarised for the initial treatment period (16 weeks for ECZTRA and 12 weeks for Ph 2b). Conjunctivitis was defined as an AE of special interest (AESI); events were captured from the AE form (ECZTRA) or by Medical Dictionary for Regulatory Activities search (Ph 2b). Cochran-Mantel-Haenszel weights were applied to calculate adjusted AE incidences to take into account different randomisation rates between tralokinumab and PBO.

### Results

Adult patients treated with tralokinumab (n=1605) or PBO (n=680) were included in the analysis. During the initial treatment period, a conjunctivitis AESI (preferred terms conjunctivitis, conjunctivitis allergic, conjunctivitis bacterial and conjunctivitis viral) occurred in 126 (7.5%) pts treated with tralokinumab vs 21 (3.2%) with PBO. Overall, 145 and 23 events of conjunctivitis occurred in the tralokinumab and PBO groups, respectively, the majority were mild (68% vs 65%) or moderate (30% vs 35%) in severity; ophthalmologists confirmed 30% of conjunctivitis events across the tralokinumab (39 events) and PBO (6 events) groups. A similar percentage of events in the tralokinumab and PBO groups resolved (78.6% vs 73.9%) or were resolving (2.8% vs 4.3%) during the initial treatment period, respectively; no events were serious and two events led to permanent discontinuation of tralokinumab. A higher baseline of AD severity and previous history of allergic conjunctivitis were associated with increased conjunctivitis incidence. Median time to first event was similar for both groups (50.0 days vs 51.5 days); however, duration of conjunctivitis was longer in the tralokinumab (21.0 days vs 14.5 days) group. The majority of patients received treatment for their conjunctivitis (81% of tralokinumab pts vs 63% of PBO pts). Common treatments included ophthalmic antiallergics (28% vs 42%), anti-infectives (30% vs 11%), corticosteroids (22% vs 11%) and combined corticosteroids and anti-infectives (13.5% vs 15.8%).

Comparison of incidence rates of conjunctivitis in dupilumab-treated patients (300 mg q2w) after 16 weeks of treatment shows dupilumab has a higher overall incidence rate compared to tralokinumab (9.3% vs 7.5%) with a greater percentage of events being moderate or severe (Akinlade et al. 2019). This suggests that specific IL-13 neutralisation is associated with lower conjunctivitis risk than dual IL-4/IL-13 blockade, and that blocking IL-4 signalling, which has been shown to skew T cells towards a Th1/Th17 phenotype, may result in more severe conjunctivitis (Reyes et al. 2014; Stern et al. 2005).

### Conclusion

The overall incidence of conjunctivitis, identified as an AESI in the initial treatment period of the AD pool, was higher for tralokinumab than for PBO, but the majority of cases were mild or moderate in severity. The majority of the conjunctivitis events were concomitantly treated and resolved or were resolving during the trials.

### Example 7: Clinical responses to tralokinumab in patients with atopic dermatitis who initially achieved sub-optimal responses and continued treatment

**Materials and methods:** Patients who did not achieve clinical response, IGA 0/1 or EASI-75, in the ECZTRA 1 and 2 trials at week 16 were transferred to open-label tralokinumab 300 mg q2w plus optional TCS for an additional 36 weeks. This *post hoc* analysis of pooled data from both studies assessed clinical responses during open-label treatment in patients who initially achieved sub-optimal responses to tralokinumab after 16 weeks.

**Results:** In the pooled analysis, 686 of 1196 (57.4%) tralokinumab-treated patients (360 and 326 from ECZTRA 1 and 2, respectively) were transferred to open-label treatment at week 16. The proportion of patients achieving IGA 0/1 or EASI-75 with open-label tralokinumab plus optional TCS continued to increase, and 20.1% and 42.9% of these patients achieved IGA 0/1 and EASI-75, respectively, at week 52. More than half of the responder proportions at week 52 were achieved within 8 weeks of starting open-label treatment; 11.4% and 31.9% achieved IGA 0/1 and EASI-75 by week 24. An alternative analysis assessed patients (49.1%) who used concomitant anti-inflammatory treatment (including TCS) to be non-responders; the response rates in patients who did not use concomitant anti-inflammatory treatment were 13.9% and 25.7% for IGA 0/1 and EASI-75, respectively. When considering the level of AD disease activity at week 16, in patients who had EASI-50 to -74 at week 16, 53.2% achieved EASI-75 at week 52, and in the subgroup of patients with IGA 2 at week 16, 36.5% achieved IGA 0/1 at week 52. In patients who had EASI 25-50 % at week 16, 40.7 % achieved EASI-75 at week 52 and in patients having IGA 3 at week 16, 17.1 % achieved IGA 0/1 at week 52.

In patients with EASI <25 at week 16, 29.3% achieved EASI-75 at week 52, and in patients with IGA 4 at week 16, 7.6% achieved IGA 0/1 at week 52.

**Discussion:** These data show that the majority of patients with initial sub-optimal responses to tralokinumab subsequently achieved EASI-75 with continued treatment and that responses correlated with AD disease activity achieved by week 16. In addition, clinical responses with continued treatment did not appear to be driven by the addition of optional TCS.

### Example 8: Impact of targeting IL-13 on Staphylococcus aureus colonisation

**Material & Methods:** In the ECZTRA 1 phase III trial, patients with moderate-to-severe AD were randomised 3:1 to subcutaneous tralokinumab 300 mg or placebo (PBO) every 2 weeks for an initial 16 weeks. The change in skin colonisation by *S. aureus* at week 16 in patients was an exploratory endpoint. Absolute abundance of *S. aureus* on lesional skin was assessed by rotation of sterile swabs on the skin, followed by rtPCR of extracted DNA. Association of *S. aureus* colonisation with disease severity and select biomarkers was assessed. The ratio between treatment groups in relative reduction of *S. aureus* colonisation from baseline to week 16 was assessed by a t-test of changes in log-transformed values.

**Results:** 802 patients were randomised 603:199 to tralokinumab:PBO; 50.7% had severe AD (IGA-4) at baseline; mean EASI score was 32.4. *S. aureus* colonisation correlated with disease severity (EASI score) at baseline and week 16. *S. aureus* colonisation further correlated significantly with gene expression of biomarkers, including IL-13, IL-22 and hBD2, at baseline and week 16. Median *S. aureus* abundance was reduced more from baseline to week 16 in patients receiving tralokinumab (n=555; from 969 to 22 gene copies/cm²) vs PBO (n=184; from 649 to 238 gene copies/cm²), with a 10-fold greater reduction for tralokinumab vs PBO treated patients (ratio=0.09; p<0.0001). Use of rescue medication did not impact the results.

In patients not using rescue medication (64.2 % of tralokinumab and 53.8% of placebo patients did not use rescue medication), there was a significant reduction in tralokinumab vs placebo treated patients (ratio=0.12; p<0.0001).

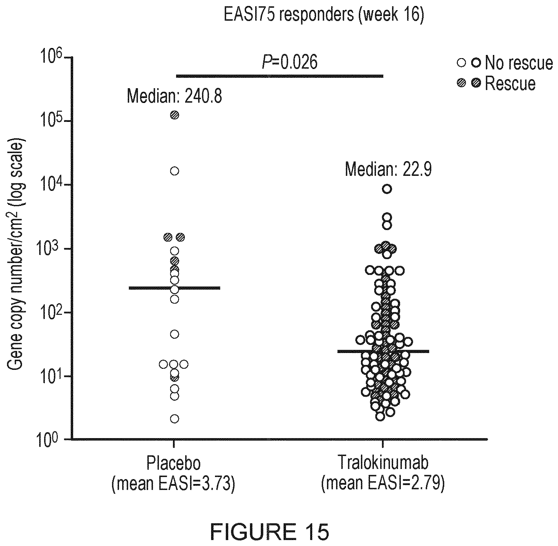

When comparing EASI-75 responders from tralokinumab and placebo groups there was a lower median *S.aureus* colonisation at week 16 in tralokinumab treated patients than in placebo EASI-75 responders, see Figure 15.

There was a significant reduction from baseline to week 16 in median count for tralokinumab vs placebo (-96.6%, p<0.0001 vs +34.2 %, ns).

**Conclusions:** Treatment with tralokinumab was associated with significant reduction in *S*. *aureus* colonisation in lesional skin compared with PBO in adult patients with moderate-to-severe AD. This suggests that reduction of *S. aureus* colonisation by neutralisation of IL-13 contributes to the efficacy of tralokinumab in improving the AD hallmarks and breaking the cycle of itching, scratching, skin barrier dysfunction, and immune-mediated inflammation.

### Example 9: Impact of targeting IL-13 on anxiety and depression

**Materials and methods:** Patients in the ECZTRA 1-3 phase III trials were assessed for anxiety and depression using the Hospital Anxiety and Depression Scale (HADS).

**Results:** HADS results are summarised for ECZTRA 1-3 and the monotherapy pool in Table 17 below (initial treatment period: FAS).

**Table 17:Composite estimand: subjects who received rescue medication considered non-responders. Subjects with missing data at Week 16 imputed as non-responders. Mantel-Haenszel analysis of risk difference stratified by region and baseline IGA (and trial ID for ECZTRA 1+2). P-value based on Cochran-Mantel-Haenszel test, stratified by region and baseline IGA (and trial ID for ECZTRA 1+2). Hypothetical estimand: data collected after permanent discontinuation of Tralokinumab or initiation of rescue medication not included. In case of no post-baseline assessments before initiation of rescue medication, the Week 4 change will be imputed as 0. Repeated measurements analysis.**

| | **ECZTRA 1** | | **ECZTRA 2** | | **ECZTRA 1+2** | | **ECZTRA 3** | |
|---|---|---|---|---|---|---|---|---|
| | **Tralo Q2W** | **Placebo** | **Tralo Q2W** | **Placebo** | **Tralo Q2W** | **Placebo** | **Tralo Q2W+TCS** | **Placebo +TCS** |
| | **(N=601)** | **(N=197)** | **(N=591)** | **(N=201)** | **(N=1192)** | **(N=398)** | **(N=252)** | **(N=126)** |
| **HADS anxiety and HADS depression scores <8 at Week 16 in subjects with baseline HADS anxiety or HADS depression subscale scores of ≥8^{a}** | | | | | | | | |
| n | 289 | 103 | 292 | 93 | 581 | 196 | 102 | 54 |
| Resp., % | 21.8% | 18.4% | 38.4% | 16.1% | 30.1% | 17.3% | 53.9% | 29.6% |
| Diff. (95% CI) | 3.0% (-5.8, 11.8) | | 21.2% (11.8, 30.5) | | **11.7%** (5.3, 18.2) | | 24.8% (9.3, 40.4) | |
| p-value | 0.52 | | <0.001 | | 0.001 | | 0.003 | |

| **Change from baseline to Week 16 in HADS total score^{b}** | | | | | | | | |
|---|---|---|---|---|---|---|---|---|
| n | 333 | 95 | 417 | 97 | 750 | 192 | 226 | 104 |
| Adj. mean change (SE) | -2.3 (0.26) | -1.8 (0.48) | -3.3 (0.26) | -1.4 (0.50) | -2.8 (0.18) | -1.6 (0.35) | -4.4 (0.37) | -2.2 (0.54) |
| Diff. (95% CI) | -0.5 (-1.5, 0.6) | | -1.9 (-3.0, -0.7) | | -1.2 (-1.9, -0.4) | | -2.2 (-3.4, -0.9) | |
| p-value | 0.38 | | 0.001 | | 0.003 | | 0.001 | |

There was a higher proportion of responders with HADS anxiety and HADS depression scores <8 at Week 16 among subjects with a baseline HADS anxiety or HADS depression subscale scores of ≥8 in the tralokinumab group compared to the placebo group across all trials (p=0.52 for ECZTRA 1, p<0.001 for ECZTRA 2, p=0.003 for ECZTRA 3). The treatment difference between tralokinumab and placebo was similar in ECZTRA 2 (21.2%) and ECZTRA 3 (24.8%), whereas it was modest in ECZTRA 1 (3.0%) (see Table 17.

Furthermore, for all pivotal phase 3 trials the HADS mean total score was reduced more from baseline to Week 16 in the tralokinumab group compared to placebo (p=0.38 for ECZTRA 1, p=0.001 for ECZTRA 2 and 3) (see Table 17).

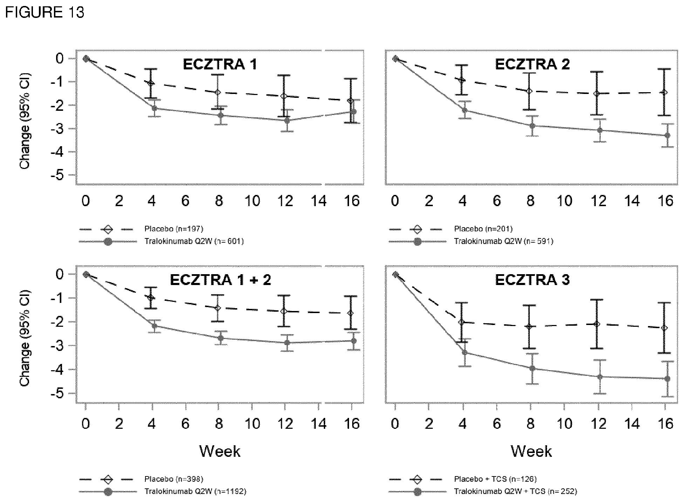

For all pivotal phase 3 trials, a higher mean change from baseline in the HADS total score was observed in the tralokinumab group compared to placebo from Week 4 and onwards (Figure 13). The same pattern was observed when considering the 2 individual components of the HADS total score, i.e. the HADS anxiety score and the HADS depression score.

In ECZTRA 5, the mean change from baseline to Week 16 in the total HADS score was higher with tralokinumab compared to placebo with an adjusted mean change of -2.4 in the tralokinumab group and -0.6 in the placebo group leading to a treatment difference of -1.8 (p=0.033).

**Conclusions:** Treatment with tralokinumab was associated with significant reduction in anxiety and depression, as measured by HADS, compared with PBO in adult patients with moderate-to-severe AD.

### Example 10: Impact of targeting IL-13 on health-related quality of life

### Short Form (36) Health Survey (SF-36)

**Material & Methods:** Short Form (36) Health Survey (SF-36) was assessed for patients in the ECZTRA 1 and 2 phase III trials described above.

**Results:** For both trials, there was an improvement in both the physical and mental component summary scores of SF-36 in both treatment groups through the initial treatment period.

In ECZTRA 1, the mean physical component summary score increased from 44.5 at baseline to 51.3 at Week 16 in the tralokinumab group and from 44.7 to 50.2 in the placebo group. The mean mental component summary score increased from 43.6 at baseline to 48.2 at Week 16 in the tralokinumab group and from 42.4 to 46.0 in the placebo group.

In ECZTRA 2, the mean physical component summary score increased from 44.3 at baseline to 51.5 at Week 16 in the tralokinumab group and from 43.4 to 48.6 in the placebo group. The mean mental component summary score increased from 43.6 at baseline to 48.4 at Week 16 in the tralokinumab group and from 43.2 to 44.8 in the placebo group.

For both trials, the change from baseline in both the physical and mental component summary scores was larger in the tralokinumab group compared with the placebo group at Week 16 is shown in Table 18 below.

**Table 18:Change from baseline to Week 16 in SF-36, initial treatment period: FAS. Hypothetical estimand: data collected after permanent discontinuation of Tralokinumab or initiation of rescue medication not included. In case of no post-baseline assessments before initiation of rescue medication, the Week 2 change will be imputed as 0. Repeated measurements analysis.**

| | **ECZTRA 1** | | **ECZTRA 2** | |
|---|---|---|---|---|
| | **Tralokinumab Q2W** | **Placebo** | **Tralokinumab Q2W** | **Placebo** |
| | **(N=601)** | **(N=197)** | **(N=591)** | **(N=201)** |
| **Change from baseline to Week 16 in SF-36 Physical Component Summary Score** | | | | |
| n | 333 | 94 | 417 | 97 |
| Adj. mean change (SE) | 4.5 (0.30) | 2.9 (0.56) | 5.8 (0.29) | 3.2 (0.57) |
| Diff. (95% CI) | 1.6 (0.3, 2.8) | | 2.6 (1.4, 3.9) | |
| p-value | 0.013 | | <0.001 | |

| **Change from baseline to Week 16 in SF-36 Mental Component Summary Score** | | | | |
|---|---|---|---|---|
| n | 333 | 94 | 417 | 97 |
| Adj. mean change (SE) | 2.5 (0.42) | 0.3 (0.78) | 3.5 (0.38) | 0.5 (0.76) |
| Diff. (95% CI) | 2.3 (0.5, 4.0) | | 3.0 (1.3,4.7) | |
| p-value | 0.010 | | <0.001 | |

In EZCTRA 1, the adjusted mean change in the SF-36 Physical Component Summary Score was 4.5 in the tralokinumab group compared to 2.9 in the placebo group leading to a treatment difference of 1.6 (p=0.013). The adjusted mean change in the SF-36 Mental Component Summary Score was 2.5 for tralokinumab and 0.3 for placebo leading to a treatment difference of 2.3 (p=0.010). In ECTZRA 2, the adjusted mean change in the SF-36 Physical Component Summary Score was 5.8 in the tralokinumab group compared to 3.2 in the placebo group leading to a treatment difference of 2.6 (p<0.001). The adjusted mean change in the SF-36 Mental Component Summary Score was 3.5 for tralokinumab and 0.5 for placebo leading to a treatment difference of 3.0 (p<0.001).

**Conclusions:** Treatment with tralokinumab was associated with significant improvement in SF-36 Physical and Mental Component Summary Scores compared with PBO in adult patients with moderate-to-severe AD.

### EuroQoL 5-Dimension Health Questionnaire 5 Level (EQ-5D-5L)

**Material & Methods:** EuroQoL 5-Dimension Health Questionnaire 5 Level (EQ-5D-5L) was assessed for patients in the ECZTRA 1-3 phase III trials.

**Results:** For all the trials, the subjects' perception of their health improved in both treatment groups through the initial treatment period based on both parts of EQ-5D-5L (i.e. the EQ-5D-5L index score and the EQ-5D-5L VAS).

In ECZTRA 1, the mean EQ-5D-5L index score increased from 0.553 at baseline to 0.787 at Week 16 in the tralokinumab group and from 0.571 to 0.739 in the placebo group. The mean EQ-5D-5L VAS score increased from 53.8 at baseline to 72.7 at Week 16 in the tralokinumab group and from 54.7 to 69.4 in the placebo group.

In ECZTRA 2, the mean EQ-5D-5L index score increased from 0.544 at baseline to 0.779 at Week 16 in the tralokinumab group and from 0.543 to 0.683 in the placebo group. The mean EQ-5D-5L VAS score increased from 58.0 at baseline to 74.4 at Week 16 in the tralokinumab group and from 55.7 to 68.0 in the placebo group.

In ECZTRA 3, the mean EQ-5D-5L index score increased from 0.561 at baseline to 0.839 at Week 16 in the tralokinumab+TCS group and from 0.589 to 0.766 in the placebo+TCS group. The mean EQ-5D-5L VAS score increased from 59.1 at baseline to 76.1 at Week 16 in the tralokinumab+TCS group and from 59.4 to 72.8 in the placebo+TCS group.

The change from baseline for both sections of the EQ-5D-5L are summarised for ECZTRA 1-3 in **Error! Reference source not found.** 19 below.

**Table 19 Endpoints related to EQ-5D-5L for the pivotal phase 3 trials, initial treatment period: FAS. Hypothetical estimand: data collected after permanent discontinuation of Tralokinumab or initiation of rescue medication not included. In case of no post-baseline assessments before initiation of rescue medication, the Week 4 change will be imputed as 0. Repeated measurements analysis.**

| | **ECZTRA 1** | | **ECZTRA 2** | | **ECZTRA 3** | |
|---|---|---|---|---|---|---|
| | **Tralo Q2W** | **Placebo** | **Tralo Q2W** | **Placebo** | **Tralo Q2W+TCS** | **Placebo +TCS** |
| | **(N=601)** | **(N=197)** | **(N=591)** | **(N=201)** | **(N=252)** | **(N=126)** |
| **Change from baseline to Week 16 in EQ-5D-5L index score** | | | | | | |
| n | 333 | 95 | 418 | 97 | 226 | 104 |
| Adj. mean change (SE) | 0.154 (0.010) | 0.092 (0.019) | 0.187 (0.010) | 0.085 (0.019) | 0.263 (0.01) | 0.176 (0.02) |
| Diff. (95% CI) | 0.062 (0.020, 0.105) | | 0.102 (0.060, 0.145) | | 0.087 (0.047, 0.127) | |
| p-value | 0.004 | | <0.001 | | <0.001 | |

| **Change from baseline to Week 16 in EQ-5D-5L VAS** | | | | | | |
|---|---|---|---|---|---|---|
| n | 333 | 95 | 418 | 97 | 226 | 104 |
| Adj. mean change (SE) | 11.0 (0.92) | 6.3 (1.70) | 12.8 (0.74) | 5.8 (1.48) | 16.3 (1.16) | 13.0 (1.69) |
| Diff. (95% CI) | 4.7 (0.9, 8.5) | | 7.0 (3.8, 10.3) | | 3.2 (-0.8, 7.3) | |
| p-value | 0.016 | | <0.001 | | 0.12 | |

Across all trials, the subjects' perception of their health improved, based on the EQ-5D-5L index score. The change from baseline to Week 16 in the EQ-5D-5L index score was higher in the tralokinumab group compared to the placebo group across all trials with similar treatment differences between trials (p=0.004 for ECZTRA 1, p<0.001 for ECZTRA 2 and 3). For all trials, differences were observed from first assessment at Week 4 and onwards.

Furthermore, the subjects' perception of their health improved, based on the EQ-5D-5L VAS across all trials. The change from baseline to Week 16 in the EQ-5D-5L VAS was higher in the tralokinumab group compared to the placebo group across all trials with a higher treatment difference in ECZTRA 2 compared to ECZTRA 1 and ECZTRA 3 (p=0.016 for ECZTRA 1, p<0.001 for ECZTRA 2, p=0.12 for ECZTRA 3). For all pivotal trials, differences were observed from Week 4 and onwards).

In ECZTRA 5, the mean change from baseline to Week 16 in the EQ-5D-5L index score was higher with tralokinumab compared to placebo with an adjusted mean change of 0.130 in the tralokinumab group and 0.097 in the placebo group leading to a treatment difference of 0.034 (p=0.19). The mean change in EQ-5D-5L VAS was 8.3 in the tralokinumab group and 0.6 in the placebo group leading to a treatment difference of 7.6 (p=0.004).

**Conclusions:** Treatment with tralokinumab was associated with significant improvement in the subjects' perception of their health as measured by EQ-5D-5L compared with PBO in adult patients with moderate-to-severe AD.

### Example 11: Use of Tralokinumab to treat AD patients who are not adequately controlled by cyclosporine A or have contraindications to cyclosporine A

This example reports the initial results of the ECZTRA 7 trial relating to tralokinumab in combination with topical corticosteroids in subjects with severe atopic dermatitis who are not adequately controlled with or have contraindications to oral cyclosporine A.

This was an international, multi-site, phase 3 trial. The trial was randomised, double-blind, and placebo-controlled.

**Table 20 Objectives and endpoints**

| **Objectives^{a}** | **Endpoints^{a}** | |
|---|---|---|
| Primary objective | Primary endpoint | |
| To demonstrate that tralokinumab in combination with TCS is superior to placebo in combination with TCS in treating severe AD in subjects who are not adequately controlled with or have contraindications to oral CSA. | | • EASI75 at Week 16. |
| Secondary objective | Secondary endpoints | |
| To evaluate the efficacy of tralokinumab in combination with TCS on severity and extent of AD, itch, and health-related quality of life compared to placebo in combination with TCS. | *Severity and extent of AD* | |
| | | • IGA score of 0 (clear) or 1 (almost clear) at Week 16. |
| | | • IGA score of 0 (clear) or 1 (almost clear) at Week 26. |
| | | • Change in SCORAD from baseline to Week 16. |
| | | • Change in SCORAD from baseline to Week 26. |
| | | • EASI75 at Week 26. |
| | *Itch* | |
| | | • Reduction of Worst Daily Pruritus NRS (weekly average) of ≥4 from baseline to Week 16. |
| | | • Reduction of Worst Daily Pruritus NRS (weekly average) of ≥4 from baseline to Week 26. |
| | *HRQoL* | |
| | | • Change in DLQI score from baseline to Week 16. |
| | | • Change in DLQI score from baseline to Week 26. |
| To evaluate the safety of tralokinumab in combination with TCS when treating severe AD in subjects who are not adequately controlled with or having contraindications to oral CSA compared to placebo in combination with TCS. | | • Number of AEs. |
| | | • Presence of ADA (yes/no). |
| Other obj ective | Other endpoints | |
| To evaluate the efficacy of tralokinumab in combination with TCS on health care resource utilisation compared to placebo with TCS. | | • Amount of TCS used from baseline to Week 16. |
| | | • Amount of TCS used from baseline to Week 26. |
| | | • Number of days without topical treatment use from baseline to Week 16. |
| | | • Number of days without topical treatment use from baseline to Week 26. |

### Trial design

The trial consisted of a screening period of 2 to 6 weeks and a treatment period of 26 weeks. After completion of Week 26 visit or the safety follow-up visit, eligible subjects were invited to enter a long-term extension trial conducted under a separate protocol (LP0162-1337, ECZTEND). Subjects who did not transfer to ECZTEND after completion of Week 26 visit were to complete a 14-week off-treatment follow-up period for the assessment of safety, pharmacokinetic (PK), and anti-drug antibody (ADA). Subjects found eligible following the screening period were randomised 1:1 to tralokinumab 300 mg+TCS or placebo+TCS every 2 weeks (Q2W). Randomisation was stratified by prior cyclosporine A (CSA) use (yes/no), country (Germany: yes/no) and baseline disease severity (Investigator's Global Assessment [IGA]= 3 or 4).

A total of 250 subjects were planned to be randomised. 277 subjects were randomised, 275 received investigational medicinal product (IMP; tralokinumab or placebo), and all 275 subjects were included in the full analysis set and the safety analysis set.

### Inclusion criteria

All of the following criteria had to be met for a subject to be included in the trial:
1. Signed and dated informed consent has been obtained prior to any protocol-related procedures.
2. Age 18 and above.
3. Diagnosis of AD as defined by the Hanifin and Rajka (1980) criteria for AD (27).
4. History of AD for ≥1 year.
5. Subjects who had a recent history (within 1 year before the screening visit) of inadequate response to treatment with topical medications.
   - Inadequate response was defined as failure to achieve and maintain remission or a low disease activity state (comparable to IGA 0=clear to 2=mild) despite treatment with a daily regimen of TCS of medium to higher potency (±TCI as appropriate), applied for at least 28 days or for the maximum duration recommended by the product prescribing information (e.g. 14 days for super-potent TCS), whichever was shorter.
   - Subjects with documented systemic treatment for AD in the past year were also considered as inadequate responders to topical treatments and were potentially eligible for treatment with tralokinumab after appropriate washout.
6. AD involvement of ≥10% body surface area at screening and baseline according to component A of SCORAD.
7. EASI score ≥20 at screening and baseline.
8. An IGA score of ≥3 at screening and at baseline.
9. A Worst Daily Pruritus numeric rating scale (NRS) average score of ≥4 during the week prior to baseline.
   - Worst Daily Pruritus NRS score at baseline was calculated from daily assessments of worst itch severity (Worst Daily Pruritus NRS) during the 7 days immediately preceding randomisation (Day -6 to 0). A minimum of 4 Worst Daily Pruritus NRS scores out of the 7 days was required to calculate the baseline average score. For subjects who did not have at least 4 scores reported during the 7 days immediately preceding the planned randomisation date, randomisation was postponed until this requirement was met, but without exceeding the 6 weeks' maximum duration for screening.
10. Subjects applied a stable dose of emollient twice daily (or more, as needed) for at least 14 days before randomisation.
11. Women of childbearing potential were required to use a highly effective* form of birth control (confirmed by the investigator) throughout the trial and at least for 16 weeks (5 half-lives) after last administration of IMP.
   *A highly effective method of birth control was defined as one which results in a low failure rate (less than 1% per year) such as bilateral tubal occlusion, intrauterine device (IUD), intrauterine hormone-releasing system (IUS), combined (oestrogen and progestogen containing) hormonal contraception associated with inhibition of ovulation (oral, intravaginal, transdermal), progestogen-only hormonal contraception associated with inhibition of ovulation (oral, injectable, implantable), sexual abstinence (when this is in line with the preferred and usual life style of the subject), vasectomised partner (given that the subject is monogamous). The subjects must have used the contraceptive method continuously for at least 1 month prior to the pregnancy test at baseline. A female was defined as not being of childbearing potential if she was postmenopausal (at least 12 months with no menses without an alternative medical cause prior to screening), or surgically sterile (hysterectomy, bilateral salpingectomy, or bilateral oophorectomy).
12. Documented history by a physician of either:
   - No prior CSA exposure and not a candidate for CSA treatment due to any of the following:
      i. medical contraindications (e.g. uncontrolled hypertension on medication) or hypersensitivity to CSA active substance or excipients.
      ii. use of prohibited concomitant medications (e.g. statins, digoxin, macrolide, antibiotics, barbiturates, anti-seizure, non-steroidal anti-inflammatory drugs, diuretics, angiotensin-converting-enzyme inhibitors, St John's Wort).
      iii. increased susceptibility to CSA-induced renal damage (elevated creatinine) and liver damage (elevated function tests), or increased risk of serious infections.
   - Previously exposed to CSA, and CSA treatment should not be continued or restarted due to any of the following:
      i. intolerance or unacceptable toxicity (e.g. elevated creatinine, elevated liver function tests, uncontrolled hypertension, paraesthesia, headache, nausea, hypertrichosis).
      ii. inadequate response to CSA (defined as flare of AD on CSA tapering after a maximum of 6 weeks of high dose [5 mg/kg/day] to maintenance dose [2 to 3 mg/kg/day] or a flare after a minimum of 3 months on maintenance dose). Flare was defined as increase in signs or symptoms leading to escalation of therapy, which could be an increase in dose, a switch to a higher potency class of TCS, or the start of another systemic non-steroidal immunosuppressive drug.
      iii. requirement for CSA at doses >5 mg/kg/day, or duration beyond those specified in the prescribing information (>1 year).

### Exclusion criteria

Any of the following criteria disqualified a subject from participating in the trial:
1. Subjects for whom TCS were medically inadvisable e.g. due to important side effects or safety risks (including intolerance to treatment, hypersensitivity reactions, significant skin atrophy, systemic effects etc.) in the opinion of the investigator.
2. Concurrent enrolment in another interventional clinical trial.
3. Previous randomisation in a tralokinumab clinical trial.
4. Active dermatologic conditions that could confound the diagnosis of AD or interfere with assessment of treatment, such as scabies, cutaneous lymphoma, or psoriasis.
5. Known active allergic or irritant contact dermatitis that was likely to interfere with the assessment of severity of AD.
6. Use of tanning beds or phototherapy (narrow band ultraviolet B [NBUVB], ultraviolet B [UVB], ultraviolet A1 [UVA1], psoralen + ultraviolet A [PUVA]), within 6 weeks prior to randomisation.
7. Treatment with the following medications within 4 weeks prior to randomisation:
   - Systemic immunosuppressive/immunomodulating drugs (e.g. methotrexate, CSA, azathioprine, mycophenolate mofetil, Janus kinase inhibitors).
   - Systemic corticosteroid use (excludes topical, inhaled, or intranasal delivery).
   - Three or more bleach baths during any week within the 4 weeks.
8. Treatment with topical PDE-4 inhibitor within 2 weeks prior to randomisation.
9. Receipt of live attenuated vaccines within 30 days prior to the date of randomisation and during the trial including the safety follow-up period.
   - Receipt of inactive/killed vaccinations (e.g. inactive influenza) was allowed, provided they were not administered within 5 days before/after any trial visit.
10. Receipt of any marketed biological therapy (e.g. immunoglobulin, anti-IgE) including dupilumab or investigational biologic agents:
   - Any cell-depleting agents including but not limited to rituximab: within 6 months prior to randomisation, or until lymphocyte count returns to normal, whichever was longer.
   - Other biologics: within 3 months or 5 half-lives, whichever was longer, prior to randomisation.
11. Receipt of any investigational non-biologic agent within 5 half-lives prior to randomisation.
12. Receipt of blood products within 4 weeks prior to screening.
13. Major surgery within 8 weeks prior to screening, or planned in-patient surgery or hospitalisation during the trial period.
14. Known or suspected allergy or reaction to any component of the IMP or auxiliary medicinal product (AxMP) formulation.
15. History of any active skin infection within 1 week prior to randomisation.
16. History of a clinically significant infection within 4 weeks prior to randomisation which, in the opinion of the investigator or sponsor's medical expert, could compromise the safety of the subject in the trial, interfere with evaluation of the IMP, or reduce the subject's ability to participate in the trial. Clinically significant infections were defined as:
   - a systemic infection.
   - a serious skin infection requiring parenteral (intravenous or intramuscular) antibiotics, antiviral, or antifungal medication.
17. A helminth parasitic infection within 6 months prior to the date informed consent was obtained that had not been treated with, or had failed to respond to, standard of care therapy.
18. History of anaphylaxis following any biological therapy.
19. History of immune complex disease.
20. History of cancer:
   - Subjects who have had basal cell carcinoma, localised squamous cell carcinoma of the skin or in situ carcinoma of the cervix are eligible provided that the subject was in remission and curative therapy was completed at least 12 months prior to the date informed consent was obtained.
   - Subjects who have had other malignancies were eligible provided that the subject was in remission and curative therapy was completed at least 5 years prior to the date informed consent was obtained.
21. Tuberculosis requiring treatment within the 12 months prior to screening. Evaluation was according to local guidelines as per local standard of care.
22. History of any known primary immunodeficiency disorder including a positive human immunodeficiency virus (HIV) test at screening, or the subject taking antiretroviral medications as determined by medical history and/or subject's verbal report.
23. History of chronic alcohol or drug abuse within 12 months prior to screening, or any condition associated with poor compliance as judged by the investigator.
24. History of attempted suicide or at significant risk of suicide (either in the opinion of the investigator or defined as a "yes" to suicidal ideation questions no. 4 or 5 or answering "yes" to suicidal behaviour on the Columbia-Suicide Severity Rating Scale [C-SSRS] Screening version).
25. Any disorder, including but not limited to, cardiovascular, gastrointestinal, hepatic, renal, neurological, musculoskeletal, infectious, endocrine, metabolic, haematological, immunological, psychiatric, or major physical impairment that was not stable, in the opinion of the investigator, and could:
   - Affect the safety of the subject throughout the trial.
   - Influence the findings of the trial or their interpretations.
   - Impede the subject's ability to complete the entire duration of trial.
26. Any clinically significant abnormal findings in physical examination, vital signs, electrocardiogram (ECG), haematology, clinical chemistry, or urinalysis during the screening period, which in the opinion of the investigator, could put the subject at risk because of his/her participation in the trial, or influence the results of the trial, or the subject's ability to complete entire duration of the trial.
27. Alanine aminotransferase (ALT) or aspartate aminotransferase (AST) level ≥2.0 times the upper limit of normal (ULN) at screening.
28. Positive hepatitis B surface antigen (HBsAg), hepatitis B surface antibody (HBsAb), hepatitis B core antibody (HBcAb) or hepatitis C virus antibody (anti-HCV) serology at screening. Subjects with positive HBsAb could be randomised provided they had a history of hepatitis B vaccination and had negative HBsAg and HBcAb serology.
29. Subjects who were not willing to abstain from donating blood and/or plasma from the time of informed consent and for 16 weeks (5 half-lives) after last dose of IMP.
30. Subjects who were legally institutionalised.
31. Pregnant, breastfeeding, or lactating women.
32. Employees of the trial site or any other individuals directly involved with the planning or conduct of the trial, or immediate family members of such individuals.

### Tralokinumab - Treatment period

- On first day of dosing (Day 0= Visit 3, baseline): tralokinumab, 600 mg, 4 subcutaneous (SC) injections (1.0 mL each) of 150 mg tralokinumab.
- At subsequent visits until the last scheduled IMP dosing visit at Week 24: tralokinumab, 300 mg Q2W, 2 SC injections (1.0 mL each) of 150 mg tralokinumab.

### Placebo - Treatment period

- On first day of dosing (Day 0= Visit 3, baseline): placebo, 4 SC injections (1.0 mL each).
- At subsequent visits until Week 24 visit: placebo, Q2W, 2 SC injections (1.0 mL each).

### Statistical methods

4 estimands were defined in this trial, incorporating 3 main types of intercurrent events that influenced how the treatment effects were estimated, that being initiation of rescue medication, permanent discontinuation of IMP and subject-onset of the COVID-19 pandemic. The overall type 1 error rate for the primary analysis of the primary estimands for the primary and secondary endpoints was protected by a hierarchical testing strategy. The endpoints were tested sequentially at a 5% significance level.

No interim analyses were conducted.

### Primary endpoint (EASI75 at Week 16)

The difference in response rates between treatment groups was analysed using the Mantel-Haenszel risk difference and associated standard errors stratified by prior CSA use (yes/no) and baseline disease severity (IGA= 3 or 4) combining multiple inferences using Rubin's rule.

Intercurrent events were handled based on the which event came first. Rescue treatment, permanent discontinuation of IMP, and missing data at the Week 16 visit not due to the COVID-19 pandemic were analysed as non-responders. Subject-onset of the COVID-19 pandemic and missing data at the Week 16 visit due to the COVID-19 pandemic were imputed using a stepwise multiple imputation procedure.

### Secondary endpoints

EASI75 at Week 26, IGA 0/1 at Week 16/Week 26, and reduction of Worst Daily Pruritus NRS weekly average of ≥4 from baseline to Week 16/Week 26 were analysed as described for the primary endpoint.

The changes in SCORAD and DLQI scores from baseline to Week 16/ Week 26 were analysed using a repeated measurements model on post-baseline responses up to the Week 26 visit, only including data prior to an intercurrent event.

### Health care resource utilisation (use of TCS)

The amount of TCS used was analysed by a repeated measurements model. To handle missing data due to non-returned tubes, the amount of TCS used was analysed based on 2 different approaches: 1) that no TCS was used from the non-returned tubes, or 2) that all TCS was used from the non-returned tubes.

### Summary of results

Treatment period: 277 subjects were randomised in a 1:1 ratio, with 140 subjects randomised to tralokinumab+TCS and 137 subjects randomised to placebo+TCS. 2 subjects in the tralokinumab+TCS group were not dosed.

125 subjects (89.3%) in the tralokinumab+TCS group and 120 subjects (87.6%) in the placebo+TCS group completed Week 26 on treatment.

### Demographics and baseline characteristics

Overall, demographics and baseline characteristics were well-balanced between the tralokinumab+TCS and placebo+TCS groups. The mean age at baseline was 36.5 years (SD: 14.1), mean weight was 75.7 kg (SD: 16.3), and 59.6% of subjects were men. At baseline, the mean BSA affected by AD was 54.7% (SD:22.2), the mean age of onset of AD was 10.3 years (SD: 15.4), and the mean duration of AD was 26.2 years (13.9). Disease severity at baseline were comparable between the treatment groups. All subjects had severe AD (EASI score ≥20 at baseline). The mean EASI score was 32.95 (SD: 12.54), mean SCORAD score was 70.51 (SD: 12.43), mean DLQI score was 16.11 (SD: 6.43), and mean Worst Daily Pruritus NRS (weekly average) was 7.37 (SD: 1.41).

The type of previous AD treatments and proportion of subjects using the treatments, as well as their atopy history were comparable between treatment groups and as expected for a population with long-term and severe AD.

### Treatment exposure and compliance

The majority of subjects were exposed to IMP for ≥16 weeks (93.5% with tralokinumab+TCS and 95.6% with placebo+TCS). 79.7% and 71.5% of subjects received all planned doses of IMP (tralokinumab+TCS or placebo+TCS, respectively) up to the end-of-study or permanent discontinuation of IMP. The COVID-19 pandemic had limited impact as only 24 subjects (8.7%) missed 1 or 2 IMP doses due to the COVID-19 pandemic (10 subjects in the tralokinumab+TCS group and 14 subjects in the placebo+TCS group), and 1 subject (0.7%) in the placebo+TCS group missed more than 3 IMP doses due to the COVID-19 pandemic.

### Efficacy results

The efficacy of tralokinumab in combination with TCS in the treatment of adult subjects with severe AD who are ineligible to treatment with CSA, was demonstrated during the treatment period of this randomised, placebo-controlled, clinical phase 3 trial. The results of the primary and secondary endpoints are summarised below.

The clinical efficacy of tralokinumab treatment was assessed by 3 different investigator assessments (EASI, IGA, and SCORAD). EASI was used for the primary and secondary endpoints, while IGA and SCORAD were used for the secondary endpoints. Several validated patient-reported questionnaires were included to assess the efficacy of tralokinumab on PROs and health-related quality of life (HRQoL).

### Primary endpoint (EASI75 at Week 16)

The proportion of EASI75 responders at Week 16 was statistically significantly higher in the tralokinumab+TCS group compared with the placebo+TCS group. The treatment difference was 14.1% (95% CI: 2.5 to 25.7, p=0.018) at Week 16, with 64.2% responders in the tralokinumab+TCS group and 50.5% responders in the placebo+TCS group (primary analysis of the primary estimand). The primary analyses of the secondary, tertiary and quaternary estimands, and sensitivity analysis led to the same conclusion as the primary analysis of the primary estimand (p<0.05; all analyses).

### Secondary endpoints

No secondary endpoints could be confirmed by the sequential testing hierarchy as the first secondary endpoint, reduction of Worst Daily Pruritus NRS (weekly average) of ≥4 from baseline to Week 16, failed to show statistically significance (p=0.106); even though the results seemed to imply different responses in the treatment groups as 45.5% with tralokinumab+TCS had achieved a 4-point reduction in Worst Daily Pruritus NRS at Week 16 (35.6% with placebo+TCS at Week 16) (at Week 26: 47.2% with tralokinumab+TCS and 39.7% with placebo+TCS). However, irrespective of the confirmatory testing hierarchy, all other secondary endpoints showed improvements in favour of tralokinumab+TCS in adult subjects with severe AD at Week 16 and Week 26 (p<0.05).
- The proportion of EASI75 responders at Week 26 was higher in the tralokinumab+TCS group compared with the placebo+TCS group. The treatment difference was 14.1% (95% CI: 2.9 to 25.3, p=0.014) at Week 26, with 68.8% responders in the tralokinumab+TCS group and 55.3% responders in the placebo+TCS group (primary analysis of the primary estimand). The primary analyses of the secondary, tertiary and quaternary estimands, and sensitivity analysis led to the same conclusion as the primary analysis of the primary estimand (p<0.05; all analyses).
- The proportion of IGA 0/1 responders at Week 16 and Week 26 was higher in the tralokinumab+TCS group compared with the placebo+TCS group. Based on the primary analyses of the primary estimand, the treatment difference was:
   ∘ 15.6% (95% CI: 4.8 to 26.3, p=0.005) at Week 16, with 40.9% responders in the tralokinumab+TCS group and 26.0% responders in the placebo+TCS group.
   ∘ 14.3% (95% CI: 2.9 to 25.6, p=0.014) at Week 26, with 47.0% responders in the tralokinumab+TCS group and 33.4% responders in the placebo+TCS group.
   The primary analyses of the secondary, tertiary and quaternary estimands, and sensitivity analyses led to the same conclusion as the primary analyses of the primary estimand at Week 16 and Week 26 (p<0.05; all analyses).
- The changes in SCORAD from baseline to Week 16 and Week 26 were larger in the tralokinumab+TCS group compared with the placebo+TCS group. Based on the primary analyses of the primary estimand, the treatment difference was:
   ∘ -8.6 (95% CI: -13.0 to -4.2, p<0.001) at Week 16, with an adjusted mean change of -42.7 (SE: 1.6) in the tralokinumab+TCS group and -34.1 (SE: 1.6) in the placebo+TCS group.
   ∘ -8.9 (95% CI: -13.2 to -4.6, p<0.001) at Week 26, with an adjusted mean change of -46.3 (SE: 1.5) in the tralokinumab+TCS group and -37.3 (SE: 1.6) in the placebo+TCS group.
   The primary analyses of the secondary, tertiary and quaternary estimands, and sensitivity analyses led to the same conclusion as the primary analyses of the primary estimand at Week 16 and Week 26 (p<0.05; all analyses).
- The changes in DLQI from baseline to Week 16 and Week 26 were larger in the tralokinumab+TCS group than in the placebo+TCS group. Based on the primary analyses of the primary estimand, the treatment difference was:
   ∘ -1.5 (95% CI: -2.6 to -0.4, p=0.009) at Week 16, with an adjusted mean change of -11.2 (SE: 0.4) in the tralokinumab+TCS group and -9.6 (SE: 0.4) in the placebo+TCS group.
   ∘ -1.6 (95% CI: -2.7 to -0.5, p=0.005) at Week 26, with an adjusted mean change of -11.5 (SE: 0.4) in the tralokinumab+TCS group and -9.9 (SE: 0.4) in the placebo+TCS group.
   At Week 26, the primary analyses of the secondary and tertiary estimands, and the sensitivity analyses led to the same conclusion as the primary analysis of the primary estimand (p<0.05; all analyses). At Week 16, there was no observed treatment difference based on the analyses of the secondary and tertiary estimands (p≥0.05; all analyses).

### Health care resource utilisation

Irrespectively of the analysis approach (e.g. whether assuming no or all TCS was used from the non-returned tubes), the amount of supplied TCS used was lower with tralokinumab than with placebo over 26 weeks.
- Cumulative TCS use was lower with tralokinumab vs placebo at Week 15-16 (p=0.006) and Week 25-26 (p=0.002). Over the periods from Week 0-16 and Week 0-26, tralokinumab-treated subjects used approximately 30% less of the supplied TCS compared with placebo-treated subjects. At Week 15-16, ~60-65% subjects with tralokinumab and ~50-55% with placebo used no or limited amounts (0-15 g) of TCS. At Week 25-26, percentages were ~55-60% with tralokinumab and ~45-50% with placebo.
- The mean number of days without topical treatment (weekly average) was higher in the tralokinumab+TCS group than in the placebo+TCS group already from Week 3 and onwards (p<0.05).
- The proportion of subjects who had 5-7 days without topical treatment use was higher with tralokinumab compared with placebo both at Week 16 (43.8% vs 21.1% subjects, p<0.001) and Week 26 (39.8% vs 24.1%, p=0.006). The sensitivity analyses led to the same conclusion at Week 16 and Week 26 (p<0.05).

### Safety results

Overall, treatment with tralokinumab in combination with TCS for a period up to 26 weeks was well-tolerated and had an acceptable safety profile in adults with severe AD who are ineligible to treatment with CSA.

### Overall summary of adverse events

- During the trial and across the treatment groups, the incidence of AEs was comparable between tralokinumab+TCS and placebo+TCS. The majority of the reported AEs were non-serious, mild or moderate in severity, considered not related to IMP by the investigator, and most events were recovered by the end of the trial.
- In the treatment period (Week 0 to Week 26), AEs were reported at a similar incidence and rate with tralokinumab+TCS (77.5% subjects; 589.1 events per 100 PYE) and with placebo+TCS (78.8% subjects; 646.7 events per 100 PYE, p=0.795). In both treatment groups, the SOC with the most frequently reported AEs was 'infections and infestations' in >50% of subjects in each group and the most frequently reported PT within this SOC was 'viral upper respiratory tract infection' in 26.8% subjects (81.10 events per 100 PYE) with tralokinumab+TCS and 25.5% subjects (70.33 events per 100 PYE) with placebo+TCS. In the SOC 'nervous system disorders', 15.2% subjects in the tralokinumab+TCS group reported 'headache' at a rate of 38.25 events per 100 PYE vs 9.5% subjects with placebo+TCS at a rate of 27.52 events per 100 PYE.
- In the safety follow-up period, 4 events in 4 subjects (5.3%, 33.95 events per 100 PYE) treated with tralokinumab+TCS and 12 events in 6 subjects (7.2%, 91.66 events per 100 PYE) treated with placebo+TCS prior to onset of the AE were reported, with no pattern in SOCs nor PTs in any groups.

### Deaths, other serious adverse events, and other clinically important events

- No deaths were reported during the trial.
- Few SAEs were reported in the trial (a total of 11 events in 7 subjects) with most events occurring with placebo+TCS (10 SAEs in 6 subjects [4.4%]; including 1 event of 'pyelonephritis' occurring in the safety follow-up period in 1 subject treated with placebo+TCS prior to onset of the event) than with tralokinumab+TCS (1 SAE in 1 subject [0.7%]; PT 'appendicitis'). Of the 11 SAEs, 6 events were severe (PTs 'appendicitis', 'cerebrovascular accident', 'seizure', anaphylactic reaction', dermatitis atopic', and 'pyelonephritis'). 3 SAEs led to permanent discontinuation of IMP (PTs 'cerebrovascular accident', 'depressed mood' and 'suicidal ideation' [all with placebo+TCS]), of which the event of 'cerebrovascular accident' also led to withdrawal from the trial. These 3 SAEs were considered related to IMP; no other SAEs were assessed as related to the IMP in the trial. Most subjects had recovered from the SAEs by the end of the trial - except 1 subject with an event of PT 'peripheral nerve injury' ('not recovered/not resolved') and 1 subject with the events of PTs 'depressed mood' and 'suicidal ideation' reported as 'unknown' as this subject is lost to follow-up.
- Few AEs led to permanent discontinuation of IMP in the treatment period (a total of 5 events in 4 subjects): 1 event of PT 'injection site pain' in 1 subject (0.7%) with tralokinumab+TCS and 4 AEs in 3 subjects (2.2%) with placebo+TCS; of which 3 AEs led to withdrawal from trial. Of the 5 events, 3 were serious (PTs: 'cerebrovascular accident' [severe], 'depressed mood', 'suicidal ideation' [moderates in severity]), and 2 events were non-serious (PTs: 'dermatitis atopic' [moderate in severity] and 'injection site pain' [mild in severity]). Except for the event of PT 'dermatitis atopic', all events were considered possibly/probably related to IMP. The events of 'cerebrovascular accident', 'dermatitis atopic' and 'injection site pain' which led to permanent discontinuation of IMP also led to withdrawal from trial in the treatment period and their outcome was reported as 'recovered/ resolved'.
- 2 AEs of special interest (AESIs) of eczema herpeticum were reported in 1 subject with tralokinumab+TCS in the treatment period. Both events were none-serious, mild in severity, considered possibly related to IMP, and their outcome was reported as 'recovered/ resolved'.
- No AESIs of malignancies diagnosed after randomisation were reported in the trial.
- 14 AESIs related to skin infections requiring systemic treatment were reported in the trial; 1 AESI in 1 subject (0.7%) with tralokinumab+TCS (PT: 'dermatitis infected') and 13 AESIs in 9 subjects (6.6%) with placebo+TCS (including 1 AESI in the safety follow-up period). None were serious nor severe and, 6 of the 14 events were considered possibly related to IMP. Most subjects had recovered from the events by the end of the trial - except for 1 event of 'staphylococcal skin infection' in the placebo+TCS group reported as 'not resolved/not recovered' at end of trial.
- 26 AESIs related to pre-defined eye disorders (conjunctivitis, keratoconjunctivitis, and keratitis) were reported in the trial (all reported in the treatment period). The majority of events were classified as conjunctivitis with a higher frequency with tralokinumab+TCS (15 events in 13 subjects [9.4%]) than with placebo+TCS (8 events in 6 subjects [4.4%]) (PTs 'conjunctivitis', 'conjunctivitis allergic' and 'conjunctivitis viral'). 1 event classified as keratoconjunctivitis (PT 'atopic keratoconjunctivitis') was reported in 1 subject (0.7%) in the tralokinumab+TCS group. 2 events classified as keratitis were reported (1 event in 1 subject [0.7%] in each treatment group). Of these 26 AESIs related to pre-defined eye disorders, none were serious nor severe, and none led to withdrawal from the trial. 11 of the 26 events were considered related to IMP. The outcome of all but 6 of the events (3 events in each treatment group) was reported as 'recovered/resolved' or 'recovering/resolving' at end of trial.
- 17 AEs related to injection site reactions were reported in the treatment period (12 events in 9 subjects with tralokinumab+TCS and 5 events in 3 subjects with placebo+TCS). The majority of events were 'injection site reaction'. Of the 17 events, none were serious nor severe. Apart from 1 event in the tralokinumab+TCS group, no withdrawals from the trial was seen due to 'injection site reaction'. All events were considered related to IMP and their outcome was reported as 'recovered/resolved'.
- Except 1 event of 'eosinophil count increased' reported in the screening period ('recovered/resolved' before the first IMP administration), no AEs of eosinophilia were reported in the trial.

### Pregnancies

No pregnancies were reported during the trial.

### Clinical laboratory evaluation

- Except for eosinophils values, no other clinically relevant changes in laboratory parameters were observed in any of the treatment groups during the trial.

- At baseline, the mean levels of eosinophils were within the normal range in subjects in both tralokinumab+TCS group (0.454×10⁹/L [SD: 0.355×10⁹/L]) and placebo+TCS group (0.543×10⁹/L [SD: 0.410×10⁹/L]). From Week 4, the mean level of eosinophils remained higher in subjects receiving tralokinumab+TCS than subjects receiving placebo+TCS. At Week 16, the mean change from baseline was 0.140×10⁹/L (SD: 0.459×10⁹/L) with tralokinumab+TCS and -0.148×10⁹/L (SD: 0.305×10⁹/L) with placebo+TCS, and the mean counts were 0.593×10⁹/L (SD: 0.510×10⁹/L) with tralokinumab+TCS and 0.409×10⁹/L (SD: 0.262×10⁹/L) with placebo+TCS. At Week 26, the mean change from baseline was 0.112×10⁹/L (SD: 0.465×10⁹/L) with tralokinumab+TCS and -0.171×10⁹/L (SD: 0.365×10⁹/L) with placebo+TCS, and the mean counts were 0.565×10⁹/L (SD: 0.514×10⁹/L) with tralokinumab+TCS and 0.394×10⁹/L (SD: 0.317×10⁹/L) with placebo+TCS.

### Other safety assessments

- No clinically relevant changes were observed in findings related to vital signs, physical examination, or electrocardiogram in any of the treatment groups.

The vast majority of subjects did not develop anti-tralokinumab antibodies nor nAB. In total 2 subjects (1.4%) in the tralokinumab+TCS group and 3 subjects (2.2%) in the placebo+TCS group had a positive ADA response.

### Conclusions

This phase 3 randomised, double-blind, placebo-controlled trial demonstrated the efficacy and safety of tralokinumab in combination with TCS in adult subjects with severe AD ineligible to treatment with CSA. The results support the following conclusions:
• The EASI75 response rate at Week 16 (primary endpoint) was statistically significantly higher with tralokinumab+TCS than with placebo+TCS (p<0.05), demonstrating that tralokinumab+TCS was superior to placebo+TCS in treating severe AD.
• As the first secondary endpoint addressing itch (reduction of weekly average Worst Daily Pruritus NRS of ≥4 from baseline to Week 16) was not statically significant (p=0.106), the rest of the hierarchy could not be confirmed. Meanwhile, irrespective of the confirmatory sequential testing hierarchy:
   ∘ At Week 16, 45.5% subjects with tralokinumab+TCS had achieved a 4-point reduction in Worst Daily Pruritus NRS (35.6% with placebo+TCS at Week 16) (at Week 26: 47.2% with tralokinumab+TCS and 39.7% with placebo+TCS).
   ∘ The improvements in other secondary endpoints addressing severity and extent of AD (EASI75 at Week 26, IGA 0/1 at Week 16/Week 26 and SCORAD at Week 16/Week 26) and HRQoL (DLQI at Week 16/Week 26) measure related to AD were higher with tralokinumab+TCS than with placebo+TCS (p<0.05).
• The amounts of supplied TCS and of rescue medication used were lower in tralokinumab-treated subjects than in placebo-treated subjects at Week 16 and Week 26 supporting the demonstrated efficacy of tralokinumab. More tralokinumab-treated subjects had 5-7 days without topical treatment use at both Week 16 and Week 26 than placebo-treated subjects.
• PROs (POEM, reduction of POEM of ≥4 points, EQ-5D-5L and physical component summary measures of the SF-36) showed greater improvements with tralokinumab+TCS than with placebo+TCS, indicating that subjects' own perception of the treatment effect was in line with the demonstrated clinical response (EASI75 or IGA 0/1) obtained with tralokinumab+TCS.
• Tralokinumab+TCS was well-tolerated and had an acceptable safety profile in adults with severe AD ineligible to treatment with CSA.

### List of abbreviations

AD, atopic dermatitis
AE, adverse event
AESI, adverse event of special interest
BSA, body surface area involvement
CI, confidence interval
DLQI, Dermatology Life Quality Index
EASI, Eczema Area and Severity Index
EASI-50, at least 50% reduction in Eczema Area and Severity Index score
EASI-75, at least 75% reduction in Eczema Area and Severity Index score
EASI-90, at least 90% reduction in Eczema Area and Severity Index score
EQ-5D-5L, EuroQoL 5-Dimension Health Questionnaire 5 Level
FAS, full analysis set
HADS = Hospital Anxiety and Depression Scale
HRQoL, Health-related quality of life
IGA, Investigator's Global Assessment
IGA-0/1, Investigators' Global Assessment score of 0 (clear) or 1 (almost clear)
IMP, investigational medicinal product
IQR, interquartile range
MedDRA, Medical Dictionary for Regulatory Activities
NRS, Numeric Rating Scale
PT, preferred term
PYE, patient-years of exposure
Q2W, every other week, i.e. every 2 weeks
Q4W, every 4 weeks
R, rate (number of AEs divided by PYE multiplied by 100)
SAEs, serious adverse events
SCORAD, SCORing Atopic Dermatitis
SE, standard error
SF-36, Short Form (36) Health Survey
TCS, topical corticosteroids.

## Claims

1. An interleukin-13 (IL-13) binding protein for use in a method of treating atopic dermatitis (AD) in a subject, wherein the method comprises the steps of:
(i) administering prior doses of the IL-13 binding protein to the subject, wherein each prior dose is administered 2 weeks after the immediately preceding prior dose, wherein the first prior dose is around 600 mg and is administered to the subject at day 0, and each prior dose after the first prior dose is around 300 mg and is administered to the subject at weeks 2, 4, 6, 8, 10, 12, 14 and 16; and wherein if the subject achieves an IGA score of 0 or 1 and/or ≥75% improvement of EASI-75 over baseline in the subject;
(ii) administering a first dose of IL-13 binding protein to the subject, wherein the first dose is around 300 mg and is administered to the subject at week 20; and
(iii) administering secondary doses of the IL-13 binding protein to the subject, wherein each secondary dose of the IL-13 binding protein is administered to the subject 4 weeks after the immediately preceding dose, wherein each secondary dose is around 300 mg of IL-13 binding protein and is administered to the subject at weeks 24, 28, 32, 36, 40, 44, 48 and 52;
and wherein the IL-13 binding protein is tralokinumab.

2. The IL-13 binding protein for use according to claim 1, wherein the AD is moderate-to-severe or severe AD.

3. The IL-13 binding protein for use according to any one of the preceding claims, wherein each dose of IL-13 binding protein is administered in one or two injections.

4. The IL-13 binding protein for use according to any one of the preceding claims, wherein each dose of the IL-13 binding protein is administered subcutaneously.

5. The IL-13 binding protein for use according to any one of the preceding claims, wherein each dose of the IL-13 binding protein is administered as a pharmaceutical composition comprising 50 mM sodium acetate buffer, 85 mM sodium chloride, 0.01% (w/v) polysorbate 80, wherein the pharmaceutical composition has a pH of 5.5.

6. The IL-13 binding protein for use according to any one of the preceding claims, wherein the IL-13 binding protein is administered as a monotherapy.

7. The IL-13 binding protein for use according to any one of the preceding claims, wherein the IL-13 binding protein is administered in combination with a second therapeutic agent selected from the group consisting of a topical corticosteroid, a topical calcineurin inhibitor, an anti-histamine, an emollient, or an anti-bacterial therapeutic.

## Patentansprüche

1. Interleukin-13- (IL-13-) bindendes Protein zur Verwendung in einem Verfahren zur Behandlung von atopischer Dermatitis (AD) in einem Individuum, wobei das Verfahren die folgenden Schritte umfasst:
(i) Verabreichen von vorherigen Dosen des IL-13-bindenden Proteins an das Individuum, wobei jede vorherige Dosis 2 Wochen nach der unmittelbar vorherigen Dosis verabreicht wird, wobei die erste vorherige Dosis etwa 600 mg beträgt und dem Individuum an Tag 0 verabreicht wird und jede vorherige Dosis nach der ersten vorherigen Dosis etwa 300 mg beträgt und dem Individuum in Woche 2, 4, 6, 8, 10, 12, 14 und 16 verabreicht wird; und wobei, wenn das Individuum eine IGA-Bewertung von 0 oder 1 und/oder eine ≥ 75%ige Verbesserung von EASI-75 über der Grundlinie in dem Individuum erreicht;
(ii) Verabreichen einer ersten Dosis des IL-13-bindenden Proteins an das Individuum, wobei die erste Dosis etwa 300 mg beträgt und dem Individuum in Woche 20 verabreicht wird; und
(iii) Verabreichen von sekundären Dosen des IL-13-bindenden Proteins an das Individuum, wobei jede sekundäre Dosis des IL-13-bindenden Proteins dem Individuum 4 Wochen nach der unmittelbar vorangegangenen Dosis verabreicht wird, wobei jede sekundäre Dosis etwa 300 mg des IL-13-bindenen Proteins beträgt und dem Individuum in Woche 24, 28, 32, 36, 40, 44, 48 und 52 verabreicht wird;
und wobei das IL-13-bindende Protein Tralokinumab ist.

2. IL-13-bindendes Protein zur Verwendung nach Anspruch 1, wobei die AD mäßig bis schwere AD oder schwere AD ist.

3. IL-13-bindendes Protein zur Verwendung nach einem der vorangegangenen Ansprüche, wobei jede Dosis des IL-13-bindenden Proteins in einer oder zwei Injektionen verabreicht wird.

4. IL-13-bindendes Protein zur Verwendung nach einem der vorangegangenen Ansprüche, wobei jede Dosis des IL-13-bindenden Proteins subkutan verabreicht wird.

5. IL-13-bindendes Protein zur Verwendung nach einem der vorangegangenen Ansprüche, wobei jede Dosis des IL-13-bindenden Proteins als pharmazeutische Zusammensetzung, umfassend 50 mM Natriumacetatpuffer, 85 mM Natriumchlorid, 0,01 % (Gew./Vol.) Polysorbat 80, verabreicht wird, wobei die pharmazeutische Zusammensetzung einen pH-Wert von 5,5 aufweist.

6. IL-13-bindendes Protein zur Verwendung nach einem der vorangegangenen Ansprüche, wobei das IL-13-bindende Protein als Monotherapie verabreicht wird.

7. IL-13-bindendes Protein zur Verwendung nach einem der vorangegangenen Ansprüche, wobei das IL-13-bindende Protein in Kombination mit einem zweiten Therapeutikum verabreicht wird, das aus der aus einem topischen Kortikosteroid, einem topischen Calcineurininhibitor, einem Anti-Histaminikum, einem Emolliens oder einem antibakteriellen Therapeutikum bestehenden Gruppe ausgewählt ist.

## Revendications

1. Protéine de liaison interleukine-13 (IL-13) pour utilisation dans un procédé de traitement de la dermatite atopique (DA) chez un sujet, dans laquelle le procédé comprend les étapes consistant à :
(i) administrer des doses antérieures de la protéine de liaison IL-13 au sujet, dans laquelle chaque dose antérieure est administrée 2 semaines après la dose antérieure immédiatement précédente, dans laquelle la première dose antérieure est d'environ 600 mg et est administrée au sujet au jour 0, et chaque dose antérieure après la première dose antérieure est d'environ 300 mg et est administrée au sujet aux semaines 2, 4, 6, 8, 10, 12, 14 et 16 ; et dans laquelle si le sujet atteint un score IGA de 0 ou 1 et/ou une amélioration ≥ 75 % de l'EASI-75 par rapport à la ligne de base chez le sujet ;
(ii) administrer une première dose de protéine de liaison IL-13 au sujet, la première dose étant d'environ 300 mg et étant administrée au sujet à la semaine 20 ; et
(iii) administrer des doses secondaires de la protéine de liaison IL-13 au sujet, dans laquelle chaque dose secondaire de la protéine de liaison IL-13 est administrée au sujet 4 semaines après la dose immédiatement précédente, dans laquelle chaque dose secondaire est d'environ 300 mg de protéine de liaison IL-13 et est administrée au sujet aux semaines 24, 28, 32, 36, 40, 44, 48 et 52 ;
et dans laquelle la protéine de liaison IL-13 est le tralokinumab.

2. Protéine de liaison IL-13 pour utilisation selon la revendication 1, dans laquelle la DA est une DA modérée à sévère ou sévère.

3. Protéine de liaison IL-13 pour utilisation selon l'une quelconque des revendications précédentes, dans laquelle chaque dose de protéine de liaison IL-13 est administrée en une ou deux injections.

4. Protéine de liaison IL-13 pour utilisation selon l'une quelconque des revendications précédentes, dans laquelle chaque dose de la protéine de liaison IL-13 est administrée par voie sous-cutanée.

5. Protéine de liaison IL-13 pour utilisation selon l'une quelconque des revendications précédentes, dans laquelle chaque dose de la protéine de liaison IL-13 est administrée sous la forme d'une composition pharmaceutique comprenant 50 mM de tampon d'acétate de sodium, 85 mM de chlorure de sodium, 0,01 % (p/v) de polysorbate 80, dans laquelle la composition pharmaceutique présente un pH de 5,5.

6. Protéine de liaison IL-13 pour utilisation selon l'une quelconque des revendications précédentes, dans laquelle la protéine de liaison IL-13 est administrée en monothérapie.

7. Protéine de liaison IL-13 pour utilisation selon l'une quelconque des revendications précédentes, dans laquelle la protéine de liaison IL-13 est administrée en combinaison avec un second agent thérapeutique choisi dans le groupe constitué d'un corticostéroïde topique, d'un inhibiteur de calcineurine topique, d'un antihistaminique, d'un émollient ou d'un agent thérapeutique anti-bactérien.
